(19) Europäisches Patentamt
European Patent Office
Office européen des brevets

(11) Veröffentlichungsnummer : **0 254 078 B1**

(12) # EUROPÄISCHE PATENTSCHRIFT

(45) Veröffentlichungstag der Patentschrift : **06.02.91 Patentblatt 91/06**

(21) Anmeldenummer : **87109306.8**

(22) Anmeldetag : **29.06.87**

(51) Int. Cl.[5] : **C07D 309/30**, C07D 309/10, C07D 407/12, C07D 309/14, A01N 43/16, A01N 43/32, A01N 43/28

(54) **Pyranderivate.**

(30) Priorität : **05.07.86 DE 3622599**

(43) Veröffentlichungstag der Anmeldung : **27.01.88 Patentblatt 88/04**

(45) Bekanntmachung des Hinweises auf die Patenterteilung : **06.02.91 Patentblatt 91/06**

(84) Benannte Vertragsstaaten : **AT BE CH DE ES FR GB GR IT LI NL SE**

(56) Entgegenhaltungen :
FR-A- 1 036 183
US-A- 2 514 168
CHEMICAL ABSTRACTS, Band 81, 1974, Seite 302, Zusammenfassung Nr. 3737d, Columbus, Ohio, US; L. PROEVSKA et al.: "Synthesis of 2,6-bis(4-phenylpyridyl)-4-phenylpyridine (terosite)", & IZV. OTD. KHIM. NAUKI, BULG. AKAD. NAUK. 1973, 6(4), 747-51
CHEMICAL ABSTRACTS, Band 73, 1970, Seite 356, Zusammenfassung Nr. 98731u, Columbus, Ohio, US; V.G. KOZYREV et al.: "Aryloxydihydropyrans. IX. Diene condensation of vinyl aryl ethers with croton- and cinnamaldehydes", & KHIM. GETEROTSIKL. SOEDIN. 1970, (6), 730-2

(73) Patentinhaber : **BASF Aktiengesellschaft**
**Carl-Bosch-Strasse 38**
**D-6700 Ludwigshafen (DE)**

(72) Erfinder : **Himmele, Walter,Dr.**
**Eichenweg 14**
**D-6909 Walldorf (DE)**
Erfinder : **Theobald, Hans, Dr.**
**Queichstrasse 6**
**D-7603 Limburgerhof (DE)**
Erfinder : **Goetz, Norbert, Dr.**
**Schoefferstrasse 25**
**D-6520 Worms 1 (DE)**
Erfinder : **Zombik, Winfried, Dr.**
**Kallstadter Strasse 4**
**D-6804 Ilvesheim (DE)**
Erfinder : **Wild, Jochen, Dr.**
**An der Marlach 7**
**D-6705 Deidesheim (DE)**
Erfinder : **Adolphi, Heinrich, Dr.**
**Kalmitweg 11**
**D.6703 Limburgerhof (DE)**
Erfinder : **Hofmeister, Peter, Dr.**
**Bernard-Humblot Strasse 12**
**D-6730 Neustadt (DE)**
Erfinder : **Kuenast, Christoph, Dr.**
**Muehlstrasse 21**
**D-6701 Waldsee (DE)**

**EP 0 254 078 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Pyranderivate der allgemeinen Formeln Ia, Ib und Ic

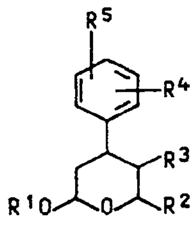
(Ia)

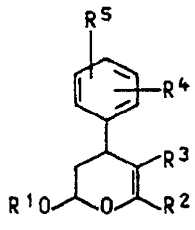
(Ib)

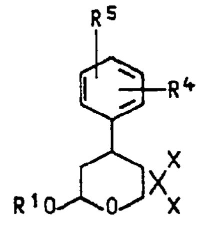
(Ic)

in denen die Substituenten folgende Bedeutung haben :

$R^1$ $C_1$-$C_{20}$-Alkyl, $C_2$-$C_{20}$-Alkenyl, $C_2$-$C_{20}$-Alkinyl, $C_3$-$C_{20}$-Cycloalkyl, Aryl, Biphenylyl, $C_7$-$C_{30}$-Arylalkyl, $C_8$-$C_{30}$-Arylalkenyl, $C_8$-$C_{30}$-Aryl-alkinyl, $C_2$-$C_{20}$-Alkoxy-alkyl, $C_3$-$C_{20}$-Alkoxy-alkoxy-alkyl, $C_4$-$C_{30}$-Alkoxy-alkoxy-alkoxy-alkyl, durch $C_7$-$C_{20}$-Alkyl-aryl, $C_8$-$C_{20}$-Dialkyl-aryl, $C_9$-$C_{20}$-Trialkyl-aryl, Aryloxy, Aryloxy-aryl, $C_3$-$C_{20}$-Cycloalkyl, $C_4$-$C_{20}$-Alkyl-cycloalkyl, $C_4$-$C_{20}$-Cycloalkenyl oder $C_5$-$C_{20}$-Alkyl-cycloalkenyl substituiertes $C_1$-$C_{20}$-Alkyl, $C_4$-$C_{20}$-Alkyl-cycloalkyl, $C_9$-$C_{20}$-Aryl-cycloalkyl, $C_9$-$C_{30}$-Aryloxy-cycloalkyl, $C_7$-$C_{20}$-Alkyl-aryl, $C_7$-$C_{20}$-Alkoxy-aryl, $C_8$-$C_{20}$-Dialkyl-aryl, $C_7$-$C_{20}$-Halogenalkyl-aryl, Halogenaryl, Dihalogenaryl, $C_7$-$C_{20}$-Alkyl-halogenaryl, $C_8$-$C_{20}$-Dialkylhalogenaryl, Aryloxy-aryl, $C_{13}$-$C_{30}$-Aryloxy-arylalkyl, oder durch einen gesättigten oder ungesättigten 5-, 6-, 7- oder 8-gliedrigen Heterocyclus, mit einem, zwei oder drei gleichen oder verschiedenen Heteroatomen aus der Gruppe bestehend aus Sauerstoff, Schwefel und Stickstoff, der gegebenenfalls durch ein, zwei oder drei gleiche oder unterschiedliche Substituenten aus der Gruppe bestehend aus $C_1$-$C_{20}$-Alkyl, Aryl, $C_7$-$C_{20}$-Arylalkyl, Halogenaryl, $C_7$-$C_{20}$-Alkylaryl, $C_8$-$C_{20}$-Dialkyl-aryl, $C_9$-$C_{20}$-Trialkyl-aryl oder ein gegebenenfalls durch $C_1$-$C_8$-Alkyl substituiertes $C_3$-$C_{20}$-Spirocycloalkyl substituiert ist, substituiertes $C_1$-$C_{20}$-Alkyl,

$R^2$ Wasserstoff, $C_1$-$C_{20}$-Alkyl, gegebenenfalls durch Phenyl oder Halogenphenyl substituiertes $C_1$-$C_{20}$-Halogenalkyl, $C_1$-$C_{20}$-Alkoxy, $C_1$-$C_{20}$-Halogenalkoxy, $C_2$-$C_{20}$-Alkenyloxy, $C_2$-$C_{20}$-Halogenalkenyloxy, $C_3$-$C_{20}$-Alkoxy-alkenyloxy, $C_2$-$C_{20}$-Alkinyloxy, $C_2$-$C_{20}$-Halogenalkinyloxy, $C_3$-$C_{20}$-Alkoxy-alkinyloxy, $C_8$-$C_{20}$-Phenyl-alkinyloxy, $C_1$-$C_{20}$-Alkylthio, $C_1$-$C_{20}$-Halogenalkylthio, $C_2$-$C_{20}$-Alkoxy-alkylthio, Formyl, Cyano, $C_7$-$C_{20}$-Phenoxy-alkoxy, $C_7$-$C_{20}$-Halogenphenoxy-alkoxy, $C_7$-$C_{20}$-Phenoxy-halogenalkoxy, $C_7$-$C_{20}$Halogenphenoxy-halogenalkoxy, $C_8$-$C_{20}$-Alkoxy-phenoxyalkoxy, $C_8$-$C_{20}$-Phenoxy-alkoxyalkoxy, $C_7$-$C_{20}$-Phenoxy-alkenyloxy, $C_8$-$C_{20}$-Halogenphenoxy-alkenyl, $C_8$-$C_{20}$-Phenoxy-halogenalkenyloxy, $C_8$-$C_{20}$-Halogenphenoxy-halogenalkenyloxy, $C_9$-$C_{20}$-Alkoxyphenoxy-alkenyloxy, $C_9$-$C_{20}$-Phenoxy-alkoxy-alkenyloxy, $C_8$-$C_{20}$-Phenoxy-alkinyloxy, $C_8$-$C_{20}$-Halogenphenoxy-alkinyloxy, $C_8$-$C_{20}$-Phenoxy-halogenalkinyloxy, $C_8$-$C_{20}$-Halogenphenoxy-halogenalkinyloxy, $C_9$-$C_{20}$-Alkoxyphenoxy-alkinyloxy, $C_9$-$C_{20}$-Phenoxy-alkoxyalkinyloxy, $C_8$-$C_{20}$-Phenyl-alkinyloxy, $C_{14}$-$C_{30}$-Phenoxyphenyl-alkinyloxy, Carboxy, $C_2$-$C_{20}$-Alkoxycarbonyl, $C_2$-$C_{20}$-Alkylcarbonyloxy, $C_3$-$C_{20}$-Alkenyloxycarbonyl, $C_3$-$C_{20}$-Alkenylcarbonyloxy, $C_3$-$C_{20}$-Alkinyloxycarbonyl, $C_3$-$C_{20}$-Alkinylcarbonyloxy, Phenoxycarbonyl, Phenylcarbonyloxy, $C_3$-$C_{20}$-Alkoxycarbonyl-alkoxy, Phenoxy, $C_7$-$C_{20}$-Alkylphenoxy, Halogenphenoxy, Dihalogenphenoxy, Trihalogenphenoxy, $C_7$-$C_{20}$-Halogenalkyl-phenoxy, $C_7$-$C_{20}$-Alkoxyphenoxy, Phenoxy-phenoxy, $C_8$-$C_{12}$-Alkylendioxy-phenoxy, Phenylthio, $C_7$-$C_{20}$-Alkylphenylthio, Halogen-phenylthio, Dihalogenphenylthio, Trihalogen-phenylthio, $C_7$-$C_{20}$-Halogenalkyl-phenylthio, $C_7$-$C_{20}$-Alkoxyphenylthio, Phenoxy-phenylthio, $C_8$-$C_{12}$-Alkylendioxyphenylthio, Phenyl, Phenoxy-phenyl, $C_7$-$C_{20}$-Phenylalkyl, $C_{13}$-$C_{30}$-Phenoxy-phenylalkyl, $C_8$-$C_{12}$-Alkylendioxy-phenylalkoxy oder einen Rest ausgewählt aus

$-CH=N-OH$ ; 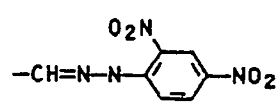 ; 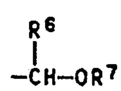 ; 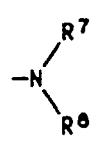 ;

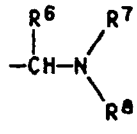 ; 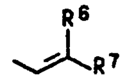 ; 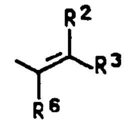 ; 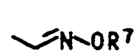 oder 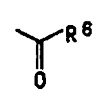

$R^3$ Wasserstoff, $C_1$-$C_{20}$-Alkyl oder $C_1$-$C_3$-Halogenalkyl,

$R^4$, $R^5$ Wasserstoff, Fluor, Chlor, $C_1$-$C_3$-Alkyl, $C_1$-$C_3$-Alkoxy oder $C_1$-$C_3$-Halogenalkoxy,

$R^6$ Wasserstoff, $C_1$-$C_{20}$-Alkyl, $C_2$-$C_{20}$-Alkenyl, $C_2$-$C_{20}$-Alkinyl, Cyano, Phenyl, Halogenphenyl, $C_7$-$C_{20}$-Alkylphenyl oder $C_7$-$C_{20}$-Alkoxyphenyl,

$R^7$ Wasserstoff, $C_1$-$C_{20}$-Alkyl, $C_1$-$C_{20}$-Halogenalkyl, $C_2$-$C_{20}$-Alkoxy-alkyl, $C_2$-$C_{20}$-Alkenyl, $C_2$-$C_{20}$-Alkinyl, Aryl, Halogenphenyl, Dihalogenphenyl, Trihalogenphenyl, Cyanophenyl, $C_7$-$C_{20}$-Alkylphenyl, $C_7$-$C_{20}$-Alkoxyphenyl, $C_7$-$C_{20}$-Halogenalkyl-phenyl, $C_8$-$C_{20}$-Alkenylphenyl, $C_8$-$C_{20}$-Alkinylphenyl, Phenoxyphenyl, $C_8$-$C_{12}$-Methylendioxy-phenyl, $C_2$-$C_{20}$-Alkylcarbonyl, $C_2$-$C_{20}$-Halogenalkylcarbonyl, $C_2$-$C_{20}$-Alkoxycarbonyl, $C_5$-$C_{20}$-Alkylcyclopropyl-carbonyl, $C_7$-$C_{20}$-Alkenyl- und -alkylcyclopropyl-carbonyl, $C_6$-$C_{20}$-Halogenalkyl- und -alkylcyclopropyl-carbonyl, $C_7$-$C_{20}$-Halogenalkenyl- und -alkylcyclopropyl-carbonyl, $C_8$-$C_{20}$-Halogen- und -phenyl-alkylcarbonyl, $C_8$-$C_{20}$-Halogen- und -halogen-phenylalkylcarbonyl, Benzoyl, Halogenbenzoyl, $C_8$-$C_{20}$-Alkylbenzoyl, $C_8$-$C_{20}$-Alkoxybenzoyl, $C_8$-$C_{20}$-Halogenalkyl-benzoyl, $C_8$-$C_{20}$-Halogenalkoxy-benzoyl, $C_8$-$C_{20}$-Phenylalkylcarbonyl, $C_8$-$C_{20}$-Halogenphenylalkylcarbonyl, $C_9$-$C_{20}$-Alkylphenylalkylcarbonyl, $C_9$-$C_{20}$-Alkoxyphenylalkylcarbonyl, $C_9$-$C_{20}$-Halogenalkylphenylalkylcarbonyl, $C_9$-$C_{20}$-Halogenalkoxyphenylalkylcarbonyl, $C_9$-$C_{20}$-Phenylalkenylcarbonyl, $C_9$-$C_{20}$-Halogenphenylalkenylcarbonyl, $C_{10}$-$C_{20}$-Alkylphenylalkenylcarbonyl, $C_{10}$-$C_{20}$-Alkoxyphenylalkenylcarbonyl, $C_{10}$-$C_{20}$-Halogenalkylphenylalkenylcarbonyl, $C_{10}$-$C_{20}$-Halogenalkoxyphenylalkenylcarbonyl, $C_9$-$C_{20}$-Phenylalkinylcarbonyl, $C_9$-$C_{20}$-Halogenphenylalkinylcarbonyl, $C_{10}$-$C_{20}$-Alkylphenylalkinylcarbonyl, $C_{10}$-$C_{20}$-Alkoxyphenylalkinylcarbonyl, $C_{10}$-$C_{20}$-Halogenalkylphenylalkinylcarbonyl, $C_{10}$-$C_{20}$-Halogenalkoxyphenylalkinylcarbonyl, $C_2$-$C_{20}$-Alkylaminocarbonyl, $C_2$-$C_{20}$-Halogenalkylaminocarbonyl, $C_3$-$C_{20}$-Alkoxyalkylaminocarbonyl, $C_3$-$C_{20}$-Halogenalkylalkylaminocarbonyl, $C_3$-$C_{20}$-Halogenalkoxyalkylaminocarbonyl, $C_3$-$C_{20}$-Dialkylaminocarbonyl, $C_3$-$C_{20}$-Halogendialkylaminocarbonyl, $C_4$-$C_{20}$-Alkoxydialkylaminocarbonyl, $C_3$-$C_{20}$-Alkyl- und -alkoxy-aminocarbonyl, $C_4$-$C_{20}$-Halogenalkyldialkylaminocarbonyl, $C_4$-$C_{20}$-Halogenalkoxydialkylaminocarbonyl, Phenylaminocarbonyl, Halogenphenylaminocarbonyl, Dihalogenphenylaminocarbonyl, Trihalogenphenylaminocarbonyl, $C_8$-$C_{20}$-Alkylphenylaminocarbonyl, $C_8$-$C_{20}$-Alkoxyphenylaminocarbonyl, $C_8$-$C_{20}$-Halogenalkoxyphenylaminocarbonyl oder gegebenenfalls durch $C_1$-$C_8$-Alkyl substituierte Isoxazole,

$R^8$ Wasserstoff oder $C_1$-$C_{20}$-Alkyl und

X Halogen,

mit der Maßgabe, daß in Formel Ia $R^1$ nicht Phenyl bedeutet, wenn $R^2$, $R^3$, $R^4$, $R^5$ gleichzeitig für Wasserstoff stehen, und in Formel Ib $R^2$, $R^3$, $R^4$, $R^5$ nicht gleichzeitig Wasserstoff bedeuten, wenn $R^1$ für $C_4$-$C_8$-Alkyl, Phenyl, Tolyl oder Methoxyphenyl steht, sowie $R^1$ nicht Octyl bedeutet, wenn $R^2$ und $R^3$ für Wasserstoff und $R^4$ oder $R^5$ für Methyl, Methoxy oder Chlor stehen, sowie ferner mit der Maßgabe, daß in Formel Ib $R^1$ nicht n-Butyl bedeutet, wenn $R^2$ für Methyl und $R^3$, $R^4$ und $R^5$ für Wasserstoff stehen.

Die für die Reste $R^1$ bis $R^8$ angegebenen C-Zahlen bedeuten die Summe aller C-Atome in den jeweiligen Resten,

Neue Beschreibungsseiten 3 und 14

Außerdem betrifft die Erfindung die Herstellung der Verbindungen Ia, Ib und Ic, Schädlingsbekämpfungsmittel, die die Verbindungen Ia, Ib und/oder Ic als Wirkstoffe enthalten, und ein Verfahren zur Bekämpfung von Schädlingen mit diesen Verbindungen.

Aus Chemical Reviews 75, 651-692 (1975) ; den Chemical Abstracts 69, 67 171z ; 73, 98731u ; 76, 14250 ; 81, 3737d ; 93, 25857t ; 101, 23272h und Khim.Geterotsikl. Soedin. 6, 730 (1970) sind Pyranderivate bekannt, die aber nicht als Wirkstoffe für Schädlingsbekämpfungsmittel beschrieben sind.

Der Erfindung lag daher die Aufgabe zugrunde, Pyranderivate bereitzustellen, die zur Bekämpfung von Schädlingen geeignet sind.

Demgemäß wurden die eingangs definierten neuen Pyranderivate Ia, Ib und Ic sowie Verfahren zu deren Herstellung gefunden.

Die Verbindungen Ib sind nach folgenden Methoden erhältlich :

a) durch Umsetzung von Vinylethern II mit monomer vorliegenden α,β-ungesättigten Aldehyden und Ketonen III nach folgender Reaktionsgleichung :

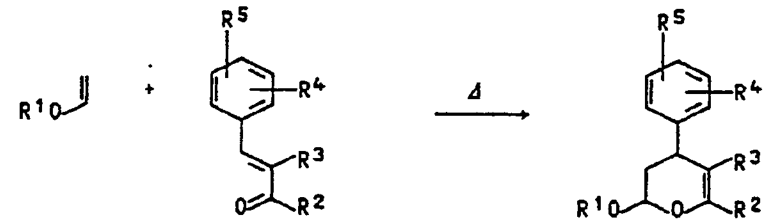


(II) (III) (Ib)

oder

b) durch Umsetzung von Vinylethern II mit dimer vorliegenden α,β-ungesättigten Aldehyden oder Ketonen IIIa

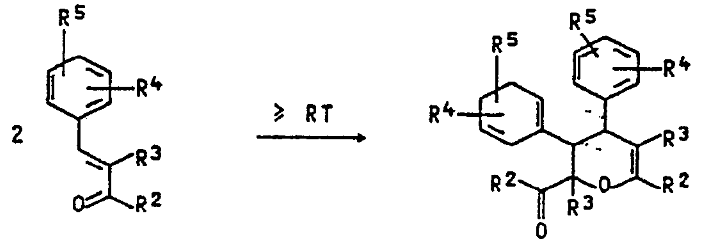


(III) monomer (IIIa) dimer

nach folgender Reaktionsgleichung

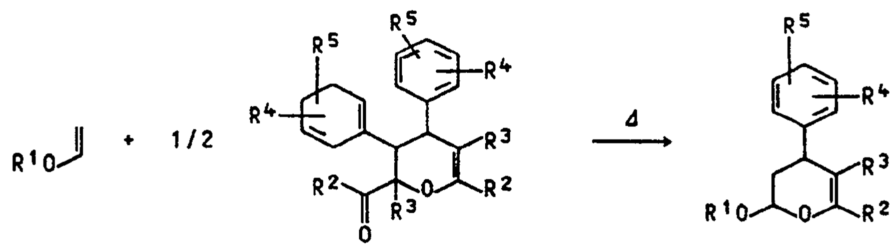


(II) (IIIa) (Ib)

In beiden Fällen ist die Reaktionsführung gleich. Die Reaktionen a) und b) werden in Gegenwart eines Radikalinhibitors und einer basischen Verbindung bei Temperaturen von 130 bis 280°C, vorzugsweise von 160 bis 220°C, besonders bevorzugt von 180 bis 210°C, bei Drücken von 1 bis 300 bar durchgeführt. Bei diesen Reaktionen wird unter Schutzgasatmosphäre gearbeitet. Als Schutzgase kommen beispielsweise Stickstoff und Argon in Betracht. Falls die Eduktkomponenten es zulassen (entsprechend hoher Siedepunkt), können die Reaktionen drucklos (bei Atmosphärendruck ; ca. 1 bar) durchgeführt werden. In allen anderen Fällen empfiehlt es sich, in einem Autoklaven unter Reaktionseigendruck von > 1 bis 300 bar zu arbeiten. Dabei bleibt die Summe des gewählten Vordrucks und des sich beim Aufheizen zusätzlich aufbauenden Eigendrucks im Bereich bis zu 300 bar.

Als Radikalinhibitoren eignen sich Phenole, bevorzugt werden Hydrochinon und Hydrochinonmonomethylether, besonders bevorzugt wird Hydrochinon.

Als basische Verbindungen eignen sich Natrium-, Kalium- und Calcium-carbonat, bevorzugt wird Natriumcarbonat.

Im allgemeinen arbeitet man ohne Lösungs- oder Verdünnungsmittel. Man kann die Umsetzungen aber auch in Lösungs- oder Verdünnungsmitteln durchführen. Hierzu eignen sich beispielsweise aromatische Kohlenwasserstoffe, wie Xylol oder Ether, wie Diphenylether.

Auch Gemische dieser Stoffe können als Lösungs- oder Verdünnungsmittel verwendet werden.

Zur Herstellung der erfindungsgemäßen Verbindungen Ib nach den voranstehend beschriebenen Methoden setzt man die Ausgangsstoffe II zu III im Molverhältnis von 0,8 : 1 bis 5 : 1, vorzugsweise 0,9 :

1 bis 2,5 : 1, besonders bevorzugt von 1 : 1 bis 1,5 : 1 ein. Entsprechendes gilt auch für die dimer vorliegenden Verbindungen IIIa

Die Verbindungen II sind an sich bekannt oder können nach üblichen Methoden (Annalen der Chemie, Band 601, 81-138 (1956)) hergestellt werden

Die Verbindungen III bzw. IIIa sind ebenfalls an sich bekannt oder können nach üblichen Methoden (Houben-Weyl, Methoden der organischen Chemie, Band VII/2b, 4. Auflage, 1976, Seiten 1457ff und 1482ff) erhalten werden

Die Umsetzung von II mit III bzw. IIIa zu Ib kann auch unter Lewissäure-Katalyse (DE-A-21 63 515) durchgeführt werden.

Die entstehenden Dihydropyrane sind biologisch wirksam, d.h. sie stellen Wirkstoffe dar und können ihrerseits zu Wirkstoffen der Formeln Ia, b, c umgesetzt werden :

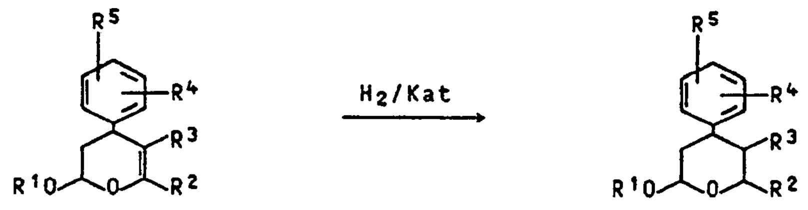


(Ia) (Ib)

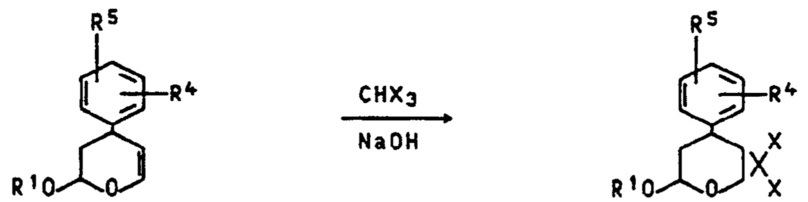


(Ib; $R^2$,$R^3$ = H) (Ic)

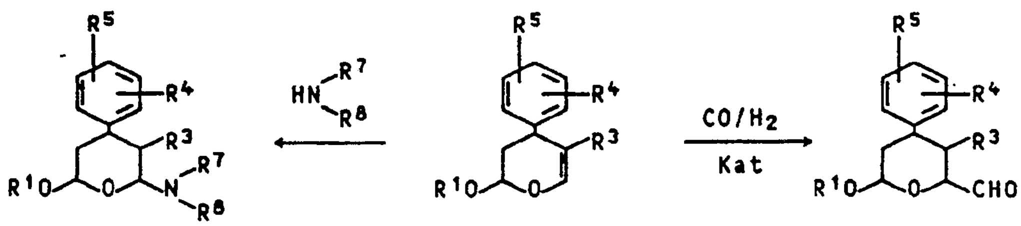


(Ia; $R^2$ = $-N(R^7)R^8$) (Ib; $R^2$ = H) (Ia; $R^2$ = CHO)

Aus den Aldehyden (Ia ; $R^2$ = CHO) können weiterhin ebenfalls als Wirkstoffe geeignete Verbindungen gewonnen werden, wie das nachstehende Schema zeigt, d.h. die erfindungsgemäßen Pyranderivate stellen vielseitig verwendbare Werkzeuge für den Synthetiker dar.

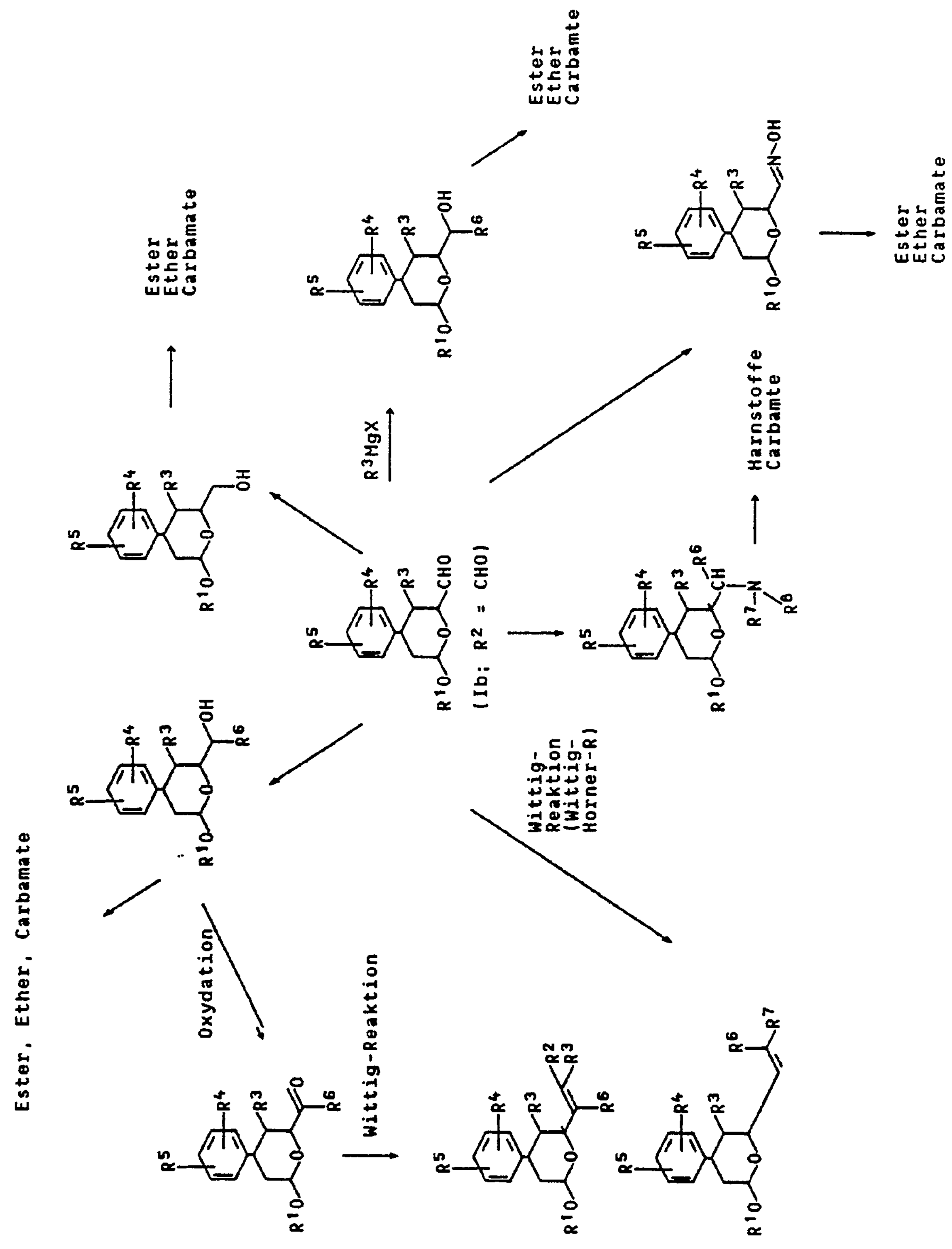


Unter den Substituenten sind folgende bevorzugt :

$R^1$ $C_1$-$C_{12}$-Alkyl, $C_2$-$C_{14}$-Alkenyl, $C_2$-$C_8$-Alkinyl, $C_3$-$C_8$-Cycloalkyl, Phenyl, Naphthyl, Biphenylyl, $C_7$-$C_{14}$-Phenylalkyl, $C_8$-$C_{14}$-Phenylalkenyl, $C_8$-$C_{14}$-Phenylalkinyl, $C_2$-$C_8$-Alkoxy-alkyl, $C_3$-$C_{12}$-Alkoxy-alkoxy-alkyl, $C_4$-$C_{16}$-Alkoxy-alkoxy-alkoxy-alkyl, durch $C_7$-$C_{10}$-Alkyl-aryl, $C_8$-$C_{14}$-Dialkyl-phenyl, $C_9$-$C_{14}$-Trialkyl-aryl, Phenoxy, Phenoxy-phenyl, $C_3$-$C_8$-Cycloalkyl, $C_4$-$C_{12}$-Alkyl-cycloalkyl, $C_4$-$C_8$-Cycloalkenyl oder $C_5$-$C_{12}$-Alkyl-cycloalkenyl substituiertes $C_1$-$C_8$-Alkyl, $C_4$-$C_{12}$-Alkyl-cycloalkyl, $C_9$-$C_{14}$-Phenyl-cycloalkyl, $C_9$-$C_{14}$-Phe-

noxy-cycloalkyl, $C_7$-$C_{14}$-Alkyl-phenyl, $C_7$-$C_{14}$-Alkoxy-phenyl, $C_8$-$C_{16}$-Dialkyl-phenyl, $C_7$-$C_{14}$-Halogenalkyl-phenyl, Halogenphenyl, Dihalogenphenyl, $C_7$-$C_{14}$-Alkyl-halogenphenyl, $C_8$-$C_{14}$-Dialkyl-halogenphenyl, Phenoxy-phenyl, $C_{13}$-$C_{20}$-Phenoxy-phenylalkyl, oder durch einen gesättigten oder ungesättigten 5- oder 6-gliedrigen Heterocyclus, mit einem ein oder zwei gleichen oder verschiedenen Heteroatomen aus der Gruppe bestehend aus Sauerstoff und Stickstoff, der gegebenenfalls durch ein, zwei oder drei gleiche oder unterschiedliche Substituenten aus der Gruppe bestehend aus $C_1$-$C_8$-Alkyl, Phenyl, $C_7$-$C_{14}$-Phenylalkyl, Halogenphenyl, $C_7$-$C_{14}$-Alkylphenyl, $C_8$-$C_{16}$-Dialkyl-phenyl, $C_9$-$C_{18}$-Trialkyl-aryl oder durch ein gegebenenfalls durch $C_1$-$C_4$-Alkyl substituiertes $C_3$-$C_8$-Spirocycloalkyl substituiert ist, substituiertes $C_1$-$C_8$-Alkyl,

$R^2$ Wasserstoff, $C_1$-$C_8$-Alkyl, $C_1$-$C_8$-Halogenalkyl, gegebenenfalls durch Phenyl oder Halogenphenyl substituiert ; $C_1$-$C_8$-Alkoxy, $C_1$-$C_8$-Halogenalkoxy, $C_2$-$C_8$-Alkenyloxy, $C_2$-$C_8$-Halogenalkenyloxy, $C_3$-$C_{12}$-Alkoxy-alkenyloxy, $C_2$-$C_8$-Alkinyloxy, $C_2$-$C_8$-Halogenalkinyloxy, $C_3$-$C_{12}$-Alkoxy-alkinyloxy, $C_8$-$C_{16}$-Phenyl-alkinyloxy, $C_1$-$C_8$-Alkylthio, $C_1$-$C_8$-Halogenalkylthio, $C_2$-$C_{12}$-Alkoxy-alkylthio, Formyl, Cyano, $C_7$-$C_{14}$-Phenoxy-alkoxy, $C_7$-$C_{14}$-Halogenphenoxy-alkoxy, $C_7$-$C_{14}$-Phenoxy-halogenalkoxy, $C_7$-$C_{14}$-Halogenphenoxy-halogenalkoxy, $C_8$-$C_{16}$-Alkoxyphenoxyalkoxy, $C_8$-$C_{16}$-Phenoxy-alkoxyalkoxy, $C_7$-$C_{16}$-Phenoxy-alkenyloxy, $C_8$-$C_{16}$-Halogenphenoxy-alkenyl, $C_8$-$C_{16}$-Phenoxy-halogenalkenyloxy, $C_8$-$C_{16}$-Halogenphenoxy-halogenalkenyloxy, $C_9$-$C_{16}$-Alkoxyphenoxy-alkenyloxy, $C_9$-$C_{16}$-Phenoxy-alkoxy-alkenyloxy, $C_8$-$C_{14}$-Phenoxy-alkinyloxy, $C_8$-$C_{14}$-Halogenphenoxy-alkinyloxy, $C_8$-$C_{14}$-Phenoxy-halogenalkinyloxy, $C_8$-$C_{14}$-Halogenphenoxy-halogenalkinyloxy, $C_9$-$C_{16}$-Alkoxyphenoxy-alkinyloxy, $C_9$-$C_{16}$-Phenoxy-alkoxyalkinyloxy, $C_8$-$C_{14}$-Phenylalkinyloxy, $C_{14}$-$C_{20}$-Phenoxyphenyl-alkinyloxy, Carboxy, $C_2$-$C_8$-Alkoxycarbonyl, $C_2$-$C_8$-Alkylcarbonyloxy, $C_3$-$C_{12}$-Alkenyloxycarbonyl, $C_3$-$C_{12}$-Alkenylcarbonyloxy, $C_3$-$C_{12}$-Alkinyloxycarbonyl, $C_3$-$C_{12}$-Alkinylcarbonyloxy, Phenoxycarbonyl, Phenylcarbonyloxy, $C_3$-$C_{12}$-Alkoxycarbonyl-alkoxy, Phenoxy, $C_7$-$C_{14}$-Alkylphenoxy, Halogenphenoxy, Dihalogenphenoxy, Trihalogenphenoxy, $C_7$-$C_{14}$-Halogenalkyl-phenoxy, $C_7$-$C_{14}$-Alkoxyphenoxy, Phenoxyphenoxy, $C_8$-$C_{14}$-Alkylendioxy-phenoxy, Phenylthio, $C_7$-$C_{14}$-Alkylphenylthio, Halogen-phenylthio, Dihalogen-phenylthio, Trihalogen-phenylthio, $C_7$-$C_{14}$-Halogenalkyl-phenylthio, $C_7$-$C_{14}$-Alkoxyphenylthio, Phenoxy-phenylthio, $C_8$-$C_{14}$-Alkylendioxy-phenylthio, Phenyl, Phenoxy-phenyl, $C_7$-$C_{14}$-Phenylalkyl, $C_{13}$-$C_{20}$-Phenoxy-phenylalkyl, $C_8$-$C_{14}$-Alkylendioxy-phenylalkyloxy oder einen Rest ausgewählt aus

$-CH=N-OH$ ; 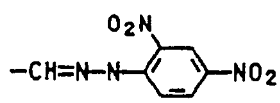 ; 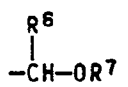 ; 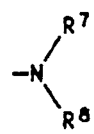 ;

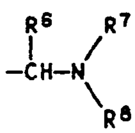 ; 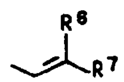 ; 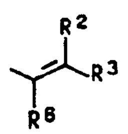 ; 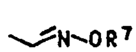 oder 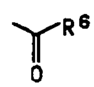

$R^3$ Wasserstoff, $C_1$-$C_8$-Alkyl oder $C_1$-$C_2$-Halogenalkyl,

$R^4$, $R^5$ Wasserstoff, Fluor, Chlor, $C_1$-$C_3$-Alkyl, $C_1$-$C_3$-Alkoxy oder $C_1$-$C_3$-Halogenalkoxy,

$R^6$ Wasserstoff, $C_1$-$C_8$-Alkyl, $C_2$-$C_8$-Alkenyl, $C_2$-$C_8$-Alkinyl, Cyano, Phenyl, Halogenphenyl, $C_7$-$C_{14}$-Alkylphenyl oder $C_7$-$C_{14}$-Alkoxyphenyl,

$R^7$ Wasserstoff, $C_1$-$C_8$-Alky $C_1$-$C_8$-Halogenalkyl, $C_2$-$C_8$-Alkoxy-alkyl, $C_2$-$C_8$-Alkenyl, $C_2$-$C_8$-Alkinyl, Aryl, Halogenphenyl, Dihalogenphenyl, Trihalogenphenyl, Cyanophenyl, $C_7$-$C_{14}$-Alkylphenyl, $C_7$-$C_{14}$-Alkoxyphenyl, $C_7$-$C_{14}$-Halogenalkyl-phenyl, $C_8$-$C_{16}$-Alkenylphenyl, $C_8$-$C_{16}$-Alkinylphenyl, Phenoxyphenyl, $C_8$-$C_{16}$-Methylendioxy-phenyl, $C_2$-$C_8$-Alkylcarbonyl, $C_2$-$C_8$-Halogenalkylcarbonyl, $C_2$-$C_8$-Alkoxycarbonyl, $C_5$-$C_{12}$-Alkylcyclopropyl-carbonyl, $C_7$-$C_{14}$-Alkenyl- und -alkylcyclopropyl-carbonyl, $C_6$-$C_{14}$-Halogenalkyl- und -alkylcyclopropyl-carbonyl, $C_7$-$C_{14}$-Halogenalkenyl- und -alkylcyclopropyl-carbonyl, $C_8$-$C_{16}$-Halogen- und -phenyl-alkylcarbonyl, $C_8$-$C_{16}$-Halogen- und -halogenphenylalkylcarbonyl, Benzoyl, Halogenbenzoyl, $C_8$-$C_{14}$-Alkylbenzoyl, $C_8$-$C_{14}$-Alkoxybenzoyl, $C_8$-$C_{14}$-Halogenalkyl-benzoyl, $C_8$-$C_{14}$-Halogenalkoxy-benzoyl, $C_8$-$C_{14}$-Phenylalkylcarbonyl, $C_8$-$C_{14}$-Halogenphenylalkylcarbonyl, $C_9$-$C_{16}$-Alkylphenylalkylcarbonyl, $C_9$-$C_{16}$-Alkoxyphenylalkylcarbonyl, $C_9$-$C_{16}$-Halogenalkylphenylalkylcarbonyl, $C_9$-$C_{16}$-Halogenalkoxyphenylalkylcarbonyl, $C_9$-$C_{16}$-Phenylalkenylcarbonyl, $C_9$-$C_{16}$-Halogenphenylalkenylcarbonyl, $C_{10}$-$C_{16}$-Alkylphenylalkenylcarbonyl, $C_{10}$-$C_{16}$-Alkoxyphenylalkenylcarbonyl, $C_{10}$-$C_{18}$-Halogenalkylphenylalkenylcarbonyl, $C_{10}$-$C_{16}$-Halogenalkoxyphenylalkenylcarbonyl, $C_9$-$C_{16}$-Phenylalkinylcarbonyl, $C_9$-$C_{16}$-Halogenphenylalkinylcarbonyl, $C_{10}$-$C_{16}$-Alkylphenylalkinylcarbonyl, $C_{10}$-$C_{16}$-Alkoxyphenylalkinylcarbonyl, $C_{10}$-$C_{16}$-Halogenalkylphenylalkinylcarbonyl, $C_{10}$-$C_{16}$-Halogenalkoxyphenylalkinylcarbonyl, $C_2$-$C_8$-Alkylaminocarbonyl, $C_2$-$C_8$-Halogenalkylaminocarbonyl, $C_3$-$C_{10}$-Alkylalkylaminocarbonyl, $C_3$-$C_{10}$-Alkoxyalkylamino-

carbonyl, $C_3$-$C_{10}$-Halogenalkylalkylaminocarbonyl, $C_3$-$C_{10}$-Halogenalkoxyalkylaminocarbonyl, $C_3$-$C_{10}$-Dialkylaminocarbonyl, $C_3$-$C_8$-Halogendialkylaminocarbonyl, $C_4$-$C_{12}$-Alkyldialkylaminocarbonyl, $C_4$-$C_{12}$-Alkoxydialkylaminocarbonyl, $C_5$-$C_{20}$-Alkyl- und -alkoxy-aminocarbonyl, $C_4$-$C_{12}$-Halogenalkyldialkylaminocarbonyl, $C_4$-$C_{12}$-Halogenalkoxydialkylaminocarbonyl, Phenylaminocarbonyl, Halogenphenylaminocarbonyl, Dihalogenphenylaminocarbonyl, Trihalogenphenylaminocarbonyl, $C_8$-$C_{14}$-Alkylphenylaminocarbonyl, $C_8$-$C_{14}$-Alkoxyphenylaminocarbonyl, $C_8$-$C_{14}$-Halogenalkoxyphenylaminocarbonyl oder gegebenenfalls durch $C_1$-$C_4$-Alkyl substituierte Isoxazole,

$R^8$ Wasserstoff oder $C_1$-$C_8$-Alkyl und

X Halogen,

mit der Maßgabe, daß in Formel Ia $R^1$ nicht Phenyl bedeutet, wenn $R^2$, $R^3$, $R^4$, $R^5$ gleichzeitig für Wasserstoff stehen, und in Formel Ib $R^2$, $R^3$, $R^4$, $R^5$ nicht gleichzeitig Wasserstoff bedeuten, wenn $R^1$ für $C_4$-$C_8$-Alkyl, Phenyl, Tolyl oder Methoxyphenyl steht, sowie $R^1$ nicht Octyl bedeutet, wenn $R^2$ und $R^3$ für Wasserstoff und $R^4$ oder $R^5$ für Methyl, Methoxy oder Chlor stehen, sowie ferner mit der Maßgabe, daß in Formel Ib $R^1$ nicht n-Butyl bedeutet, wenn $R^2$ für Methyl und $R^3$, $R^4$ und $R^5$ für Wasserstoff stehen.

Besonders bevorzugt sind die Substituenten mit folgender Bedeutung :

$R^1$ Methyl, Ethyl, n-Propyl, Isopropyl, n-Butyl, 2-Methylpropyl, n-Hexyl, n-Octyl, 2-Ethylhexyl, 3,7-Dimethyloctyl, n-Dodecyl, Ethenyl, Prop-2-en-1-yl, But-3-en-1-yl, But-3-en-2-yl, But-2-en-1-yl, 2,6-Dimethylhept-6-en-1-yl, 3,7-Dimethyloct-6-en-1-yl, 1,5,9-Trimethyldec-4-en-1-yl, 2,5,9-Trimethyldec-4-en-1-yl, Ethinyl, Prop-2-in-1-yl, But-3-in-1-yl, But-3-in-2-yl, But-2-in-1-yl, Cyclopropyl, Cyclobutyl, Cyclopentyl, Cyclohexyl, Cycloheptyl, Cyclooctyl, Phenyl, Naphthyl, p-Diphenyl, Benzyl, 2-Phenethyl, 2-Phenylpropyl, 3-Phenylpropyl, 3-Phenylbutyl, 4-Phenylbutyl, 2-Methyl-3-phenethyl, 3-Methyl-3-phenethyl, 2-Methyl-3-phenylpropyl, 4-Phenyl-but-3-in-1-yl, Methoxymethyl, Methoxyethyl, Ethoxymethyl, Ethoxyethyl, Propoxymethyl, Propoxyethyl, Methoxymethoxymethyl, Ethoxymethoxymethyl, Ethoxyethoxymethyl, Methoxyethoxymethyl, Ethoxyethoxyethyl, Butoxyethoxyethyl, Methoxymethoxymethoxymethyl, Methoxyethoxyethoxyethyl, Ethoxyethoxyethoxyethyl, Butoxyethoxyethoxyethyl, 2-Methyl-3-(4-tert.butylphenyl)-propyl, 2-(p-Methylphenyl)-ethyl, Phenoxymethyl, 2-(Phenoxyphenyl)-ethyl, Phenoxymethyl, Phenoxyethyl, Phenoxybutyl, Cyclopropylmethyl, 2-(Cyclopropyl)-ethyl, Cyclobutylmethyl, 2-(Cyclobutyl)-ethyl, Cyclopentylmethyl, 2-(Cyclopentyl)-ethyl, 2-(Cyclopentyl)-propyl, Cyclohexylmethyl, 2-Methyl-(2-cyclohexyl)-ethyl, 2-(Cyclohexyl)-propyl, 3-(4-Methylcyclohexyl)-butyl, 3-(4′-Methylcyclohex-4-en-1-yl)-butyl, 2-Methyl-2-(4′-chlorphenyl)-1,3-dioxolan-5-methyl, 2-(1′-Phenylethyl)-5-ethyl-1,3-dioxolan-5-methyl, 2-Phenyl-2-methyl-5-ethyl-1,3-dioxolan-5-methyl, 2-(2′,4′,4′-Trimethylphenyl)-1,3-dioxolan-5-methyl, 2-tert.-Butyl-1,3-dioxolan-5-methyl, 2-(2′-Methyl-1-3,-dioxolan-5-yl)-ethyl, 2-(1′-Phenethyl)-1,3-dioxolan-5-methyl, 2-(4′-Chlorphenyl)-1,3-dioxolan-5-methyl, 2-(4′-Chlorphenyl)-5-ethyl-1,3-dioxolan-5-methyl, 2-(2′,4′,4′-Trimethylpentyl)-1,3-dioxolan-5-methyl, 2-(Methyl-1,3-dioxolan-5-yl)-ethyl, 2,5-Dimethyl-1,3-dioxolan-5-methyl, 2-Phenyl-1,3-dioxolan-5-methyl, 2-(2-Phenethyl)-1,3-dioxolan-5-methyl, 2,2-Dimethyl-1,3-dioxolan-5-methyl, 2-Isopropyl-1,3-dioxolan-5-methyl, 2-Methyl-1,3-dioxolan-5-methyl, 2-Methyl-1,3-dioxolan-5-ethyl, 2-(2′-Methylpropyl)-1,3-dioxolan-5-methyl, 2-(2,5,5-Trimethylpentyl)-1,3-dioxolan-5-methyl, 2-Methyl-2-ethyl-1,3-dioxolan-5-methyl, 2,2-Diethyl-1,3-dioxolan-5-methyl, 2-Methyl-2-(4-chlorphenyl)-1,3-dioxolan-5-methyl, 2-Benzyl-1,3-dioxolan-5-methyl, 2-Phenyl-1,3-dioxolan-4-methyl, 2-Phenyl-1,3-dioxolan-5-methyl, 2-Methyl-1,4-dioxolan-3-methyl, 2-(1′-Phenylethyl)-5-ethyl-1,3-dioxan-5-methyl, 2-Methyl-2-phenyl-5-ethyl-1,3-dioxan-5-methyl, 2-Phenyl-5-ethyl-1,3-dioxan-5-methyl, 2-Methyl-5-ethyl-1,3-dioxan-5-methyl, 2-Methyl-1,3-dioxan-5-methyl, 2-Methyl-1,4-dioxan-3-methyl, 2,2-Spirocyclohexyl-1,3-dioxolan-4-methyl, 2,2-Spirocyclohexyl-1,3-dioxolan-5-methyl, 2,2-Spirocyclohexyl-5-ethyl-1,3-dioxolan-5-methyl, 2,2-Spirocyclopentyl-1,3-dioxolan-5-methyl, 2,2-Spirocyclohexyl-5-ethyl-1,3-dioxan-5-methyl, Furyl-2-methyl, Tetrahydrofur-2-yl-methyl, Tetrahydrofur-3-yl-methyl, 2-Methyl-tetrahydrofuran-3-methyl, 3-Methylcyclopentyl, 4-Methylcyclohexyl, 2-Phenylcyclohexyl, 4-Phenylcyclohexyl, 4-Phenoxycyclohexyl, 2-Methylphenyl, 4-Methylphenyl, 4-Ethylphenyl, 4-tert.-Butylphenyl, 4-(2′,2′,4′,4′-Tetramethylbutyl)-phenyl, (1,1,3,3-Tetramethylbutyl)-phenyl, 4-Methoxyphenyl, 4-Ethoxyphenyl, 2,4-Dimethylphenyl, 4-Trifluormethylphenyl, 3-(4′-Methylcyclohex-3-en-1-yl)-benzyl, 2-Chlorphenyl, 3-Chlorphenyl, 4-Chlorphenyl, 4-Fluorphenyl, 2,4-Dichlorphenyl, 2,6-Dichlorphenyl, 2,4-Difluorphenyl, 2,6-Difluorphenyl, 4-Chlor-2-methylphenyl, 4-Chlor-3-methylphenyl, 4-Chlor-3,5-dimethylphenyl, 3-Phenoxyphenyl, 4-Phenoxyphenyl oder 3-Phenoxybenzyl,

$R^2$ Wasserstoff, Methyl, Ethyl, n-Propyl, Isopropyl, n-Butyl, tert.-Butyl, Trifluormethyl, Trichlormethyl, 1,2-Dibrom-2-(4-chlorphenyl)-ethyl, Methoxy, Ethoxy, Prop-2-oxy, Trifluormethoxy, Trichlormethoxy, 1,3-Difluorprop-2-oxy, Butin-2-oxy, 4-Chlor-butin-1-oxy, Formyl, Cyano, 3-Phenyl-propin-2-oxy, 4-Phenyl-but-3-in-1-oxy, 4-(3-Chlorphenoxy)-but-2-in-1-oxy, 4-(4-Fluorphenoxy)-but-2-in-1-oxy, 1-(3-Phenoxyphenyl)-propinyloxy, Hydroxycarbonyl, Methoxycarbonyl, Ethoxycarbonyl, Methylcarbonyloxy, Ethylcarbonyloxy, Propionyloxy, Phenoxycarbonyl, Methoxycarbonylmethoxy, Ethoxycarbonylmethoxy, Methoxycarbonylethoxy, Ethoxycarbonylethoxy, Phenoxycarbonyl, Phenoxycarbonyloxy, Phenoxy, 4-Methylphenoxy, 4-Ethylphenoxy, 4-Chlorphenoxy, 4-Fluorphenoxy, 2,4-Difluorphenoxy, 2,4-Dichlorphenoxy, 4-Trifluormethylphenoxy, Phenyl, 3-Phenoxyphenyl, 4-Phenoxyphenyl, Benzyl, 3-Phenoxy-benzyl, 4-Phenoxy-benzyl, 3,4-Methylendioxybenzyloxy,

$-CH=N-OH$, 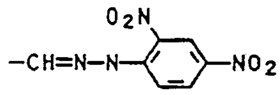, $CH_3-NH-CH_2-$ oder $NH_2-CH_2-$,

$R^3$ Wasserstoff, Methyl, Ethyl, n-Propyl, Isopropyl, n-Butyl, tert.-Butyl oder n-Hexyl,
$R^4$, $R^5$ Wasserstoff, Fluor, Chlor, Methyl, Methoxy oder Trifluormethyl,
$R^6$ Wasserstoff, Methyl, Ethyl, n-Propyl, Isopropyl, n-Butyl, tert.-Butyl, n-Hexyl, Ethenyl, Ethinyl oder Cyano,
$R^7$ Wasserstoff, Methyl, Ethyl, n-Propyl, Isopropyl, n-Butyl, tert.-Butyl oder n-Hexyl, Ethenyl, Ethinyl, Propinyl, Phenyl, 1-Naphthyl, 2-Naphthyl, 2-Fluorphenyl, 4-Fluorphenyl, 2-Chlorphenyl, 4-Chlorphenyl, 4-Cyanophenyl, 2-Methylphenyl, 4-Methylphenyl, 4-Methoxyphenyl, 4-Trifluormethylphenyl, Phenoxyphenyl, Acetyl, Propionyl, Methylpropionyl, Trifluormethylcarbonyl, Methoxycarbonyl, Methylcarbonyl, Ethoxycarbonyl, 2-Ethyl-cyclopropylcarbonyl, 2,2-Dimethyl-3-ethyl-cyclopropylcarbonyl, 2,2-Dimethyl-3-vinyl-cyclopropylcarbonyl, 2,2-Dimethyl-3-(2′,2′-dichlorvinyl)-cycloprop-1-yl-carbonyl, 2,2-Dimethyl-3-(2′,2′-dimethylvinyl)-cycloprop-1-yl-carbonyl, 2,2-Dimethyl-3-(1′,2′-dibrom-2′,2′-dichlorethyl)-cycloprop-1-yl-carbonyl, 2,2-Dimethyl-3-(1′,1′-dibrom-2′,2′-dichlorethyl)-cycloprop-1-yl-carbonyl, 2-Fluorbenzylcarbonyl, 2-Chlorbenzylcarbonyl, 4-Fluorbenzylcarbonyl, Benzoyl, Chloracetyl, 2-Chlor-6-fluorphenylacetyl, 4-Chlorphenyl-1-carbonyl, 4-Chlorbenzyl, Methylaminocarbonyl, Phenylaminocarbonyl, 4-Chlorphenylaminocarbonyl, 3,4-Dichlorphenylaminocarbonyl, 3-Methylphenylaminocarbonyl oder 3-Methyl-isoxazol-5-yl,
$R^8$ Wasserstoff, Methyl, Ethyl, n-Propyl, Isopropyl, n-Butyl, tert.-Butyl oder n-Hexyl und
X Fluor, Chlor oder Brom,
mit der Maßgabe, daß in Formel Ia $R^1$ nicht Phenyl bedeutet, wenn $R^2$, $R^3$, $R^4$, $R^5$ gleichzeitig für Wasserstoff stehen, und in Formel Ib $R^2$, $R^3$, $R^4$, $R^5$ nicht gleichzeitig Wasserstoff bedeuten, wenn $R^1$ für $C_4$-$C_8$-Alkyl, Phenyl, Tolyl oder Methoxyphenyl steht, sowie $R^1$ nicht Octyl bedeutet, wenn $R^2$ und $R^3$ für Wasserstoff und $R^4$ oder $R^5$ für Methyl, Methoxy oder Chlor stehen, sowie ferner mit der Maßgabe, daß in Formel Ib $R^1$ nicht n-Butyl bedeutet, wenn $R^2$ für Methyl und $R^3$, $R^4$ und $R^5$ für, Wasserstoff stehen.

Die Pyranderivate der allgemeinen Formeln Ia, Ib und Ic sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Im Gegensatz zu den meisten bisher bekannten Wirkstoffen, die als Kontakt- oder Fraßgifte die Tiere töten, lähmen oder vertreiben, griefen die meisten der Verbindungen der Formel I in das hormonale System des tierischen Organismus ein. Bei Insekten wird beispielsweise die Umwandlung zur Imago, die Ablage von entwicklungsfähigen Eiern und die Entwicklung von abgelegten normalen Eiern gestört und dadurch die Generationsfolge unterbrochen. Für Wirbeltiere sind die erfindungsgemäßen Wirkstoffe praktisch ungiftig. Die meisten der Verbindungen der Formeln Ia, Ib un Ic werden überdies leicht zu Stoffen abgebaut, die in der natürlichen Umgebung vorkommen und von Mikroorganismen weiter zerlegt werden.

Zu den schädlichen Insekten gehören aus der Ordnung der Schmetterlinge (Lepidoptera) beispielsweise Plutella maculipennis (Kohlschabe), Leucoptera coffeella (Kaffeemotte, hyponomeuta malinellus (Apfelbaumgespinstmotte), Argyresthia conjugella (Apfelmotte), Sitotroga cerealella (Getreidemotte), Phthorimaea operculella (Kartoffelmotte), Capua reticulana (Apfelschalenwickler), Sparganothis pilleriana (Springwurm), Cacoecia murinana (Tannentriebwickler), Tortrix viridana (Eichenwickler), Clysia ambiguella (Heu- und Sauerwurm), Evetria buoliana (Kieferntriebwickler), Polychrosis botrana (Bekreuzter Traubenwickler), Cydia pomonella (Obstmade), Laspeyresia molesta (Pfirsichtriebbohrer), Laspeyresia funebrana (Pflaumenwickler), Ostrinia nubilalis (Maiszünsler), Loxostege sticticalis (Rübenzünsler), Ephestia kuehniella (Mehlmotte), Chilo suppressalis (Reisstengelbohrer), Galleria mellonella (Wachsmotte), Malacosoma neustria (Ringelspinner), Dendrolimus pini (Kiefernspinner), Thaumatopoea ptiyocampa (Pinienprozessionsspinner), Cheimatobia brumata (Kleiner Frostspanner), Hibernia defoliaria (Großer Frostspanner), Bupalus piniarius (Kiefernspanner), Hyphantria cunea (Weißer Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe), Laphygma exigua (Rüben-Heerwurm), Panolis flammea (Forleule), Earias insulana (Baumwollkapselwurm), Plusia gamma (Gammaeule), Alabama argillacea (Baumwollblattwurm), Lymantria dispar (Schwammspinner), Lymantria monacha (Nonne), Pieris brassicae (Kohlweißling), Aporia

crataegi (Baumweißling) ;
aus der Ordnung der Käfer (Coleoptera) beispielsweise Blitophaga undata (Schwarzer Rübenaaskäfer), Melanotus communis (Drahtwurm), Limonius californicus (Drahtwurm), Agriotes lineatus (Saatschnellkäfer), Agriotes obscurus (Humusschnellkäfer), Meligethes aeneus (Rapsglanzkäfer), Atomaria linearis (Moosknopfkäfer), Epilachna varivestis (Mexikanischer Bohnenkäfer), Phyllopertha horticola (Junikäfer), Popillia japonica (Japankäfer), Melolontha melolontha (Feldmaikäfer), Melolontha hippocastani (Waldmaikäfer), Amphimallus solstitialis (Brachkäfer), Crioceris asparagai (Spargelhähnchen), Lema melanopus (Getreidehähnchen), Leptinotarsa decemlineata (Kartoffelkäfer), Phaedon cochleariae (Meerrettich-Blattkäfer), Phyllotreta nemorum (Kohlerdfloh), Chaetocnema tibialis (Rübenflohkäfer), Phylloides chrysocephala (Rape-Flohkäfer), Diabrotica 12-puncta (Südlicher Maiswurzelwurm), Cassida nebulosa (Nebliger Schildkäfer), Bruchus lentis (Linsenkäfer), Bruchus rufimanus (Pferdebohnenkäfer), Bruchus pisorum (Erbsenkäfer), Sitona lineatus (Liniierter Blattrandkäfer), Ortiorrhynchus sulcatus (Gefurchter Lappenrüßler), Otiorrhynchus ovatus (Erdbeerwurzelrüßler), Hylobius abietis (Großer Brauner Rüsselkäfer), Byctiscus betulae (Rebenstecher), Anthonomus pomorum (Apfelblütenstecher), Anthonomus grandis (Kapsel käfer), Ceuthorrhynchus assimilis (Kohlschotenrüßler), Ceuthorrynchus napi (Großer Kohltriebrüßler), Sitophilus granaria (Kornkäfer), Anisandrus dispar (Ungleicher Holzborkenkäfer), Ips typographus (Buchdrucker), Blastophagus piniperda (Gefurchter Waldgärtner) ;
aus der Ordnung der Zweiflügler (Diptera) beispielsweise Lycoria pectoralis, Mayetiola destructor (Hessenfliege), Basineura brassicae (Kohlschoten-Gallmücke), Contarinia tritici (Gelbe Weizen-Gallmücke), Haplodiplosis equestris (Sattelmücke), Tipula paludosa (Wiesenschnake), Tipula oleracea (Kohlschnake), Dacus cucurbitae (Melonenfliege), Dacus oleae (Olivenfliege), Ceratitis capitata (Mittelmeerfruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata (Brachfliege), Phorbia antiqua (Zwiebelfliege), Phorbia brassicae (Kleine Kohlfliege), Pegomya hysocyami (Rübenfliege), Anopheles maculipennis, Culex pipiens, Aedes aegypti (Gelbfiebermücke), Aedes vexans, Tabanus bovinus (Rinderbremse), Tipula paludosa (Wiesenschnake), Musca domestica (Stubenfliege), Fannia canicularis (Kleine Stubenfliege), Muscina stabulans, Glossina morsitans (Tsetse-Fliege), Oestrus ovis, Chrysomya macellaria, Chrysomya hominivorax, Lucilia cuprina, Lucilia sericata, Hypoderma lineata ;
aus der Ordnung der Hautflügler (Hymenoptera) beispielsweise Athalia rosae (Rübenblattwespe), Hoplocampa minuta (Pflaumensägewespe), Monomorium pharaonis (Pharaoameise), Solenopsis geminata (Feuerameise), Atta sexdens (Blattschneiderameise) ;
aus der Ordnung der Wanzen (Heteroptera) beispielsweise Nezara viridula (Grüne Reiswanze), Eurygaster integriceps (Asiatische Getreidewande), Blissus leucopterus (Chinch bug), Dysdercus cingulatus (Kapok-Wanze), Dysdercus intermedius (Baumwollwanze), Piesma quadrata (Rübenwanze), Lygus pratensis (Gemeine Wiesenwanze) ;
aus der Ordnung der Pflanzensauger (Homoptera) beispielsweise Perkinsiella saccharicida (Zuckerrohrzikade), Nilaparvata lugens (Braune Zikade), Empoasca fabae (Kartoffelzikade), Psylla mali (Apfelblattsauger), Psylla piri (Birnblattsauger), Trialeurodes vaporariorum (Weiße Fliege), Aphis fabae (Schwarze Bohnenlaus), Aphis pomi (Grüne Apfellaus), Aphis sambuci (Holunderblattlauf), Aphidula nasturtii (Kreuzdornblattlaus), Cerosipha gossypii (Gukrenblattlauf), Sappaphis mali (Rosige Apfellaus), Sappaphis mala (Mehlige Birnblattlaus), Dysphis radicola (Mehige Apfelfaltenlaus), Brachycaudus cardui (Große Pflaumenblattlaus), Brevicoryne brassicae (Kohlblattlaus), Phorodon humuli (Hopfenblattlaus), Rhopalomyzus ascalonicus (Zwiebellaus), Myzodes persicae (Grüne Pfirsichlaus), Myzus cerasi (Schwarze Sauerkirschenlaus), Dysaulacorthum pseudosolani (Gefleckte Kartoffellaus), Acyrthosiphon onobrychis (Grüne Erbsenalaus), Macrosiphon rosae (Große Rosenblattlaus), Megoura viciae (Wickenlaus), Schizoneura lanuginosa (Birnenblattlaus), Pemphigus bursarius (Salatwurzellaus), Dreyfusia nordmannianae (Tannentrieblaus), Dreyfusia piceae (Weißtannenstammlaus), Adelges laricis (Rote Fichtengallenlaus), Viteus vitifolii (Reblaus) ;
aus der Ordnung der Termiten (Isoptera) beispielsweise Reticulitermes lucifugus, Calotermes flavicollis, Leucotermes flavipes, Termes natalensis ;
aus der Ordnung der Geradflügler (Orthoptera) beispielsweise Forficula auricularia (Gemeiner Ohrwurm), Acheta domestica (Heimchen), Gryllotalpa gryllotalpa (Maulwurfsgrille), Tachycines asynamorus (Gewächshausschrecke), Locusta migratoria (Wanderheuschrecke), Stauronotus maroccanus (Marokkanische Wanderheuschrecke), Schistocerca peregrina (Wanderheuschrecke), Nomadacris septemfasciata (Wanderheuschrecke), Melanoplus spretus (Felsengebirgsheuschrecke), Melanoplus femur-rubrum (Rotbeinige Heuschrecke), Blatta orientalis (Küchenschabe), Blattella germanica (Deutsche Schabe), Periplaneta americana (Amerikanische Schabe), Blaberus giganteus (Riesenschabe).

Zur Klasse der Arachnoidea gehören Spinntentiere (Acarina) beispielsweise Ixodes ricinus (Holzbock),

Ornithodorus moubata, Amblyomma americanum, Dermacentor silvarum, Boophilus microplus, Tetranychus telarius, Tetranychus pacificus, Paratetranychus pilosus, Bryobia praetiosa.

Zur Klasse der Nematoden zählen beispielsweise Wurzelgallennematoden, z.B. Meloidogyne incognita, Meloidogyne hapla, Meloidogyne javanica, Zysten bildende Nematoden, z.B. Globodera rostochiensis, Heterodera schatii, Heterodera avenae, Hetrodera glycinae, Hetrodera triflolii, Stock- und Blattälchen, z.B. Ditylenchus dipsaci, Ditylenchus destructor, Pratylenchus neglectus, Pratylenchus penetrans, Partylenchus goodeyi, Paratylenchus curvitatus sowie Tylenchorhynchus dubius, Tylenchorhynchus claytoni, Rotylenchus robustus, Heliocotylenchus multicinctus, Radopholus similis, Belonolaimus longicaudatus, Longidorus elongatus, Trichodorus primitivus.

Die Wirkstoffe können als solche, in Form ihrer Formulierungen oder den daraus bereiteten Anwendungsmöglichkeiten, z.B. in Form direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken ; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können als Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkali, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Aklylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolylglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Beispiele für Formulierungen sind :

I. 5 Gew.-Teile der Verbindung Nr. 98 werden mit 95 Gew.-Teilen feinteiligem Kaolin innig vermischt. Man erhält auf diese Weise ein Stäubemittel, das 3 Gew.-% des Wirkstoffs enthält.

II. 30 Gew.-Teile der Verbindung Nr. 179 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 10 Gew.-Teile der Verbindung Nr. 400 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht.

IV. 20 Gew.-Teile der Verbindung Nr. 177 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht.

V. 80 Gew.-Teile der Verbindung Nr. 214 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer

Sulfit-Ablauge und 7 Gew.-Teilen pulverförmigem Kieselsäuregel gut vermischt und in einer Hammermühle vermahlen.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.-% Wirkstoff, vorzugsweise zwischen 0,5 und 90 Gew.-%.

Die Wirkstoffkonzentrationen in den anwendungsfertigen Zubereitungen können in größeren Bereichen variiert werden.

Im allgemeinen liegen sie zwischen 0,0001 und 10%, vorzugsweise zwischen 0,01 und 1%. Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Die Aufwandmenge an Wirkstoff beträgt unter Freilandbedingungen 0,02 bis 10, vorzugsweise 0,08 bis 2 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1 : 10 bis 10 : 1 zugemischt werden.

Beispielsweise können folgende Mittel zugemischt werden : 1,2-Dibrom-3-chlorpropan, 1,3-Dichlorpropen, 1,3-Dichlorpropen + 1,2-Dichlorpropan, 1,2-Dibromethan, 2-sec.-Butyl-phenyl-N-methylcarbamat, o-Chlorphenyl-N-methylcarbamat, 3-Isopropyl-5-methyphenyl-N-methylcarbamat, o-Isopropoxyphenyl-N-methylcarbamat, 3,5-Dimethyl-4-methylmercapto-phenyl-N-methylcarbamat, 4-Dimethylamino-3,5-xylyl-N-methylcarbamat, 2-(1,3-Dioxolan-2-yl)-phenyl-N-methylcarbamat, 1-Naphthyl-N-methylcarbamat, 2,3-Dihydro-2,2-dimethyl-benzofuran-7-yl-N-methylcarbamat, 2,2-Dimethyl-1,3-benzodioxol-4-yl-N-methylcarbamat, 2-Dimethylamino-5,6-dimethyl-4-pyrimidinyl-dimethylcarbamat, 2-Methyl-2-(methylthio)-propionaldehyd-O-(methylcarbamoyl)-oxim, S-Methyl-N[(methylcarbamoyl)-oxyl]-thio-acetimidat, Methyl-N′,N′-dimethyl-N-[(methylcarbamoyl)oxy]-1-thiooxamidat, N-(2-Methylchlorphenyl)-N′,N′-dimethylformamidin, Tetrachlorthiophen, 1-(2,6-Difluor-benzoyl)-3-(4-chlorphenyl)-harnstoff, O,O-Dimethyl-O-(p-nitrophenyl)-phosphorthioat, O,O-Diethyl-O-(p-nitrophenyl)-phosphorthioat, O-Ethyl-O-(p-nitrophenyl)-phenyl-phosphonothioat, O,O-Dimethyl-O-(3-methyl-4-nitrophenyl)-phosphorthioat, O,O-Diethyl-O-(2,4-dichlorphenyl)-phosphorthioat, O-Ethyl-O-(2,4-dichlorphenyl)-phenyl-phosphonothioat, O,O-Dimethyl-O-(2,4,5-trichlorphenyl)-phosphorthioat, O-Ethyl-O-(2,4,5-trichlorphenyl)-ethyl-phosphorthioat, O,O-Dimethyl-O-(4-brom-2,5-dichlorphenyl)-phosphorthioat, O,O-Dimethyl-O-(2,5-dichlor-4-jodphenyl)-phosphorthioat, O,O-Dimethyl-O-(3-methyl-4-methylthiophenyl)-phosphorthioat, O-Ethyl-O-(3-methyl-4-methylthiophenyl)-isopropyl-phosphoramidat, O,O-Diethyl-O-(p-methylsulfinyl-phenyl)-phosphorthioat, O-Ethyl-S-phenylethyl-phosphonodithioat, O,O-Diethyl-[2-chlor-1-(2,4-dichlorphenyl)-vinyl]-phosphat, O,O-Dimethyl-[2-chlor-1-(2,4,5-trichlorphenyl)]-vinylphosphat, O,O-Dimethyl-S-(1′-phenyl)-ethylacetat-phosphordithioat, Bis-(dimethylamino)-fluorphosphinoxid, Octamethyl-pyrophosphoramid, O,O,O,O-Tetraethyldithio-pyrophosphat, S-Chlormethyl-O,O-diethyl-phosphordithioat, O-Ethyl-S,S-dipropyl-phosphordithioat, O,O-Dimethyl-O-2,2-dichlorvinyl-phosphat, O,O-Dimethyl-1,2-dibrom-2,2-dichlorethylphosphat, O,O-Dimethyl-2,2,2-trichlor-1-hydroxy-ethylphosphonat, O,O-Dimethyl-S-[1,2-biscarbethoxy-ethyl-(1)]-phosphordithioat, O,O-Dimethyl-O-(1-methyl-2-carbmethoxy-vinyl)-phosphat, O,O-Dimethyl-S-(N-methyl-carbamoyl-methyl)-phosphordithioat, O,O-Dimethyl-S-(N-methylcarbamoyl-methyl)-phosphorthioat, O,O-Dimethyl-S-(N-methoxyethyl-carbamoylmethyl)-phosphordithioat, O,O-Dimethyl-S-(N-formyl-N-methyl-carbamoyl-methyl)-phosphordithioat, O,O-Dimethyl-O-[1-methyl-2-(methyl-carbamoyl)-vinyl]-phosphat, O,O-Dimethyl-O-[(1-methyl-2-dimethylcarbamoyl)-vinyl]-phosphat, O,O-Dimethyl-O-[(1-methyl-2-chlor-2-diethylcarbamoyl)-vinyl]-phosphat, O,O-Diethyl-S-(ethylthiomethyl)-phosphordithioat, O,O-Diethyl-S[(p-chlorphenylthio)-methyl]-phosphordithioat, O,O-Dimethyl-S-(2-ethylthioethyl)-phosphorthioat, O,O-Dimethyl-S-(2-ethylthioethyl)-phosphordithioat, O,O-Dimethyl-S-(2-ethylsulfinyl-ethyl)-phosphorthioat, O,O-Diethyl-S-(2-ethylthio-ethyl)-phosphordithioat, O,O-Diethyl-S-(2-ethylsulfinylethyl)-phosphorthioat, O,O-Diethyl-thiophosphoryliminophenyl)-acetonitril,

O,O-Diethyl-S-(2-chlor-1-phthalimidoethyl)-phosphordithioat, O,O-Diethyl-S-[6-chlor-benzoxazolon-(2)-yl(3)]-methyldithiophosphat, O,O-Dimethyl-S-[2-methoxy-1,3,4-thiadiazol-5-[4H]-onyl-(4)-methyl]-phosphordithioat, O,O-Diethyl-O-[3,5,6-trichlor-pyridyl-(2)]-phosphorthioat, O,O-Diethyl-O-(2-pyrazinyl)-phosphorthioat, O,O-Diethyl-O-[2-isopropyl-4-methylpyrimidinyl(6)]-phosphorthioat, O,O-Diethyl-O-[2-(diethylamino)-6-methyl-4-pyrimidinyl]-thionophosphat, O,O-Dimethyl-S-(4-oxo-1,2,3-benzotriazin-3-[4H]-yl-methyl)-phosphordithioat, O,O-Dimethyl-S-[(4,6-diamino-1,3,5-triazin-2-yl)-methyl]-phosphordithioat, O,O-Diethyl-(1-phenyl-1,2,4-triazol-3-yl)-thionophosphat, O,S-Dimethyl-phosphor-amidothioat, O,S-Dimethyl-N-acetyl-phosphoramidothioat, alpha-Hexachlorcyclohexan, 1,1-Di-(p-methoxyphenyl)-2,2,2-trichlor-ethan, 6,7,8,9,10,10-Hexachloro-1,5,5a,6,9,9a-hexahydro-6,9-methano-2,4,3-benzodioxa-thiepin-3-oxid, Pyrethrine, DL-2-Allyl-3-methyl-cyclopenten-(2)-on-(1)-yl-(4)-DL-cis,-trans-chrysanthemat, 5-Benzyl-furyl-(3)-methyl-DL-cis,-trans-chrysanthemat, 3-Phenoxybenzyl(±)-cis,-trans-2,2-dimethyl-3-(2,2-dichlorvinyl)-cyclopropancarboxylat, alpha-Cyano-3-phenoxybenzyl(±)-cis,-trans-2,2-dimethyl-3-(2,2-dichlorvinyl)-cyclopropancarboxylat, (s)-alpha-Cyano-3-phenoxybenzyl-cis(1R,3R)-2,2-dimethyl-3-(2,2-dibromvinyl)-cyclopropancarboxylat, 3,4,5,6-Tetrahydrophthalimidoethyl-DL-cis,-trans-chrysanthemat, 2-Methyl-5-(2-propinyl)-3-furylmethyl-chrysanthemat, (alpha-Cyano-3-phenoxybenzyl)-alpha-isopropyl-4-chlorphenylacetat.

Herstellungsbeispiele

Beispiel 1

2-Methyl-4-phenyl-6-p-chlorphenoxy-5,6-dihydropyran

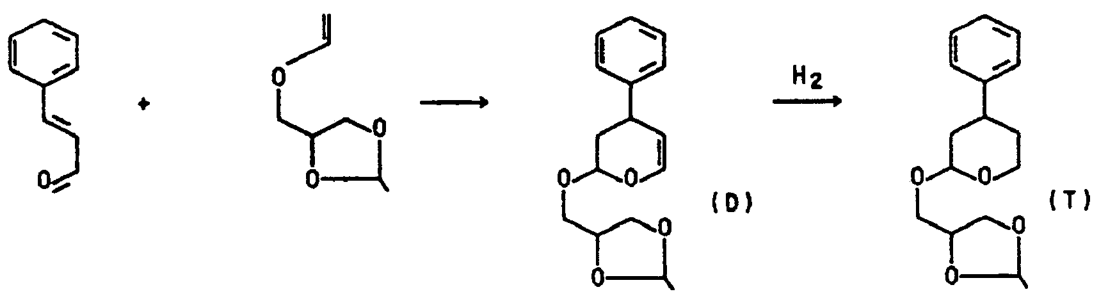


In einem Vierhalskolben, der mit Rührer, Thermometer, Rückflußkühler und Gaseinleitungsrohr ausgestattet ist, wird unter Stickstoff die Cycloaddition durchgeführt. Im 2-l-Kolben werden 2 Mol 4-Chlorphenyl-vinylether (309 g) und 2 Mol Benzalaceton (292 g) eingefüllt. Als Polymerisationsstabilisator wird 1 g Hydrochinon und zum Abfangen eventuell auftretender acider Verbindungen 0,5 g Natriumcarbonat zugegeben. Das Reaktionsgemisch wird unter Rühren auf 200°C aufgeheizt. Nach jeweils 6 Stunden Reaktionszeit werden Proben zur GC-Bestimmung aus dem Kolben entnommen.

Der Umsatz beträgt nach

2 Stunden 4,5%
8 Stunden 13,7%
16 Stunden 24,9%
22 Stunden 30,8%
28 Stunden 39,0%
34 Stunden 44,1%

Danach wird das Reaktionsgemisch durch Fraktionierung bei einem Druck von 2 mbar aufgetrennt.

Es werden erhalten :

bis zu einer Übergangstemperatur/bei einer Sumpftemperatur

| | |
|---|---|
| 1) 140°C/184°C | 197 g |
| 2) 170°C/188°C | 12 g |
| 3) 178°C/200°C | 103 g |
| 4) 185°C/214°C | 43 g |
| 5) 195°C/290°C | 34 g |
| Dest. Rückstand | 116 g |

Fraktion 1 besteht aus den beiden Einsatzstoffen.

Fraktion 2 enthält ca. 50% Cycloadditionsprodukt + Einsatzstoffe.

Fraktion 3 und 4 bestehen aus über 97,5% aus Cycloadditionsprodukt.

Fraktion 5 enthält 78% erwünschtes Reaktionsprodukt, neben Zersetzungsanteilen.

Die H-NMR-Analyse zeigt, daß das Cycloadditionsprodukt zu 2/3 aus der trans-Verbindung und zu etwa einem Drittel aus der cis-Verbindung besteht. Im Verlauf von mehrwöchigem Stehen kristallisieren 3 und 4 aus. Aus Methanol unkristallisiert wird ein Schmelzpunkt von 46-64° ermittelt. Eine Auftrennung der beiden stereoisomeren Formen konnte nicht erreicht werden.

Beispiel 2

4-Phenyl-2-(2′-methyl-1′,3′-dioxonlanyl-4′-methyl)-tetrahydropyran

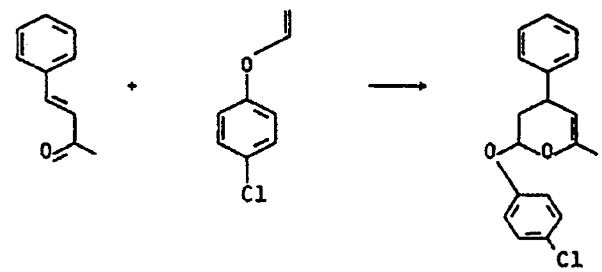

35 g des Cycloadditionsproduktes aus Zimtaldehyd und 2-Methyl-4-vinoxylmethyl-1,3-dioxolan(4-phenyl-6-(2′-methyl-1′,3′-dioxolanyl-4-methyl)-5,6-dihydropyran) werden in 90 g Tetrahydrofuran gelöst und als Hydrierkatalysator 1 g Pallidium-Kohle-Katalysator (10% Pd auf Kohle) zugegeben. Die Hydrierung wird im Autoklaven (300 ml) unter Rühren bei 40°C unter einem Wasserstoffdruck von 40 bar durchgeführt. Die Wasserstoffaufnahme - 25 bar - wird in einer Reaktionszeit von 5 Stunden ermittelt.

Nach Beendigung der Wasserstoffaufnahme wird der Autoklav abgekühlt und die Restwasserstoffmenge abgegast. Der suspendierte Katalysator wird abfiltriert und das Lösungsmittel abgezogen. Es bleibt ein farbloser Sirup zurück. Die NMR- und IR-Spektren zeigen das Vorliegen der Tetrahydropyranverbindung (T). Nach der NMR-Analyse liegen 60% der Substitution am Tetrahydropyranring in trans-Form, der Rest als cis-Form vor.

Beispiel 3

4-Phenyl-6-(2′-methyl-1′,3′-dioxolanyl-4′-methyl)-5,6-dihydropyran

Ein Gemisch aus 792 g (6 Mol) Zimtaldehyd und 889 g 2-Methyl-4-vinyloxymethyl-1,3-dioxolan sowie 1 g Natriumcarbonat und 2 g Hydrochinon werden in einem 3-l-Autoklaven unter Stickstoff 24 Stunden auf 190°C erhitzt. Das Reaktionsgemisch wird durch Kurzwegfraktionierung gereinigt. Bei 1 mbar gehen zwischen 142-198°C 963 g über 97%iges D über. Im Vorlauf sind weitere 112 g und im Nachlauf weitere 156 g D vorhanden. Der Destillationsrückstand beträgt 90 g. Die Selektivitäten liegen, bezogen auf Zimtaldehyd und Vinylether, bei 73,8% bzw. 71,2%. Dabei wurden die im Vorlauf noch vorhandenen Anteile an Aldehyd und Vinylether nicht berücksichtigt.

Beispiel 4

2-Formyl-4-phenyl-6(p-chlor-phenoxy)-tetrahydropyran

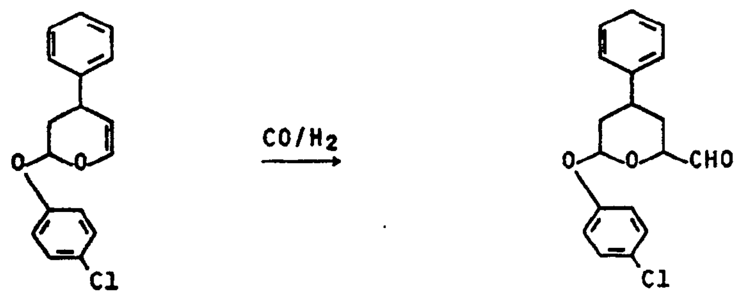


250 g trans-4-Phenyl-6-p-chlorphenoxy-5,6-dihydro-4H-pyran (hergestellt aus Zimtaldehyd und p-Chlorphenyl-vinylether, gereinigt durch Umkristallisation aus Ethanol Fp = 98°C) werden in 3500 g Toluol gelöst. Als Katalysator für die Umsetzung mit Kohlenoxyd und Wasserstoff (Hydroformylierung) werden zu dieser Lösung 250 mg Rhodium-cyclooctadienyl-chlorid zugegeben.

Das Reaktionsgemisch wird in einen 10-l-Autoklaven eingefüllt. Der Autoklav wird mit Stickstoff gespült und bei Raumtemperatur ein Gasgemisch aus gleichen Teilen Kohlenmonoxid und Wasserstoff im Volumenverhältnis 1 : 1 bis zu einem Druck von 50 bar aufgepreßt. Danach wird das Rührwerk des Autoklaven in Gang gesetzt (Magnethubrührung) und durch Aufheizen des Autoklaven eine Inntentemperatur von 100°C eingestellt. Nach Erreichen der Reaktionstemperatur wird der Druck des Gasgemisches auf 650 bar gebracht. Unter diesen Reaktionsbedingungen wird die Hydroformylierungsreaktion im Verlauf von 18 Stunden fast vollständig erzielt. Anschließend wird nach Abschalten der Heizung und Abkühlen auf 40°C das noch im Autoklaven verbliebene Restgas entspannt.

Das Reaktionsgemisch wird durch Abdestillieren des Lösungsmittels (Toluol) aufkonzentriert. Es bleibt ein zähflüssiger Sirup zurück, der bei längerem Stehen partiell kristallisiert. Das NMR-Spektrum und auch das IR-Spektrum zeigen, daß praktisch kein Olefin mehr vorhanden ist. Das Aldehydproton liefert ein Signal bei 9,6 ppm ; die CO-Bande liegt bei 1737. Die im Rohprodukt verbleibenden Anteile an Katalysator (Rhodiumkomplexe) stören die weiteren Umsetzungen nicht.

Beispiel 5

2-Aminomethyl-4-phenyl-6-p-chlorphenoxy-5,6-tetrahydropyran

In einem 5-l-Magnethubrührautoklaven werden 500 g Ethanol und 50 g Raney-Kobalt vorgelegt. Der Autoklav wird nach der Probe auf Druckdichtigkeit mit Stickstoff gespült. Anschließend werden 500 g Ammoniak eingespreßt. Der Autoklav wird auf eine Temperatur von 100°C aufgeheizt und durch Einpressen von Wasserstoff ein Druck von 150 bar eingestellt.

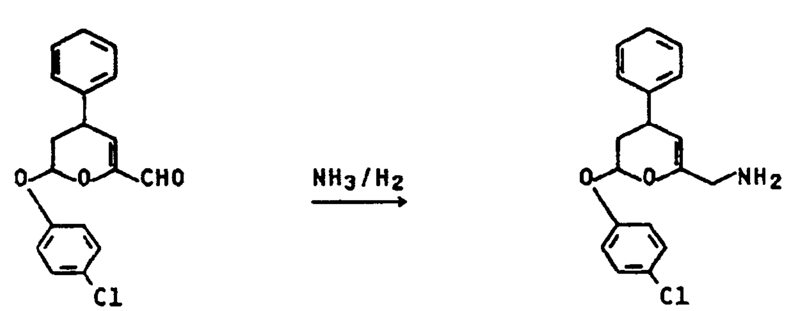


Im Verlauf von 6 Stunden wird unter Rühren des Reaktionsgemisches eine Lösung von 200 g 2-Formyl-4-phenyl-6-p-chlorphenoxy-tetrahydropyran in 400 g THF in den Autoklaven eingepumpt. Zum Spülen der Einpreßleitung werden 100 g Ethanol nachgepumpt. Nach Beendigung des Zupumpens wird das Reaktionsgemisch im Autoklaven noch weitere 6 Stunden unter den genannten Reaktionsbedingungen gehalten.

Nach Ausschalten der Autoklavenheizung und Abkühlen auf 40°C wird der noch im Autoklaven vorhandene Wasserstoff entspannt ; dabei entweicht auch ein Teil des Ammoniaküberschusses. Danach wird das flüssige Reaktionsprodukt aus dem Autoklaven entnommen. In einer Destillationsapparatur wird unter vermindertem Druck Ammoniak, THF und Ethanol abgetrennt und anschließend das bei der aminierenden Hydrierung gebildete Wasser abdestilliert bis zu einer Sumpftemperatur von 80°C bei einem Druck von 0,05 bar. Es werden 218 g des Rohamins erhalten. NMR- und IR-Spektren zeigen das Vorliegen eines ca. 75%igen Amins.

Beispiel 6

Herstellung des Hydrochlorids von 2-Aminomethyl-4-phenyl-6-p-chlorphenoxy-tetrahydropyran

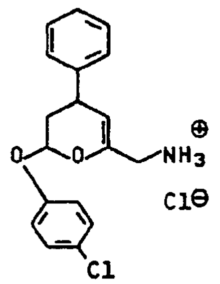

40 g des nach Beispiel 5 erhaltenen Amins werden in 50 g Ethanol gelöst und mit einer Chlorwasserstofflösung in Ethanol neutralisiert. Beim Stehen über Nacht scheiden sich 14 g gut kristallisiertes Hydrochlorid des Amins als einheitliches Kristallisat ab. Fp. 160°C. NMR- und IR-Spektren bestätigen das Vorliegen des Salzes.

Beispiel 7

2-(3'-Fluorphenoxy)-4-phenyl-6-(4-chlorphenoxy)tetrahydropyran

14,3 Teile 2-(4-Chlorphenoxy)-4-phenyl-2,3-dihydropyran werden in 60 Teilen Dioxan mit 5,6 Teilen 3-Fluorphenol und einem Kristall p-Toluolsulfonsäure versetzt und 12 Stunden bei Raumtemperatur gerührt.

Danach wird mit Natriumcarbonat neutralisiert, filtriert und vom Lösungsmittel befreit. Der erhaltene Rückstand (20,2 Teile) wird über neutrales Kieselgel mit Toluol gereinigt.

200-MHZ-NMR-Spektrum in $CDCl_3$ (ppm) :
1,9-2.4 (4H) ; 3,4-4,0 (1H) ; 5,55-5,9 (1H) ; 5,9-6,0 (1H) ; 6,4-7,5 (13H).

Beispiel 8

2-(4'-Chlorphenoxy)-4-phenyl-6-(2'-hydroxyprop-2'-enyl)-tetrahydropyran

70,6 Volumenteile einer 1,7-molaren Vinylmagnesiumchloridlösung in THF werden bei 10-20°C zu einer Lösung von 31,7 Teilen 2-(4'-Chlorphenoxy)-4-phenyl-6-formyltetrahydropyran in 80 Teilen THF zugetropft. Danach wird 8 Stunden bei Raumtemperatur nachgerührt, mit Eiswasser zersetzt und mit HCl angesäuert. Nach Extraktion mit Ether wird mit die organische Masse mit Natriumsulfat getrocknet und vom Lösungsmittel befreit. Als Rückstand (33 Teile) erhält man ein hellgelbes Öl, das über Kieselgel gereinigt werden kann.

300-MHZ-NMR-Spektrum in $CDCl_3$ (ppm) :
1,6-2,5 (2H) ; 3,2-3,8 (1H) ; 3,65-4,4 (1H) ; 3,9 (OH) ; 4,95-5,9 (4H) ; 6,9-7,1 (2H) ; 7,2-7,6 (7H).

Beispiel 9

2-(4'-Chlorphenoxy)-4-phenyl-6-(1'-phenylaminocarboxyprop-2'-enyl)-tetrahydropyran

6,9 Teile 2-(4'-Chlorphenoxy)-4-phenyl-6-(1'-hydroxyprop-2'-enyl)-tetrahydropyran in 100 Teilen Toluol werden mit 2,4 Teilen Phenylisocyanat und 3 Tropfen Triethylamin versetzt und 5 Stunden bei 60°C gerührt. Danach wird von Lösungsmittel befreit und 9 Teile eines hellgelben Öls erhalten, das über Kieselgel mit Toluol gereinigt werden kann.

300-MHZ-NMR-Spektrum in $CDCl_3$ (ppm) :
1,6-2,3 (2H) ; 3,2-3,6 (1H) ; 3,7-4,3 (1H) ; 5,0-5,4 (1H) ; 5,3-5,9 (3H) ; 6,9-7,1 (2H) ; 7,1-7,6 (12H).

Beispiel 10

2-Methylpropionsäure-1[2′(4″-chlorphenoxy)-4′-phenyl-tetrahydropyran-6-yl]-prop-2-enylester

6,3 Teile 2-(4′-Chlorphenoxy)-4-phenyl-6-(1′-hydroxyprop-2-enyl)tetrahydropyran in 100 Teilen Toluol werden mit 3 Teilen Triethylamin und danach tropfenweise bei 10-20°C mit 2,3 Teilen 2-Methylpropionsäurechlorid versetzt. Nach Rühren bei Raumtemperatur wird gut mit Wasser gewaschen, über Natriumsulfat getrocknet und eingeengt. Man erhält 6,9 Teile eines hellgelben Öls.

300-MHZ-NMR-Spektrum in $CDCl_3$ (ppm) :
1,0-1,4 (2H) ; 1,8-2,2 (2H) ; 2,4-2,8 (1H) ; 3,2-3,6 (1H) ; 3,7-4,05 (1H) ; 5,0-5,9 (4H) ; 6,8-7,1 (2H) ; 7,1-7,4 (7H).

Beispiel 11

2-(4′-Chlorphenoxy)-4-phenyl-6-[2′(4-cyanophenyl)vinyl]-tetrahydropyran

3,6 Teile Natriumhydrid (80%ig in Paraffin) werden in 100 Teilen Dimethylsulfoxid (DMSO) vorgelegt und bei 10-15°C mit einer Lösung von 25,3 Teilen 4-Cyanobenzylphosphonsäure-diethylester in 100 Teilen DMSO tropfenweise versetzt. Es wird 1 Stunde bei 50°C nachgerührt und danach mit einer Lösung von 31,7 Teilen 2-(4-Chlorphenoxy)-4-phenyl-6-formyltetrahydropyran in 150 ml DMSO versetzt. Nach fünfstündigem Rühren bei 50°C wird auf Wasser gegossen und mit HCl angesäuert. Der ausgefallene Niederschlag wird abgesaugt und mit Wasser und Petrolether gewaschen. Man erhält 38,5 g einer kristallinen Substanz vom Fp. 134°C.

Die in den nachstehenden Tabellen aufgeführten Stoffe wurden sämtlich hergestellt unter entsprechener Abwandlung der Angaben der vorstehenden Beispiele. Da es sich bei den Verbindungen der Tabellen 1 bis 4 in der Regel um Gemische von Stereoisomeren handelt (es sind i.a. mehrere Asymmetriezentren vorhanden), treten keine scharfen Schmelzpunkte auf, sondern es handelt sich i.a. um ölige Flüssigkeiten; die Struktur der Verbindungen wurde jeweils mit Hilfe von IR- und NMR-Messungen gesichert.

Tabelle 1

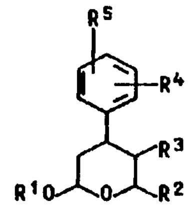

(Ia)

| Nr. | $R^1$ | $R^2$ | $R^3$ | $R^4$ | $R^5$ | $^1H$-NMR [ppm] in $CDCl_3$ | IR [$cm^{-1}$] |
|---|---|---|---|---|---|---|---|
| 1 | Cyclohexyl | $NH_2-CH_2-$ | H | H | H | | |
| 2 | Cyclohexyl | $CH_3NH-CH_2-$ | H | H | H | 5,1 (s) Acetal | |
| 3 | Tetrahydro-fur--2-yl-methyl | $CH_3NH-CH_2-$ | H | H | H | 5,0 (m) Acetal | |
| 4 | Tetrahydro-fur--2-yl-methyl | $NH_2-CH_2-$ | H | H | H | 5,1 (m) Acetal | |
| 5 | 2-Phenylpropyl | $CH_3NH-CH_2-$ | H | H | H | 4,9 (m) Acetal | |
| 6 | 2-Phenylpropyl | $NH_2-CH_2-$ | H | H | H | 4,9 (m) Acetal | |
| 7 | 2-Cyclohexylpropyl | $CH_3NH-CH_2-$ | H | H | H | 4,9 (m) Acetal | |
| 8 | 2-Cyclohexylpropyl | $NH_2-CH_2-$ | H | H | H | | |
| 9 | 3-Phenylbutyl | $CH_3NH-CH_2-$ | H | H | H | | |
| 10 | 3-Phenylbutyl | $NH_2-CH_2-$ | H | H | H | | |
| 11 | 2-Methyl-1,3-dioxolan-5-methyl | $CH_3NH-CH_2-$ | H | H | H | | 1124,1066,700 |
| 12 | 2-Methyl-1,3-dioxolan-5-methyl | $NH_2-CH_2-$ | H | H | H | | 3384,1150,1066,701 |
| 13 | 2-(2-Methyl-1,3-dioxolan-5)-ethyl | $CH_3-NH_2-$ | H | H | H | 5,0 (m) Acetal | |
| 14 | 2-(2-Methyl-1,3-dioxolan-5)-ethyl | $CH_3-NH-CH_2-$ | H | H | H | 5,0 (m) Acetal | |

Tabelle 1 (Fortsetzung)

| Nr. | $R^1$ | $R^2$ | $R^3$ | $R^4$ | $R^5$ | $^1H$-NMR [ppm] in $CDCl_3$ |
|---|---|---|---|---|---|---|
| 15 | 2-Phenylethyl | $CH_3-NH_2-$ | H | H | H | 5,0 |
| 16 | 2-Phenylethyl | $CH_3-NH-CH_2-$ | H | H | H | 5,0 |
| 17 | 4-Chlorphenyl | $CH_3-NH-CH_2-$ | H | H | H | 5,7 |
| 18 | 4-Chlorphenyl | $CH_3-NH_2-$ | H | H | H | |
| 19 | 4-Methylphenyl | $CH_3-NH-CH_2-$ | H | H | H | |
| 20 | 4-Methylphenyl | $CH_3-NH_2-$ | H | H | H | |
| 21 | Phenyl | $CH_3-NH-CH_2-$ | H | H | H | |
| 22 | Phenyl | $CH_3-NH_2-$ | H | H | H | |
| 23 | 4-Ethylphenyl | $CH_3-NH_2-$ | H | H | H | |
| 24 | Phenyl | $CH_3-NH_2-$ | $CH_3$ | H | H | |
| 25 | Phenyl | $CH_3-NH-CH_2-$ | $CH_3$ | H | H | 5,3 (d) |
| 26 | 4-Methylphenyl | $CH_3-NH-CH_2-$ | $CH_3$ | H | H | 5,3 (d) |
| 27 | 4-Methylphenyl | $CH_3-NH_2-$ | $CH_3$ | H | H | 5,3 (d) |
| 28 | 4-Chlorphenyl | $CH_3-NH_2-$ | $CH_3$ | H | H | 5,9 (d) Acetal |
| 29 | 2-Phenylpropyl | CHO | H | H | H | 9,6 (s) CHO |
| 30 | Tetrahydrofur-2-yl-methyl | CHO | H | H | H | |
| 31 | 2-Phenylpropyl | CHO | H | H | H | |
| 32 | 3-Phenylbutyl | CHO | H | H | H | 9,8 (d) CHO |
| 33 | 2-Methyl-5-ethyl--1,3-dioxan-5-methyl | CHO | H | H | H | 9,7 (s) CHO |
| 34 | 2-Methyl-1,3-di-oxolan-5-methyl | CHO | H | H | H | 9,7 (s) CHO |
| 35 | $CH_3$ | CHO | H | H | H | |
| 36 | 2-Methylpropyl | CHO | H | H | H | |
| 37 | n-Butyl | CHO | H | H | H | |
| 38 | 2-Ethoxyethyl | CHO | H | H | H | |

Tabelle 1 (Fortsetzung)

| Nr. | $R^1$ | $R^2$ | $R^3$ | $R^4$ | $R^5$ | $^1H$-NMR [ppm] in $CDCl_3$ | IR [$cm^{-1}$] |
|---|---|---|---|---|---|---|---|
| 39 | Ethoxyethoxy-ethoxyethyl | CHO | H | H | H | | |
| 40 | 2-Ethylhexyl | CHO | H | H | H | | |
| 41 | Butoxyethoxyethyl | CHO | H | H | H | | |
| 42 | 2,6-Dimethyl-hept-6-en-1-yl | CHO | H | H | H | 5,7 (s) CHO | |
| 43 | n-Dodecyl | CHO | H | H | H | 9,7 (s) CHO | |
| 44 | 4-Chlorphenyl | CHO | H | H | H | | |
| 45 | 4-Methylphenyl | CHO | H | H | H | | |
| 46 | 4-Fluorphenyl | CHO | H | H | H | 9,6 (s) CHO | |
| 47 | 4-Trifluorphenyl | CHO | H | H | H | | |
| 48 | 2,4-Difluorphenyl | CHO | H | H | H | | |
| 49 | 2-Chlorphenyl | CHO | H | H | H | | |
| 50 | 2-Methyl-4-chlorphenyl | CHO | H | H | H | | |
| 51 | 2,4-Dichlorphenyl | CHO | H | H | H | | |
| 52 | 4-Chlorphenyl | CHO | H | H | H | | |
| 53 | Phenyl | CHO | H | H | H | | |
| 54 | 4-Methylphenyl | CHO | H | H | H | 4,4 (d) | |
| 55 | 4-Ethylphenyl | CHO | H | H | H | | |
| 56 | 4-Methylphenyl | CH=N-N-2,4-di-nitrophenyl | H | H | H | | |
| 57 | p-Diphenyl | CHO | H | H | H | 9,8 (s) CHO | |
| 58 | p-Methoxyphenyl | CHO | H | H | 4-Cl | 9,65 (s) | |
| 59 | p-Methoxyphenyl | CHO | $CH_3$ | H | H | 9,6 (d) | |
| 60 | p-Methylphenyl | CHO | $CH_3$ | H | H | 9,6 (d) | |
| 61 | p-Ethylphenyl | CHO | $CH_3$ | H | H | | 1738,1510,1228 |

Tabelle 1 (Fortsetzung)

| Nr. | $R^1$ | $R^2$ | $R^3$ | $R^4$ | $R^5$ | $^1$H-NMR [ppm] in $CDCl_3$ |
|---|---|---|---|---|---|---|
| 62 | p-Fluorphenyl | CHO | $CH_3$ | H | H | 9,6 (s) |
| 63 | p-Chlorphenyl | CHO | H | 2-F | 4-F | 9,6 (s) |
| 64 | p-Chlorphenyl | H | n-Hexyl | H | H | 4,6 (d); 4,8 (s) Acetal |
| 65 | 2-Methyl-3-phenylpropyl | H | n-Hexyl | H | H | |
| 66 | Methyl | H | Methyl | H | H | |
| 67 | 4-Methylphenyl | H | Methyl | H | H | |
| 68 | Butoxyethoxy-ethoxyethyl | H | Methyl | H | H | |
| 69 | 2-Methyl-1,3-dioxolan-5-methyl | H | Methyl | H | H | 5,1 Acetal |
| 70 | n-Dodecyl | H | Methyl | H | H | 4,5 (d); 4,9 Acetal |
| 71 | 2-Phenylpropyl | H | Methyl | H | H | 4,4 (d) Acetal |
| 72 | 2-Methyl-5-ethyl--1,3-dioxan-5-methyl | H | Methyl | H | H | 4,5 Acetal |
| 73 | 3-(4-Methylcyclo-hexyl)-butyl | H | Methyl | H | H | 4,5 Acetal |
| 74 | Methyl | $CH_3$ | H | H | H | |
| 75 | 4-Methoxyphenyl | $CH_3$ | H | H | H | 5,1 (d); 5,6 (s) |
| 76 | 4-Methylphenyl | $CH_3$ | H | H | H | |
| 77 | 2-Phenylpropyl | $CH_3$ | H | H | H | |
| 78 | 2-Methyl-1,3-di-oxolan-5-methyl | $CH_3$ | H | H | H | 5,1 Acetal |
| 79 | 2-Methyl-1,3-dioxolan-5-methyl (cis-Isomer) | $CH_3$ | H | H | H | |
| 80 | 4-Methylcyclohexyl | $CH_3$ | H | H | H | |

Tabelle 1 (Fortsetzung)

| Nr. | $R^1$ | $R^2$ | $R^3$ | $R^4$ | $R^5$ | $^1$H-NMR [ppm] in $CDCl_3$ | IR [$cm^{-1}$] |
|---|---|---|---|---|---|---|---|
| 81 | 2-(2-Methyl-1,3-dioxolan-5)-ethyl | $CH_3$ | H | H | H | 5,0 Acetal | |
| 82 | 2-Methyl-5-ethyl-1,3-dioxolan-5-methyl | $CH_3$ | H | H | H | 4,6 Acetal | |
| 83 | 2-(2-Methyl-1,3-dioxolan-5)-ethyl (cis-Isomer) | $CH_3$ | H | H | H | 5,1 Acetal | |
| 84 | 2,2-Dimethyl-1,3-dioxolan-5-methyl | $CH_3$ | H | H | H | 4,6 (d) Acetal | |
| 85 | 4-Methyl-cyclohexyl | H | H | H | H | 5,1 Acetal | |
| 86 | 2-Methyl-3-phenyl-propyl | H | H | H | H | 4,9 Acetal | |
| 87 | 3-(4-Methyl-cyclohexyl)-butyl | H | H | H | H | 6,4 Acetal | |
| 88 | 2-Phenyl-1,3-dioxolan-5-methyl | H | H | H | H | | |
| 89 | 2-(2-Phenylethyl)--1,3-di-oxolan-5--methyl | H | H | H | H | | 1119,1068,699 |
| 90 | 2-Spirocyclohexyl--1,3-dioxolan-5--methyl | H | H | H | H | | 1102,1069,699 |
| 91 | 2,2-Dimethyl-1,3-dioxolan-5-methyl | H | H | H | H | 4,5 (d); 4,95 (s) Acetal | |
| 92 | 2-Isopropyl-1,3--dioxolan-5-methyl | H | H | H | H | | 1121,1070,699 |

Tabelle 1 (Fortsetzung)

| Nr. | $R^1$ | $R^2$ | $R^3$ | $R^4$ | $R^5$ | $^1H$-NMR [ppm] in $CDCl_3$ | IR [$cm^{-1}$] | Fp [°C] | Kp [°C]/mbar |
|---|---|---|---|---|---|---|---|---|---|
| 93 | 2-(2,5,5-Trimethyl-pentyl)-1,3-dioxo-lan-5-methyl | H | H | H | H | | 1130,1070,699 | | |
| 94 | 2-Methylpropyl | H | H | H | H | | | | 128-132 / 2 |
| 95 | n-Butyl | H | H | H | H | | | | 118-120 / 2 |
| 96 | 2-Methyl-3-(4-tert.-butyl-phenyl)-propyl | H | H | H | H | | | | 215-220 / 2 |
| 97 | 2,4-Dimethylphenyl | H | H | H | H | | | | 180-185 / 2 |
| 98 | 4-Chlorphenyl | H | H | H | H | | 1489,1104,825 | 51-54 | |
| 99 | Phenyl | H | H | H | H | | | 59-65 | |
| 100 | 4-Ethylphenyl | H | H | H | H | | | | |
| 101 | 2-Methyl-1,3-di-oxolan-5-methyl | H | H | H | H | | | | |
| 102 | 2-Cyclohexyl-propyl | H | H | H | H | | | | |
| 103 | 2-(2-Methyl-1,3-dioxolan-5)-ethyl | H | H | H | H | 5,1 Acetal | | | |
| 104 | 2-Methyl-1,3-dioxolan-5-methyl (2,5-cis-Isomer) | H | H | H | H | 5,1 Acetal | | | |
| 105 | 2-Methyl-5-ethyl--1,3-dioxan-5-methyl | H | H | H | H | | 2855,1117,700 | | |

Tabelle 1 (Fortsetzung)

| Nr. | $R^1$ | $R^2$ | $R^3$ | $R^4$ | $R^5$ | $^1$H-NMR (ppm) in $CDCl_3$ |
|---|---|---|---|---|---|---|
| 106 | 4-Chlorphenyl | 4-Chlorphenoxy | H | H | H | 300 MHz / 6,92 |
| 107 | 4-Chlorphenyl | 4-Methylphenoxy | H | H | H | 300 MHz / 5,03 |
| 108 | 4-Chlorphenyl | Prop-2-in-1-yloxy | H | H | H | 220 MHz / 5,24 |
| 109 | 4-Chlorphenyl | 4-Phenoxyphenoxy | H | H | H | 220 MHz / 5,00 |
| 110 | 4-Chlorphenyl | 1,3-Difluorpropyl-2-oxy | H | H | H | 200 MHz / 5,06 |
| 111 | 4-Chlorphenyl | 2,4-Dichlorphenoxy | H | H | H | 300 MHz / 5,57 |
| 112 | 4-Chlorphenyl | 4-Trifluorphenoxy | H | H | H | 200 MHz / 5,75 |
| 113 | 4-Chlorphenyl | 4-Fluorphenoxy | H | H | H | 200 MHz / 3,55 |
| 114 | 4-Chlorphenyl | 2-Trifluormethylphenoxy | H | H | H | 300 MHz / 3,58 |
| 115 | 4-Chlorphenyl | 2-Fluor-phenoxy | H | H | H | 200 MHz / 3,52 |
| 116 | 4-Chlorphenyl | Butin-2-oxy | H | H | H | 300 MHz / 4,72 |
| 117 | 4-Chlorphenyl | Propyl-2-oxy | H | H | H | 300 MHz / 3,78 |
| 118 | 4-Chlorphenyl | Phenoxy | H | H | H | 300 MHz / 3,57 |
| 119 | 4-Chlorphenyl | 4-Chlorbut-2-in-1-yl-oxy | H | H | H | 200 MHz / 3,48 |
| 120 | 4-Chlorphenyl | 3-Phenylprop-2-in-1-yl-oxy | H | H | H | 200 MHz / 3,45 |
| 121 | 3-Phenoxyphenyl | 3-Phenyloxy-phenoxy | H | H | H | 200 MHz / 3,24 |
| 122 | 4-Chlorphenyl | 3-Phenoxybenzyl | H | H | H | 200 MHz / 4,16 |
| 123 | 4-Chlorphenyl | 1-(3-Phenoxyphenyl)--prop-2-in-1-yl-oxy | H | H | H | 200 MHz / 2,12 |
| 124 | 4-Chlorphenyl | 4-(3-Chlorphenoxy)-but--2-in-1-yl-oxy | H | H | H | 300 MHz / 5,12 |
| 125 | 4-Chlorphenyl | 4-(4-Fluorphenoxy)-but--2-in-1-yl-oxy | H | H | H | 300 MHz / 4,69 |
| 126 | 4-Chlorphenyl | 4-Phenyl-but-3-in-1-yl-oxy | H | H | H | 300 MHz / 2,75 |
| 127 | 4-Phenylbut-3--in-1-yl | 4-Phenyl-but-3-in-1-yl-oxy | H | H | H | 300 MHz / 3,24 |

Tabelle 1 (Fortsetzung)

| Nr. | $R^1$ | $R^2$ | $R^3$ | $R^4$ | $R^5$ | $^1H$-NMR (ppm) in $CDCl_3$ |
|---|---|---|---|---|---|---|
| 128 | 4-Chlorphenyl | 3,4-Methylendioxybenzyloxy | H | H | H | 300 MHz / 3,37 |
| 129 | 4-Chlorphenyl | Ethoxycarbonylmethoxy | H | H | H | 220 MHz / 5,15 |
| 130 | 4-Chlorphenyl | 2-Fluorphenoxy | H | H | H | 300 MHz / 3,57 |
| 131 | 2,2-Dimethyl-1,3-dioxolan-5-methyl | 4-Fluorphenoxy | H | H | H | 200 MHz / 1,38 |
| 132 | 4-Chlorphenyl | 1,2-Dibrom-2-(4-chlorphenyl)-ethyl | H | H | H | 300 MHz / 6,97 |
| 133 | 4-Chlorphenyl | Carboxyl | H | H | H | 300 MHz / 6,75 |
| 134 | 4-Methylphenyl | Formyl | H | H | H | 300 MHz / 4,43 |
| 135 | 4-Chlorphenyl | Formyl | H | H | H | 300 MHz / 9,73 |
| 136 | 4-Chlorphenyl | -CH=N-OH (Z/E = 30/70) | H | H | H | 300 MHz / 4,48 |
| 137 | 4-Chlorphenyl | -CH=N-OH (Z/E = 85/15) | H | H | H | 300 MHz / 5,86 |
| 138 | 4-Chlorphenyl | $-CH=N-O-\overset{O}{\overset{\|}{C}}-CH_2-Cl$ | H | H | H | 300 MHz / 5,94 |

Tabelle 2

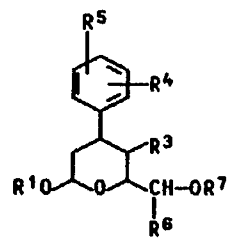

| Nr. | $R^1$ | $R^3$ | $R^4$ | $R^5$ | $R^6$ | $R^7$ | $^1$H-NMR (ppm) in $CDCl_3$ |
|---|---|---|---|---|---|---|---|
| 139 | 2-Methyl-1,3-dioxolan-5-methyl | H | H | H | CN | 2,2-Dimethyl-3-(2',2'-dichlorvinyl)-cyclopropyl-1-carbonyl | 300 MHz / 5,62 |
| 140 | 4-Chlorphenyl | $CH_3$ | H | H | CN | 2,2-Dimethyl-3-(2',2'-dimethylvinyl)-cyclopropan-1-carbonyl | 300 MHz / 4,95 |
| 141 | 4-Chlorphenyl | $CH_3$ | H | H | CN | 4-Chlorphenyl-carbonyl(1) | 300 MHz / 5,18 |
| 142 | 4-Chlorphenyl | H | H | H | CN | 2-Fluorbenzylcarbonyl | 300 MHz / 3,71 |
| 143 | 4-Chlorphenyl | $CH_3$ | H | H | CN | Phenylcarbonyl | 300 MHz / 5,90 |
| 144 | 4-Chlorphenyl | H | H | H | CN | 2-Chlorbenzylcarbonyl | 300 MHz / 3,78 |
| 145 | 2-Methylpropyl | H | H | H | $CH_3$ | 2-Chlorbenzylcarbonyl | 200 MHz / 5,00 |
| 146 | 2-Methylpropyl | H | H | H | $CH_3$ | 2,2-Dimethyl-3-(2',2'-dichlorvinyl)-cyclopropan-1-carbonyl | 300 MHz / 6,14 |
| 147 | 2-Methylpropyl | H | H | H | H | 2,2-Dimethyl-3-(2',2'-dichlorvinyl)-cyclopropan-1-carbonyl | 300 MHz / 5,58 |
| 148 | 4-Chlorphenyl | H | 2-F | 6-F | CN | 2,2-Dimethyl-3-(2',2'-dichlorvinyl)-cyclopropan-1-carbonyl | 200 MHz / 5,70 |

Tabelle 2 (Fortsetzung)

| Nr. | $R^1$ | $R^3$ | $R^4$ | $R^5$ | $R^6$ | $R^7$ | $^1H$-NMR (ppm) in $CDCl_3$ |
|---|---|---|---|---|---|---|---|
| 149 | 4-Chlorphenyl | H | 2-F | 6-F | $-CH=CH_2$ | 2,2-Dimethyl-3-(2',2'-dichlorvinyl)-cyclopropan-1-carbonyl | 300 MHz / 6,22 |
| 150 | Methyl | H | H | H | CN | 2,2-Dimethyl-3-(2',2'-dichlorvinyl)-cyclopropan-1-carbonyl | 200 MHz / 6,18 |
| 151 | Methyl | H | H | H | H | 2,2-Dimethyl-3-(2',2'-dichlorvinyl)-cyclopropan-1-carbonyl | 200 MHz / 5,72 |
| 152 | 2-Methylpropyl | H | H | H | $-C\equiv CH$ | 2,2-Dimethyl-3-(2',2'-dichlorvinyl)-cyclopropan-1-carbonyl | 300 MHz / 6,23 |
| 153 | Methyl | H | H | H | $-C\equiv CH$ | 2,2-Dimethyl-3-(2',2'-dichlorvinyl)-cyclopropan-1-carbonyl | 270 MHz / 5,58 |
| 154 | 2-Methylpropyl | H | H | H | $-C\equiv CH$ | 2,2-Dimethyl-3-(2',2'-dichlorvinyl)-cyclopropan-1-carbonyl | 200 MHz / 4,25 |
| 155 | 4-Chlorphenyl | H | H | H | $-CH=CH_2$ | 2,2-Dimethyl-3-(2',2'-dichlorvinyl)-cyclopropan-1-carbonyl | 300 MHz / 4,06 |
| 156 | 2-Chlorphenyl | H | H | H | CN | 2,2-Dimethyl-3-(2',2'-dichlorvinyl)-cyclopropan-1-carbonyl | 200 MHz / 6,12 |
| 157 | 2,4-Dichlorphenyl | H | H | H | CN | 2,2-Dimethyl-3-(2',2'-dichlorvinyl)-cyclopropan-1-carbonyl | 200 MHz / 6,20 |

Tabelle 2 (Fortsetzung)

| Nr. | $R^1$ | $R^3$ | $R^4$ | $R^5$ | $R^6$ | $R^7$ | $^1H$-NMR (ppm) in $CDCl_3$ |
|---|---|---|---|---|---|---|---|
| 158 | 4-Chlorphenyl | H | H | H | $CH_3$ | 2,2-Dimethyl-3-(2',2'-dichlorvinyl)-cyclopropan-1-carbonyl | 300 MHz / 3,34 |
| 159 | 4-Chlorphenyl | H | H | H | -C≡CH | 2,2-Dimethyl-3-(2',2'-dichlorvinyl)-cyclopropan-1-carbonyl | 270 MHz / 6,22 |
| 160 | 4-Chlorphenyl | H | H | H | CN | 2,2-Dimethyl-3-(2',2'-dichlorvinyl)-cyclopropan-1-carbonyl | 200 MHz / 4,30 |
| 161 | 2-Methyl-4-chlorphenyl | H | H | H | H | 2,2-Dimethyl-3-(2',2'-dichlorvinyl)-cyclopropan-1-carbonyl | 200 MHz / 4,15 |
| 162 | 2-Methyl-4-chlorphenyl | H | H | H | CN | 2,2-Dimethyl-3-(2',2'-dichlorvinyl)-cyclopropan-1-carbonyl | 270 MHz / 5,64 |
| 163 | 2-Methylpropyl | H | H | H | $CH_3$ | 2,2-Dimethyl-3-(1',2'-dibrom-2',2'-dichlorethyl)cyclopropan-1-carbonyl | |
| 164 | 2-Methylpropyl | H | H | H | H | 2,2-Dimethyl-3-(1',2'-dibrom-2',2'-dichlorethyl)cyclopropan-1-carbonyl | 300 MHz / 3,23 |
| 165 | 2-Methylpropyl | H | H | H | -C≡CH | 2,2-Dimethyl-3-(1',2'-dibrom-2',2'-dichlorethyl)cyclopropan-1-carbonyl | 300 MHz / 3,21 |

Tabelle 2 (Fortsetzung)

| Nr. | $R^1$ | $R^3$ | $R^4$ | $R^5$ | $R^6$ | $R^7$ | $^1$H-NMR (ppm) in $CDCl_3$ |
|---|---|---|---|---|---|---|---|
| 166 | Methyl | H | H | H | -C≡CH | 2,2-Dimethyl-3-(1',2'-dibrom-2',2'-dichlor-ethyl)cyclopropan-1-carbonyl | 270 MHz / 4,93 |
| 167 | 4-Chlorphenyl | H | H | H | CN | 2-Chlor-6-Fluor-phenylacetyl | 300 MHz / 5,44 |
| 168 | 4-Chlorphenyl | H | H | H | $-CH=CH_2$ | H | 300 MHz / 3,38 |
| 169 | 4-Methylphenyl | H | H | H | $-CH=CH_2$ | H | |
| 170 | 4-Chlorphenyl | H | H | H | $-CH=CH_2$ | Chloracetyl | 300 MHz / 3,39 |
| 171 | 4-Chlorphenyl | H | H | H | $-CH=CH_2$ | Phenylaminocarbonyl | 300 MHz / 5,65 |
| 172 | Methyl | H | H | H | -C≡CH | H | |
| 173 | 4-Chlorphenyl | H | H | H | H | 4-Chlorphenylamino-carbonyl | 300 MHz / 5,18 |
| 174 | 4-Chlorphenyl | H | H | H | H | 3-Methylphenyl-aminocarbonyl | 200 MHz / 5,75 |
| 175 | 4-Chlorphenyl (cis) | H | H | H | H | 2,2-Dimethyl-3(2',2'-dichlorvinyl)-cyclo-propan-1-carbonyl | 200 MHz / 6,25 |
| 176 | 4-Chlorphenyl (cis) | H | H | H | $-CH=CH_2$ | H | 220 MHz / 5,34 |
| 177 | 4-Chlorphenyl (cis) | H | H | H | H | Phenylaminocarbonyl | 300 MHz / 4,19 |
| 178 | 4-Chlorphenyl | H | H | H | $CH_3$ | H | 300 MHz / 5,69 |
| 179 | 4-Chlorphenyl | H | H | H | H | H | 300 MHz / 5,71 |
| 180 | 4-Chlorphenyl (cis) | H | H | H | $-CH=CH_2$ | H | 300 MHz / 5,76 |

Tabelle 2 (Fortsetzung)

| Nr. | $R^1$ | $R^3$ | $R^4$ | $R^5$ | $R^8$ | $R^7$ | $^1H$-NMR (ppm) in $CDCl_3$ |
|---|---|---|---|---|---|---|---|
| 181 | 4-Chlorphenyl (cis) | H | H | H | $-CH=CH_2$ | Chloracetyl | 300 MHz / 4,14 |
| 182 | 4-Chlorphenyl (cis) | H | H | H | $-CH=CH_2$ | Phenylaminocarbonyl | 300 MHz / 5,74 |
| 183 | 4-Chlorphenyl (cis) | H | H | H | $-CH=CH_2$ | Methylpropionyl | 300 MHz / 5,11 |
| 184 | 4-Chlorphenyl | H | H | H | H | Benzoyl | 300 MHz / 4,33 |
| 185 | 4-Chlorphenyl | H | H | H | $CH_3$ | Propinyl | 300 MHz / 4,63 |
| 186 | 4-Chlorphenyl | H | H | H | $CH_3$ | Methylaminocarbonyl | 300 MHz / 1,13 |
| 187 | 4-Chlorphenyl | H | H | H | H | Methyl | 300 MHz / 5,18 |
| 188 | 4-Chlorphenyl | H | 2-F | 6-F | CN | Methylpropionyl | 200 MHz / 2,60 |
| 189 | 4-Chlorphenyl | H | H | H | $-CH=CH_2$ | Methylpropionyl | 300 MHz / 5,12 |
| 190 | 4-Chlorphenyl | H | H | H | $-CH=CH_2$ | 3,4-Dichlorphenyl-aminocarbonyl | 270 MHz / 5,11 |
| 191 | 4-Chlorphenyl | H | H | H | $-CH=CH_2$ | 3-Methylphenyl-aminocarbonyl | 300 MHz / 5,10 |
| 192 | 4-Chlorphenyl | H | H | H | $-C\equiv CH$ | H | 300 MHz / 5,74 |
| 193 | 4-Chlorphenyl (cis) | H | H | H | H | H | |
| 194 | 4-Chlorphenyl (cis) | H | H | H | H | H | 300 MHz / 5,69 |
| 195 | 2-Methylpropyl | H | H | H | H | H | 300 MHz / 4,99 |

Tabelle 3

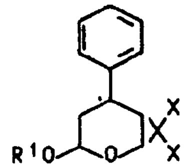

| Nr. | $R^1$ | X | $^1$H-NMR (ppm) in $CDCl_3$ |
|---|---|---|---|
| 196 | 2-Methylpropyl | Br | 300 MHz / 3,74 |
| 197 | 4-Chlorphenyl | Cl | 300 MHz / 5,60 |
| 198 | 4-Chlorphenyl | Br | 300 MHz / 3,98 |

Tabelle 4

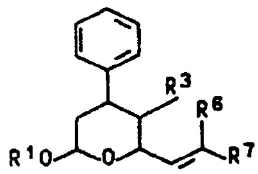

| Nr. | $R^1$ | $R^3$ | $R^6$ | $R^7$ | $^1$H-NMR (ppm) in $CDCl_3$ |
|---|---|---|---|---|---|
| 199 | 4-Chlorphenyl | H | H | $CH_3$ | |
| 200 | 2-Methylpropyl | H | H | 4-Chlorphenyl | 300 MHz / 6,67 |
| 201 | 4-Chlorphenyl | H | H | 1-Naphthyl | 300 MHz / 5,18 |
| 202 | 4-Chlorphenyl | H | H | 4-Cyanophenyl | 200 MHz / 4,60 |
| 203 | 4-Chlorphenyl | H | H | 2-Chlorphenyl | 300 MHz / 6,77 |
| 204 | 4-Chlorphenyl | H | H | Acetyl | 300 MHz / 4,79 |
| 205 | 4-Chlorphenyl | H | H | 3-Methyl-isoxazolyl-5 | 200 MHz / 6,22 |
| 206 | 4-Chlorphenyl | H | H | $CH_3$ | 300 MHz / 6,46 |
| 207 | 4-Chlorphenyl | H | H | Ethoxycarbonyl | |
| 208 | 4-Chlorphenyl | H | H | 4-Chlorphenyl | |

Tabelle 5

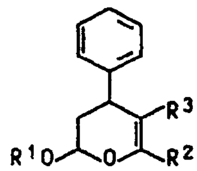

| Nr. | $R^1$ | $R^2$ | $R^3$ | Physik. Daten |
|---|---|---|---|---|
| 209 | 4-Methoxyphenyl | H | H | $Kp_4$=155-160°C |
| 210 | 4-Diphenyl | H | H | $Kp_2$=236-345°C |
| 211 | 4-Fluorphenyl | H | H | Fp = 92- 95°C |
| 212 | Phenyl | H | H | Fp = 85-103°C |
| 213 | 4-Ethylphenyl | H | H | Fp = 90- 91°C |
| 214 | 4-Fluorphenyl | H | H | $Kp_4$=170-175°C |
| 215 | 4-Methylphenyl (trans) | H | H | Fp = 68- 96°C |
| 216 | 4-Methylphenyl (cis) | H | H | Fp = 55- 67°C |
| 217 | 4-Chlorphenyl (trans) | H | H | Fp = 97-100°C |
| 218 | 4-Chlorphenyl (cis) | H | H | Fp = 53- 66°C |
| 219 | 4-Phenoxyphenyl | H | H | $Kp_2$=214-230°C |
| 220 | 4-Chlorphenyl | $CH_3$ | H | $Kp_2$=205-215°C |
| 221 | 2-Methyl-4-chlorphenyl | $CH_3$ | H | $Kp_2$=195-210°C |
| 222 | 2,4-Dichlorphenyl | $CH_3$ | H | $Kp_2$=200-223°C |
| 223 | 4-tert.-Butylphenyl | $CH_3$ | H | $Kp_1$=162°C |
| 224 | 2,5-Dimethyl | $CH_3$ | H | $Kp_4$=222°C |
| 225 | 3-Chlorphenyl | $CH_3$ | H | $Kp_2$=186-190°C |
| 226 | 2,4-Dimethylphenyl | $CH_3$ | H | $Kp_2$=170-200°C |
| 227 | 4-Methylphenyl | $CH_3$ | H | $Kp_2$=160°C |
| 228 | 4-Methoxyphenyl | $CH_3$ | H | $Kp_4$=190-200°C |
| 229 | 4-Fluorphenyl | $CH_3$ | H | $Kp_2$=200°C |
| 230 | Phenyl | $CH_3$ | H | $Kp_4$=170-172°C |
| 231 | 4-Ethylphenyl | $CH_3$ | H | $Kp_2$=186-195°C |
| 232 | 3,5-Dimethyl-4-chlorphenyl | $CH_3$ | H | $Kp_2$=180-190°C |
| 233 | 4-Chlorphenyl | H | H | |
| 234 | 2,4-Dichlorphenyl | H | H | Fp = 83- 85°C |
| 235 | 2-Methyl-4-chlorphenyl | H | H | Fp =131-134°C |
| 236 | 4-Chlor-3-methylphenyl | H | H | Fp =176-178°C |
| 237 | 2-Chlorphenyl | H | H | $Kp_2$=180-190°C |
| 238 | 2-Chlorphenyl (cis) | H | H | Fp = 58- 59°C |

Tabelle 5 (Fortsetzung)

| Nr. | $R^1$ | $R^2$ | $R^3$ | Physik. Daten |
|---|---|---|---|---|
| 239 | 2-Chlorphenyl (trans) | H | H | Fp = 99-106°C |
| 240 | 4-tert.-Butylphenyl | H | H | Fp =151-156°C |
| 241 | 2,4-Dichlorphenyl (trans) | H | H | Fp =129-133°C |
| 242 | 2,4-Dichlorphenyl (cis) | H | H | Fp = 83- 86°C |
| 243 | 1,1,3,3-Tetramethyl-butylphenyl | H | H | Fp = 99-102°C |
| 244 | 2,4-Dimethylphenyl | H | H | Fp = 77-114°C |
| 245 | 4-Methylphenyl | H | H | Fp = 95- 96°C |
| 246 | 2-Fluorphenyl | H | H | $Kp_2$=146-156°C |
| 247 | 3,7-Dimethyl-oct-6-en-1-yl | H | $CH_3$ | $Kp_1$=160°C |
| 248 | n-Dodecyl | H | $CH_3$ | Kp =196-205°C |
| 249 | 2-Methyl-2-ethyl-1,3- -dioxolan-5-methyl | H | $CH_3$ | $Kp_3$=153°C |
| 250 | 2,2-Diethyl-1,3- dioxolan-5-methyl | H | $CH_3$ | $Kp_2$=160-180°C |
| 251 | 2-Methyltetrahydro- furan-3-methyl | H | $CH_3$ | $Kp_4$=170-190°C |
| 252 | Methoxyethoxyethoxyethyl | H | $CH_3$ | $Kp_4$=202-210°C |
| 253 | 2-Methyl-2-(4-chlorphenyl)- -1,3-dioxolan-5-methyl | H | $CH_3$ | $Kp_2$=210-217°C |
| 254 | 2-Benzyl-1,3-dioxolan-5-methyl | H | $CH_3$ | $Kp_2$=220-230°C |
| 255 | 2-Isopropyl-1,3-dioxolan- -5-methyl | H | $CH_3$ | $Kp_2$=160°C |
| 256 | 2-(2'-Methylpropyl)-1,3- -dioxolan-5-methyl | H | $CH_3$ | $Kp_2$=152-160°C |
| 257 | Methyl | H | H | $Kp_2$=135-138°C |
| 258 | Methyl (trans) | H | H | $Kp_8$=107°C |
| 259 | Methyl (cis) | H | H | $Kp_8$=111°C |
| 260 | 2-Methylpropyl | H | H | $Kp_2$= 93°C |
| 261 | Isopropyl (cis) | H | H | $Kp_2$= 84- 85°C |
| 262 | Isopropyl (trans) | H | H | $Kp_2$= 80°C |
| 263 | n-Butyl (cis) | H | H | $Kp_8$=140°C |
| 264 | n-Butyl (trans) | H | H | $Kp_8$=140-141°C |
| 265 | 2-Methylpropyl (cis) | H | H | $Kp_2$=108°C |
| 266 | 2-Methylpropyl (trans) | H | H | $Kp_2$=107°C |
| 267 | 2-Ethylhexyl (cis) | H | H | $Kp_1$=140°C |
| 268 | 2-Ethylhexyl (trans) | H | H | $Kp_1$=138°C |
| 269 | n-Octyl | H | H | Kp =150-156°C |
| 270 | 4-Methylcyclohexyl | H | $CH_3$ | $Kp_2$=130-146°C |

Tabelle 5 (Fortsetzung)

| Nr. | $R^1$ | $R^2$ | $R^3$ | Physik. Daten |
|---|---|---|---|---|
| 271 | 3-(4'-Methylcyclohex-3-en-1-yl)-butyl | H | $CH_3$ | $Kp_2$=190-196°C |
| 272 | 2-Phenylcyclohexyl | H | $CH_3$ | $Kp_2$=190-210°C |
| 273 | Benzyl | H | $CH_3$ | $Kp_5$=180-187°C |
| 274 | 3-Phenoxybenzyl | H | $CH_3$ | $Kp_2$=220-230°C |
| 275 | Phenethyl | H | $CH_3$ | $Kp_2$=160-168°C |
| 276 | 2-Phenylpropyl | H | $CH_3$ | $Kp_2$=150-175°C |
| 277 | 3-Phenylpropyl | H | $CH_3$ | $Kp_2$=160-168°C |
| 278 | 2-Methyl-3-phenylpropyl | H | $CH_3$ | $Kp_2$=195-205°C |
| 279 | 3-Phenylbutyl | H | $CH_3$ | $Kp_2$=195-210°C |
| 280 | 3,7-Dimethyloctyl | H | H | $Kp_1$=196-206°C |
| 281 | 3,7-Dimethyl-oct-6-en-1-yl | H | H | $Kp_4$=175-180°C |
| 282 | n-Dodecyl | H | H | $Kp_4$=196-198°C |
| 283 | 2,5,9-Trimethyl-dec-4-en-1-yl | H | H | $Kp_2$=192-220°C |
| 284 | 1,5,9-Trimethyl-dec-4-en-1-yl | H | H | $Kp_2$=190-230°C |
| 285 | 2-Methyl-1,3-dioxolan-5-methyl | H | $CH_3$ | $Kp_2$=156-178°C |
| 286 | Tetrahydrofuran-3-methyl | H | $CH_3$ | $Kp_2$=150-158°C |
| 287 | 2-Methyl-5-ethyl-1,3--dioxan-5-methyl | H | $CH_3$ | $Kp_2$=157-170°C |
| 288 | 2-Methyl-1,3-dioxolan--5-methyl (cis) | H | $CH_3$ | $Kp_2$=147-160°C |
| 289 | 2,2-Dimethyl-1,3-dioxolan--5-methyl | H | $CH_3$ | $Kp_2$=155-170°C |
| 290 | 2,5-Dimethyl-1,3-dioxolan--5-methyl | H | $CH_3$ | $Kp_2$=155°C |
| 291 | 2,2-Spirocyclohexyl-5-ethyl--1,3-dioxolan-5-methyl | H | $CH_3$ | $Kp_2$=190-210°C |
| 292 | 2,2-Spirocyclohexyl-1,3--dioxolan-4-methyl | H | $CH_3$ | $Kp_2$=196-210°C |
| 293 | 2-Phenyl-1,3-dioxolan-4-methyl | H | $CH_3$ | $Kp_2$=190-216°C |
| 294 | 2-(1'-Phenethyl)-5-ethyl--1,3-dioxolan-5-methyl | H | $CH_3$ | $Kp_2$=210-245°C |
| 295 | 2-Phenyl-2-methyl-5-ethyl--1,3-dioxolan-5-methyl | H | $CH_3$ | $Kp_2$=200-230°C |
| 296 | 2-(2',4',4'-Trimethylphenyl)--1,3-dioxolan-5-methyl | H | $CH_3$ | $Kp_2$=155-160°C |
| 297 | 2-tert.-butyl-1,3-dioxolan--5-methyl | H | $CH_3$ | $Kp_8$=176°C |

Tabelle 5 (Fortsetzung)

| Nr. | $R^1$ | $R^2$ | $R^3$ | Physik. Daten |
|---|---|---|---|---|
| 298 | 2-Isopropyl-1,3-dioxolan--5-methyl | H | $CH_3$ | $Kp_4$=130-150°C |
| 299 | 2-Methyl-1,4-dioxolan-3-methyl | H | $CH_3$ | $Kp_2$=135-140°C |
| 300 | 2,2-Spirocyclopentyl-1,3--dioxolan-5-methyl | H | $CH_3$ | $Kp_2$=150-153°C |
| 301 | Benzyl | H | H | $Kp_4$=188-200°C |
| 302 | 2-Methyl-3-(4'-tert.--butylphenyl)-propyl | H | H | $Kp_2$=194-210°C |
| 303 | 2-Methyl-2-phenethyl | H | H | $Kp_{95}$=162-164°C |
| 304 | 3-Methyl-3-phenyl-propyl | H | H | $Kp_1$=185-194°C |
| 305 | 3-Phenylpropyl | H | H | $Kp_1$=182-184°C |
| 306 | 2-Methyl-3-phenyl-propyl | H | H | $Kp_2$=182-200°C |
| 307 | 3-Phenoxybutyl | H | H | $Kp_2$=215-220°C |
| 308 | Cyclohexyl (cis) | H | H | $Kp_2$=150°C |
| 309 | Cyclohexyl (trans) | H | H | $Kp_2$=145°C |
| 310 | 2-Methyl-2-cyclohexylethyl | H | H | $Kp_{95}$=157-158°C |
| 311 | 4-Methylcyclohexyl | H | H | $Kp_2$=185°C |
| 312 | 3-(4'-Methylcyclohex-3-en-1-yl)-butyl | H | H | $Kp_1$=182-184°C |
| 313 | 2-Phenylcyclohexyl | H | H | $Kp_2$=204-210°C |
| 314 | Ethoxyethyl | H | H | $Kp_2$=135-136°C |
| 315 | Ethoxyethoxyethoxy-ethyl | H | H | $Kp_2$=185-187°C |
| 316 | Butoxyethoxy-ethyl | H | H | $Kp_{0,5}$=168°C |
| 317 | 2-Methyl-1,3-dioxolan-5-methyl | H | H | $Kp_2$=155-170°C |
| 318 | Tetrahydrofuran-2-methyl | H | H | $Kp_{0,5}$=146-150°C |
| 319 | 2-Methyl-1,3-dioxan-5-methyl (trans) | H | H | $Kp_2$=150-155°C |
| 320 | 2-Methyl-1,3-dioxan-5-methyl (cis) | H | H | $Kp_2$=150-155°C |
| 321 | 2-Methyl-5-ethyl-1,3--dioxan-5-methyl | H | H | $Kp_2$=160-175°C |
| 322 | 2-(2'-Methyl-1,3-dioxolan-5)-ethyl | H | H | $Kp_2$=165-170°C |
| 323 | 2,5-Dimethyl-1,3-dioxan--5-methyl | H | H | $Kp_2$=152-160°C |
| 324 | 2,5-Dimethyl-1,3-dioxolan--5-methyl | H | H | $Kp_2$=145-150°C |
| 325 | 2,2-Dimethyl-1,3-dioxolan--5-methyl | H | H | $Kp_2$=165-168°C |

Tabelle 5 (Fortsetzung)

| Nr. | $R^1$ | $R^2$ | $R^3$ | Physik. Daten |
|---|---|---|---|---|
| 326 | 2,2-Diethyl-1,3-dioxolan--5-methyl | H | H | $Kp_2$=168-180°C |
| 327 | 2-Isopropyl-1,3-dioxolan--5-methyl | H | H | $Kp_6$=174°C |
| 328 | 2-(2'-Methylpropyl)-1,3--dioxolan-5-methyl | H | H | $Kp_8$=145°C |
| 329 | 2-Methyl-1,4-dioxan-3-methyl | H | H | $Kp_2$=145°C |
| 330 | 2-Isopropyl-1,3-dioxolan--5-methyl | H | H | $Kp_1$=150°C |
| 331 | 2-tert.-Butyl-1,3-dioxolan--5-methyl | H | H | $Kp_8$=176-196°C |
| 332 | 2-Isopropyl-1,3-dioxan--5-methyl | H | H | $Kp_4$=176-177°C |
| 333 | 2-(2',4',4'-Trimethyl)-1,3-dioxolan-5-methyl | H | H | $Kp_2$=192-200°C |
| 334 | 2,2-Spirocyclohexyl-5-ethyl-1,3-dioxan-5-methyl | H | H | $Kp_2$=210-215°C |
| 335 | 2,2-Spirocyclohexyl-1,3-dioxolan-5-methyl | H | H | $Kp_2$=185-200°C |
| 336 | 2-Phenyl-1,3-dioxolan--5-methyl | H | H | $Kp_2$=200-220°C |
| 337 | 2-Phenyl-5-ethyl-1,3--dioxan-5-methyl | H | H | $Kp_2$=200-235°C |
| 338 | 2-(1'-Phenethyl)-1,3--dioxolan-5-methyl | H | H | $Kp_2$=220-225°C |
| 339 | 2-(1'-Phenethyl)-5-ethyl--1,3-dioxan-5-methyl | H | H | $Kp_2$=240-255°C |
| 340 | 2-(4'-Chlorphenyl)-1,3--dioxolan-5-methyl | H | H | $Kp_2$=200-230°C |
| 341 | 2-Methyl-2-(4-chlorphenyl)--1,3-dioxolan-5-methyl | H | H | $Kp_2$=210-230°C |
| 342 | 2-Phenyl-2-methyl-5-ethyl--1,3-dioxan-5-methyl | H | H | $Kp_2$=210-220°C |
| 343 | 2-(4'-Chlorphenyl)-5-ethyl--1,3-dioxan-5-methyl | H | H | $Kp_2$=210-240°C |
| 344 | 2-Benzyl-1,3-dioxolan-5-methyl | H | H | $Kp_2$=208-224°C |
| 345 | 2,4-Dichlorphenyl | H | $CH_3$ | $Kp_1$=181-201°C |
| 346 | 4-p-tert.-Butylphenyl (70 % trans) | H | $CH_3$ | Fp = 68- 70°C |

Tabelle 5 (Fortsetzung)

| Nr. | $R^1$ | $R^2$ | $R^3$ | Physik. Daten |
|---|---|---|---|---|
| 347 | 4-p-tert.-Butylphenyl (70 % cis) | H | $CH_3$ | Fp =112-117°C |
| 348 | 4-(2',2',4',4'-Tetramethyl--butyl)-phenyl | H | $CH_3$ | Kp =200-220°C |
| 349 | Benzyl | H | $CH_3$ | |
| 350 | 4-Methylphenyl | H | $CH_3$ | $Kp_4$=165-170°C |
| 351 | 4-Chlorphenyl | H | $CH_3$ | $Kp_3$=186-192°C |
| 352 | 4-Methoxyphenyl | H | $CH_3$ | $Kp_4$=190-210°C |
| 353 | 4-Diphenyl | H | $CH_3$ | $Kp_2$=230-250°C |
| 354 | 4-Ethylphenyl | H | $CH_3$ | Fp = 44- 68°C |
| 355 | Phenyl | H | $CH_3$ | $Kp_4$=178-189°C |
| 356 | 4-Fluorphenyl | H | $CH_3$ | $Kp_4$=190-208°C |
| 357 | 2,2-Spirocyclohexyl-5-ethyl--1,3-dioxan-5-methyl | $CH_3$ | H | $Kp_2$=190-210°C |
| 358 | 2,2-Spirocyclohexyl-1,3--dioxolan-5-methyl | $CH_3$ | H | $Kp_2$=200-210°C |
| 359 | 2-Phenyl-1,3-dioxolan-5-methyl | $CH_3$ | H | $Kp_2$=205-218°C |
| 360 | 2-(1'-Phenethyl)-5-ethyl--1,3-dioxan-5-methyl | $CH_3$ | H | $Kp_2$=216-220°C |
| 361 | 2-(4'-Chlorphenyl)-5-ethyl--1,3-dioxan-5-methyl | $CH_3$ | H | $Kp_2$=220-240°C |
| 362 | 2-Methyl-2-phenyl-5-ethyl--1,3-dioxan-5-methyl | $CH_3$ | H | $Kp_2$=200-210°C |
| 363 | 2-Methyl-2-(4'-chlorphenyl)--1,3-dioxolan-5-methyl | $CH_3$ | H | $Kp_2$=210-226°C |
| 364 | 2-Benzyl-1,3-dioxolan-5-methyl | $CH_3$ | H | $Kp_2$=195-215°C |
| 365 | 2-Methyl-1,3-dioxolan-5-methyl | $CH_3$ | H | $Kp_4$=178-190°C |
| 366 | 2-Methyl-1,3-dioxolan--5-methyl-(trans) | $CH_3$ | H | $Kp_2$=150-170°C |
| 367 | Furyl-2-methyl | $CH_3$ | H | $Kp_2$=150-170°C |
| 368 | 2-Methyl-5-ethyl-1,3--dioxan-5-methyl | $CH_3$ | H | $Kp_2$=155-165°C |
| 369 | 2-(2-Methyl-1,3-dioxolan--5)-ethyl-(cis) | $CH_3$ | H | $Kp_2$=186-192°C |
| 370 | 2-Methyl-1,3-dioxolan-5-ethyl | $CH_3$ | H | $Kp_2$=155-168°C |
| 371 | 2,5-Dimethyl-1,3-dioxolan--5-methyl | $CH_3$ | H | $Kp_2$=160-178°C |
| 372 | 2,2-Dimethyl-1,3-dioxolan--5-methyl | $CH_3$ | H | $Kp_2$=167-180°C |

Tabelle 5 (Fortsetzung)

| Nr. | $R^1$ | $R^2$ | $R^3$ | Physik. Daten |
|---|---|---|---|---|
| 373 | 2-Isopropyl-1,3-dioxolan--5-methyl | $CH_3$ | H | $Kp_2$=158-162°C |
| 374 | 2-Methyl-2-ethyl-1,3--dioxolan-5-methyl | $CH_3$ | H | $Kp_5$=177-180°C |
| 375 | 2-(2',4',4'-Trimethylpentyl)-1,3-dioxolan-5-methyl | $CH_3$ | H | $Kp_2$=200-210°C |
| 376 | 2-tert.-Butyl-1,3-dioxolan--5-methyl | $CH_3$ | H | $Kp_4$=190°C |
| 377 | Cyclohexyl | $CH_3$ | H | $Kp_2$=137°C |
| 378 | 4-Methyl-cyclohexyl | $CH_3$ | H | $Kp_2$=145-150°C |
| 379 | 3-(4-Methyl-cyclo-hex-3-en-1-yl)-butyl | $CH_3$ | H | $Kp_2$=197-205°C |
| 380 | 2-Cyclohexylpropyl | $CH_3$ | H | $Kp_1$=170-185°C |
| 381 | 2-Phenyl-cyclohexyl | $CH_3$ | H | $Kp_2$=210-232°C |
| 382 | Methyl (cis) | $CH_3$ | H | $Kp_1$= 96°C |
| 383 | Methyl (trans) | $CH_3$ | H | $Kp_2$= 93°C |
| 384 | 2-Ethylhexyl (trans) | $CH_3$ | H | $Kp_2$=147°C |
| 385 | 2-Ethylhexyl (cis) | $CH_3$ | H | $Kp_2$=144-145°C |
| 386 | Butyl (trans) | $CH_3$ | H | $Kp_2$=118°C |
| 387 | Butyl (cis) | $CH_3$ | H | $Kp_2$=120°C |
| 388 | Butoxy-ethoxy-ethyl | $CH_3$ | H | $Kp_4$=190-198°C |
| 389 | n-Dodecyl | $CH_3$ | H | $Kp_2$=190-200°C |
| 390 | 3,7-Dimethyl-oct-6-en-1-yl | $CH_3$ | H | $Kp_2$=165-170°C |
| 391 | 1,5,9-Trimethyl-dec-4-en-1-yl (cis) | $CH_3$ | H | $Kp_2$=204°C |
| 392 | 1,5,9-Trimethyl-dec-4-en-1-yl (trans) | $CH_3$ | H | $Kp_2$=204-210°C |
| 393 | 3,7-Dimethyl-octyl | $CH_3$ | H | $Kp_2$=165-170°C |
| 394 | 2-Phenylpropyl | $CH_3$ | H | $Kp_2$=190-210°C |
| 395 | 3-Phenylbutyl | $CH_3$ | H | $Kp_2$=180-187°C |
| 396 | 3-Phenylpropyl | $CH_3$ | H | $Kp_2$=183-185°C |
| 397 | 2-Phenethyl | $CH_3$ | H | $Kp_2$=170-175°C |
| 398 | 3-Phenoxy-benzyl | $CH_3$ | H | $Kp_2$=220-230°C |
| 399 | 2-Methyl-3-phenyl-propyl | $CH_3$ | H | $Kp_2$=200-210°C |

Tabelle 6

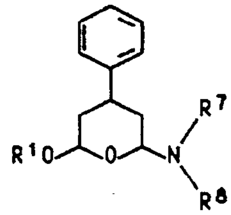

| Nr. | $R^1$ | $R^7$ | $R^8$ | Physik. Daten |
|---|---|---|---|---|
| 400 | 4-Chlorphenyl | 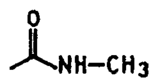 | Methyl | |
| 401 | 4-Chlorphenyl | 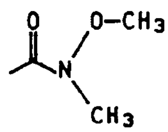 | Methyl | |
| 402 | 4-Chlorphenyl | 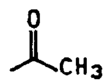 | Methyl | |
| 403 | 4-Chlorphenyl | 3,4-Dichlor-phenylamino-carbonyl | Methyl | |

Anwendungsbeispiele

Beispiel A

Zuchtversuch mit Baumwollwanzen (Dysdercus intermedius)

Baumwollwanzen Dysdercus intermedius werden im 4. Larvenstadium in Petrischalen (Ø 10 cm) dem Wirkstoffbelag der Testsubstanz für 24 Stunden ausgesetzt.

Die Überlebenden züchtet man in 1 l-Gläsern auf feuchtem Quarzsand, der vorher mit der Wirkstofflösung versetzt wurde bis zum Schlüpfen der $F_1$-Generation.

Dabei entsprechen in der Behandlung

2,5 mg/Schale 25 ppm im Sand
1,0 mg/Schale 10 ppm im Sand
0,5 mg/Schale 5 ppm im Sand
u.s.w.

Beurteilt wird Mortalität und Vermehrung.

In diesem Versuch weisen die nachstehend durch ihre Nummern (vgl. Tabellen) gekennzeichneten Verbindungen Wirkungsschwellen von unter 1 ppm, teilweise von unter 0,1 und teilweise von unter 0,01 ppm auf:

18, 19, 20, 44, 52, 58, 76, 83, 98, 101, 133, 136, 137, 138, 158, 159, 160, 173, 174, 175, 176, 177, 178, 179, 180, 181, 182, 183, 184, 185, 186, 192, 197, 198, 199, 201, 202, 203, 204, 205, 206, 207, 208, 211, 214, 218, 220, 221, 228, 229, 230, 233, 245, 288, 320, 350, 351, 365, 369, 400, 401.

Das Vergleichsmittel Fenoxycarb weist eine Wirkungsschwelle von 0,04 ppm auf.

Beispiel B

Wirkung auf Eier der Baumwollwanze (Dysdercus intermedius)

Ca. 200 frisch abgelegte Eier der Baumwollwanze heftet man auf Klebestreifen und taucht diese in die

wäßrige Wirkstoffaufbereitung. Darauf lagert man die Streifen bei 25°C und 70% Luftfeuchtigkeit bis zum Schlüpfen der unbehandelten Kontrolle.

In diesem Versuch weisen die nachstehend durch ihre Nummern (vgl. Tabellen) gekennzeichneten Verbindungen Wirkungsschwellen von unter 0,04 ppm, teilweise von unter 0,004 und teilweise von unter 0,0004 ppm auf:

12, 14, 17, 52, 98, 103, 121, 126, 128, 133, 136, 137, 138, 173, 174, 175, 176, 177, 179, 183, 184, 185, 186, 197, 201, 202, 203, 205, 206, 207, 217, 218, 220, 233, 245, 317, 322, 335, 369, 400, 401.

Das Vergleichsmittel Carbaryl erzielt hierbei eine Wirkungsschwelle von 0,01 ppm.

Beispiel C

Ovo-larvizide Wirkung bei Heliothis virescens

Behandelte Blattstanzstücke werden mit ca. 15 Eiern belegt, nach 4 Tagen werden Schlupf und Mortalität der Jungraupen bonitiert.

In diesem Versuch wurden folgende Ergebnisse erzielt:

| Verb. Nr. | Wirkungsschwelle |
|---|---|
| 23 | 0,04 |
| 34 | 0,1 |
| 90 | 0,04 |
| 104 | 0,04 |
| 324 | 0,04 |
| Chlorpyriphos | 0,1 - 0,04 (Vergleich) |

## Ansprüche

**Patentansprüche fur die Vertragsstaaten : AT BE CH DE FR GB GR IT LI NL SE**

1. Pyranderivate der allgemeinen Formeln Ia, Ib und Ic

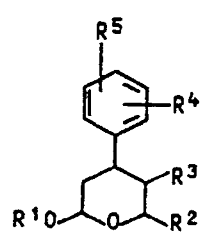
(Ia)

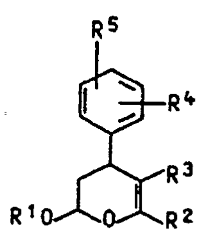
(Ib)

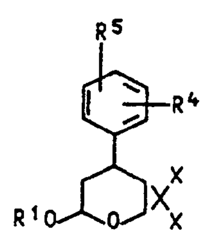
(Ic)

in denen die Substituenten folgende Bedeutung haben:

$R^1$ $C_1$-$C_{20}$-Alkyl, $C_2$-$C_{20}$-Alkenyl, $C_2$-$C_{20}$-Alkinyl, $C_3$-$C_{20}$-Cycloalkyl, Aryl, Biphenylyl, $C_7$-$C_{30}$-Arylalkyl, $C_8$-$C_{30}$-Arylalkenyl, $C_8$-$C_{30}$-Aryl-alkinyl, $C_2$-$C_{20}$-Alkoxy-alkyl, $C_3$-$C_{20}$-Alkoxy-alkoxy-alkyl, $C_4$-$C_{30}$-Alkoxy-alkoxy-alkoxy-alkyl, durch $C_7$-$C_{20}$-Alkyl-aryl, $C_8$-$C_{20}$-Dialkyl-aryl, $C_9$-$C_{20}$-Trialkyl-aryl, Aryloxy, Aryloxy-aryl, $C_3$-$C_{20}$-Cycloalkyl, $C_4$-$C_{20}$-Alkyl-cycloalkyl, $C_4$-$C_{20}$-Cycloalkenyl oder $C_5$-$C_{20}$-Alkyl-cycloalkenyl substituiertes $C_1$-$C_{20}$-Alkyl, $C_4$-$C_{20}$-Alkyl-cycloalkyl, $C_9$-$C_{20}$-Aryl-cycloalkyl, $C_9$-$C_{30}$-Aryloxy-cycloalkyl, $C_7$-$C_{20}$-Alkyl-aryl, $C_7$-$C_{20}$-Alkoxy-aryl, $C_8$-$C_{20}$-Dialkyl-aryl, $C_7$-$C_{20}$-Halogenalkyl-aryl, Halogenaryl, Dihalogenaryl, $C_7$-$C_{20}$-Alkyl-halogenaryl, $C_8$-$C_{20}$-Dialkyl-halogenaryl, Aryloxy-aryl, $C_{13}$-$C_{30}$-Aryloxy-arylalkyl, oder durch einen gesättigten oder ungesättigten 5-, 6-, 7- oder 8-gliedrigen Heterocyclus, mit einem, zwei oder drei gleichen oder verschiedenen Heteroatomen aus der Gruppe bestehend aus Sauerstoff, Schwefel und Stickstoff, der gegebenenfalls durch ein, zwei oder drei gleiche oder unterschiedliche Substituenten aus der Gruppe bestehend aus $C_1$-$C_{20}$-Alkyl, Aryl, $C_7$-$C_{20}$-Arylalkyl, Hälogenaryl, $C_7$-$C_{20}$-Alkylaryl, $C_8$-$C_{20}$-Dialkyl-

aryl, $C_9$-$C_{20}$-Trialkyl-aryl oder ein gegebenenfalls durch $C_1$-$C_8$-Alkyl substituiertes $C_3$-$C_{20}$-Spirocycloalkyl substituiert ist, substituiertes $C_1$-$C_{20}$-Alkyl,

$R^2$ Wasserstoff, $C_1$-$C_{20}$-Alkyl, $C_1$-$C_{20}$-Halogenalkyl, gegebenenfalls durch Phenyl oder Halogenphenyl substituiert ; $C_7$-$C_{20}$-Halogenphenyl-halogenalkyl, $C_1$-$C_{20}$-Alkoxy, $C_1$-$C_{20}$-Halogenalkoxy, $C_2$-$C_{20}$-Alkenyloxy, $C_2$-$C_{20}$-Halogenalkenyloxy, $C_3$-$C_{20}$-Alkoxy-alkenyloxy, $C_2$-$C_{20}$-Alkinyloxy, $C_2$-$C_{20}$-Halogenalkinyloxy, $C_3$-$C_{20}$-Alkoxy-alkinyloxy, $C_8$-$C_{20}$-Phenyl-alkinyloxy, $C_1$-$C_{20}$-Alkylthio, $C_1$-$C_{20}$-Halogenalkylthio, $C_2$-$C_{20}$-Alkoxy-alkylthio, Formyl, Cyano, $C_7$-$C_{20}$-Phenoxy-alkoxy, $C_7$-$C_{20}$-Halogenphenoxyalkoxy, $C_7$-$C_{20}$-Phenoxy-halogenalkoxy, $C_7$-$C_{20}$Halogenphenoxy-halogenalkoxy, $C_8$-$C_{20}$-Alkoxy-phenoxyalkoxy, $C_8$-$C_{20}$-Phenoxyalkoxyalkoxy, $C_7$-$C_{20}$-Phenoxy-alkenyloxy, $C_8$-$C_{20}$-Halogenphenoxy-alkenyl, $C_8$-$C_{20}$-Phenoxy-halogenalkenyloxy, $C_8$-$C_{20}$-Halogenphenoxy-halogenalkenyloxy, $C_9$-$C_{20}$-Alkoxyphenoxy-alkenyloxy, $C_9$-$C_{20}$-Phenoxy-alkoxyalkenyloxy, $C_8$-$C_{20}$-Phenoxy-alkinyloxy, $C_8$-$C_{20}$-Halogenphenoxy-alkinyloxy, $C_8$-$C_{20}$-Phenoxy-halogenalkinyloxy, $C_8$-$C_{20}$-Halogenphenoxy-halogenalkinyloxy, $C_9$-$C_{20}$-Alkoxyphenoxy-alkinyloxy, $C_9$-$C_{20}$-Phenoxy-alkoxyalkinyloxy, $C_8$-$C_{20}$-Phenylalkinyloxy, $C_{14}$-$C_{30}$-Phenoxyphenyl-alkinyloxy, Carboxy, $C_2$-$C_{20}$-Alkoxycarbonyl, $C_2$-$C_{20}$-Alkylcarbonyloxy, $C_3$-$C_{20}$-Alkenyloxycarbonyl, $C_3$-$C_{20}$-Alkenylcarbonyloxy, $C_3$-$C_{20}$-Alkinyloxycarbonyl, $C_3$-$C_{20}$-Alkinylcarbonyloxy, Phenoxycarbonyl, Phenylcarbonyloxy, $C_3$-$C_{20}$-Alkoxycarbonyl-alkoxy, Phenoxy, $C_7$-$C_{20}$-Alkylphenoxy, Halogenphenoxy, Dihalogenphenoxy, Trihalogenphenoxy, $C_7$-$C_{20}$-Halogenalkyl-phenoxy, $C_7$-$C_{20}$-Alkoxyphenoxy, Phenoxy-phenoxy, $C_8$-$C_{12}$-Alkylendioxy-phenoxy, Phenylthio, $C_7$-$C_{20}$-Alkylphenylthio, Halogen-phenylthio, Dihalogen-phenylthio, Trihalogen-phenylthio, $C_7$-$C_{20}$-Halogenalkyl-phenylthio, $C_7$-$C_{20}$-Alkoxyphenylthio, Phenoxy-phenylthio, $C_8$-$C_{12}$-Alkylendioxy-phenylthio, Phenyl, Phenoxy-phenyl, $C_7$-$C_{20}$-Phenylalkyl, $C_{13}$-$C_{30}$-Phenoxy-phenylalkyl, $C_8$-$C_{12}$-Alkylendioxy-phenylalkoxy oder einen Rest ausgewählt aus

$-CH=N-OH$ ; 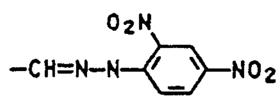 ; 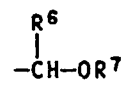 ; 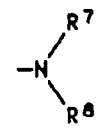 ;

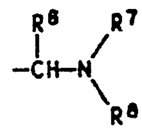 ; 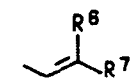 ; 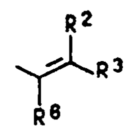 ; 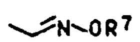 oder 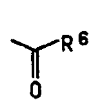 ,

$R^3$ Wasserstoff, $C_1$-$C_{20}$-Alkyl oder $C_1$-$C_3$-Halogenalkyl,

$R^4$, $R^5$ Wasserstoff, Fluor, Chlor, $C_1$-$C_3$-Alkyl, $C_1$-$C_3$-Alkoxy oder $C_1$-$C_3$-Halogenalkoxy,

$R^6$ Wasserstoff, $C_1$-$C_{20}$-Alkyl, $C_2$-$C_{20}$-Alkenyl, $C_2$-$C_{20}$-Alkinyl, Cyano, Phenyl, Halogenphenyl, $C_7$-$C_{20}$-Alkylphenyl oder $C_7$-$C_{20}$-Alkoxyphenyl,

$R^7$ Wasserstoff, $C_1$-$C_{20}$-Alkyl, $C_1$-$C_{20}$-Halogenalkyl, $C_2$-$C_{20}$-Alkoxy-alkyl, $C_2$-$C_{20}$-Alkenyl, $C_2$-$C_{20}$-Alkinyl, Aryl, Halogenphenyl, Dihalogenphenyl, Trihalogenphenyl, Cyanophenyl, $C_7$-$C_{20}$-Alkylphenyl, $C_7$-$C_{20}$-Alkoxyphenyl, $C_7$-$C_{20}$-Halogenalkyl-phenyl, $C_8$-$C_{20}$-Alkenylphenyl, $C_8$-$C_{20}$-Alkinylphenyl, Phenoxyphenyl, $C_8$-$C_{12}$-Methylendioxy-phenyl, $C_2$-$C_{20}$-Alkylcarbonyl, $C_2$-$C_{20}$-Halogenalkylcarbonyl, $C_2$-$C_{20}$-Alkoxycarbonyl, $C_5$-$C_{20}$-Alkylcyclopropylcarbonyl, $C_7$-$C_{20}$-Alkenyl- und -alkylcyclopropyl-carbonyl, $C_6$-$C_{20}$-Halogenalkyl- und -alkylcyclopropyl-carbonyl, $C_7$-$C_{20}$-Halogenalkenyl- und -alkylcyclopropyl-carbonyl, $C_8$-$C_{20}$-Halogen- und -phenyl-alkylcarbonyl, $C_8$-$C_{20}$-Halogen- und -halogenphenylalkylcarbonyl, Benzoyl, Halogenbenzoyl, $C_8$-$C_{20}$-Alkylbenzoyl, $C_8$-$C_{20}$-Alkoxybenzoyl, $C_8$-$C_{20}$-Halogenalkyl-benzoyl, $C_8$-$C_{20}$-Halogenalkoxy-benzoyl, $C_8$-$C_{20}$-Phenylalkylcarbonyl, $C_8$-$C_{20}$-Halogenphenylalkylcarbonyl, $C_9$-$C_{20}$-Alkylphenylalkylcarbonyl, $C_9$-$C_{20}$-Alkoxyphenylalkylcarbonyl, $C_9$-$C_{20}$-Halogenalkylphenylalkylcarbonyl, $C_9$-$C_{20}$-Halogenalkoxyphenylalkylcarbonyl, $C_9$-$C_{20}$-Phenylalkenylcarbonyl, $C_9$-$C_{20}$-Halogenphenylalkenylcarbonyl, $C_{10}$-$C_{20}$-Alkylphenylalkenylcarbonyl, $C_{10}$-$C_{20}$-Alkoxyphenylalkenylcarbonyl, $C_{10}$-$C_{20}$-Halogenalkylphenylalkenylcarbonyl, $C_{10}$-$C_{20}$-Halogenalkoxyphenylalkenylcarbonyl, $C_9$-$C_{20}$-Phenylalkinylcarbonyl, $C_9$-$C_{20}$-Halogenphenylalkinylcarbonyl, $C_{10}$-$C_{20}$-Alkylphenylalkinylcarbonyl, $C_{10}$-$C_{20}$-Alkoxyphenylalkinylcarbonyl, $C_{10}$-$C_{20}$-Halogenalkylphenylalkinylcarbonyl, $C_{10}$-$C_{20}$-Halogenalkoxyphenylalkinylcarbonyl, $C_2$-$C_{20}$-Alkylaminocarbonyl, $C_2$-$C_{20}$-Halogenalkylaminocarbonyl, $C_3$-$C_{20}$-Alkoxyalkylaminocarbonyl, $C_3$-$C_{20}$-Halogenalkylalkylaminocarbonyl, $C_3$-$C_{20}$-Halogenalkoxyalkylaminocarbonyl, $C_3$-$C_{20}$-Dialkylaminocarbonyl, $C_3$-$C_{20}$-Halogendialkylaminocarbonyl, $C_4$-$C_{20}$-Alkoxydialkylaminocarbonyl, $C_5$-$C_{20}$-Alkyl- und -alkoxy-aminocarbonyl, $C_4$-$C_{20}$-Halogenalkyldialkylaminocarbonyl, $C_4$-$C_{20}$-Halogenalkoxydialkylaminocarbonyl, Phenylaminocarbonyl, Halogenphenylaminocarbonyl, Dihalogenphenylaminocarbonyl, Trihalogenphenylaminocarbonyl, $C_8$-$C_{20}$-Alkylphenylaminocarbonyl, $C_8$-$C_{20}$-Alkoxyphenylaminocarbonyl, $C_8$-$C_{20}$-Halogenalkoxyphenylaminocarbonyl oder gegebenenfalls durch $C_1$-$C_8$-Alkyl substituierte Isoxazole,

$R^8$ Wasserstoff oder $C_1$-$C_{20}$-Alkyl und
X Halogen,
mit der Maßgabe, daß in Formel Ia $R^1$ nicht Phenyl bedeutet, wenn $R^2$, $R^3$, $R^4$, $R^5$ gleichzeitig für Wasserstoff stehen, und in Formel Ib $R^2$, $R^3$, $R^4$, $R^5$ nicht gleichzeitig Wasserstoff bedeuten, wenn $R^1$ für $C_4$-$C_8$-Alkyl, Phenyl, Tolyl oder Methoxyphenyl steht, sowie $R^1$ nicht Octyl bedeutet, wenn $R^2$ und $R^3$ für Wasserstoff und $R^4$ oder $R^5$ für Methyl, Methoxy oder Chlor stehen, sowie ferner mit der Maßgabe, daß in Formel Ib $R^1$ nicht n-Butyl bedeutet, wenn $R^2$ für Methyl und $R^3$, $R^4$ und $R^5$ für Wasserstoff stehen.

2. Pyranderivate der allgemeinen Formeln Ia, Ib und Ic gemäß Anspruch 1, in denen die Substituenten die folgende Bedeutung haben :
$R^1$ $C_1$-$C_{12}$-Alkyl, $C_2$-$C_{14}$-Alkenyl, $C_2$-$C_8$-Alkinyl, $C_3$-$C_8$-Cycloalkyl,phenyl, Naphthyl, Biphenylyl, $C_7$-$C_{14}$-Phenylalkyl, $C_8$-$C_{14}$-Phenylalkenyl, $C_8$-$C_{14}$-Phenylalkinyl, $C_2$-$C_8$-Alkoxy-alkyl, $C_3$-$C_{12}$-Alkoxy-alkoxy-alkyl, $C_4$-$C_{16}$-Alkoxy-alkoxy-alkoxy-alkyl, durch $C_7$-$C_{10}$-Alkyl-aryl, $C_8$-$C_{14}$-Dialkyl-phenyl, $C_9$-$C_{14}$-Trialkyl-aryl, Phenoxy, Phenoxy-phenyl, $C_3$-$C_8$-Cycloalkyl, $C_4$-$C_{12}$-Alkyl-cycloalkyl, $C_4$-$C_8$-Cycloalkenyl oder $C_5$-$C_{12}$-Alkyl-cycloalkenyl substituiertes $C_1$-$C_8$-Alkyl, $C_4$-$C_{12}$-Alkyl-cycloalkyl, $C_9$-$C_{14}$-Phenyl-cycloalkyl, $C_9$-$C_{14}$-Phenoxy-cycloalkyl, $C_7$-$C_{14}$-Alkyl-phenyl, $C_7$-$C_{14}$-Alkoxy-phenyl, $C_8$-$C_{16}$-Dialkyl-phenyl, $C_7$-$C_{14}$-Halogenalkyl-phenyl, Halogenphenyl, Dihalogenphenyl, $C_7$-$C_{14}$-Alkyl-halogenphenyl, $C_8$-$C_{14}$-Dialkyl-halogenphenyl, Phenoxy-phenyl, $C_{13}$-$C_{20}$-Phenoxy-phenylalkyl, oder durch einen gesättigten oder ungesättigten 5- oder 6-gliedrigen Heterocyclus, mit einem ein oder zwei gleichen oder verschiedenen Heteroatomen aus der Gruppe bestehend aus Sauerstoff und Stickstoff, der gegebenenfalls durch ein, zwei oder drei gleiche oder unterschiedliche Substituenten aus der Gruppe bestehend aus $C_1$-$C_8$-Alkyl, Phenyl, $C_7$-$C_{14}$-Phenylalkyl, Halogenphenyl, $C_7$-$C_{14}$-Alkylphenyl, $C_8$-$C_{16}$-Dialkyl-phenyl, $C_9$-$C_{16}$-Trialkyl-aryl oder durch ein gegebenenfalls durch $C_1$-$C_4$-Alkyl substituiertes $C_3$-$C_8$-Spirocycloalkyl substituiert ist, substituiertes $C_1$-$C_8$-Alkyl,
$R^2$ Wasserstoff, $C_1$-$C_8$-Alkyl, $C_1$-$C_8$-Halogenalkyl, gegebenenfalls durch Phenyl oder Halogenphenyl substituiert ; $C_1$-$C_8$-Alkoxy, $C_1$-$C_8$-Halogenalkoxy, $C_2$-$C_8$-Alkenyloxy, $C_2$-$C_8$-Halogenalkenyloxy, $C_3$-$C_{12}$-Alkoxyalkenyloxy, $C_2$-$C_8$-Alkinyloxy, $C_2$-$C_8$-Halogenalkinyloxy, $C_3$-$C_{12}$-Alkoxyalkinyloxy, $C_8$-$C_{16}$-Phenyl-alkinyloxy, $C_1$-$C_8$-Alkylthio, $C_1$-$C_8$-Halogenalkylthio, $C_2$-$C_{12}$-Alkoxy-alkylthio, Formyl, Cyano, $C_7$-$C_{14}$-Phenoxyalkoxy, $C_7$-$C_{14}$-Halogenphenoxy-alkoxy, $C_7$-$C_{14}$-Phenoxy-halogenalkoxy, $C_7$-$C_{14}$-Halogenphenoxy-halogenalkoxy, $C_8$-$C_{16}$-Alkoxyphenoxyalkoky, $C_8$-$C_{16}$-Phenoxy-alkoxyalkoxy, $C_7$-$C_{16}$-Phenoxy-alkenyloxy, $C_8$-$C_{16}$-Halogenphenoxy-alkenyl, $C_8$-$C_{16}$-Phenoxy-halogenalkenyloxy, $C_8$-$C_{16}$-Halogenphenoxy-halogenalkenyloxy, $C_9$-$C_{16}$-Alkoxyphenoxy-alkenyloxy, $C_9$-$C_{16}$-Phenoxy-alkoxyalkenyloxy, $C_8$-$C_{14}$-Phenoxy-alkinyloxy,$C_8$-$C_{14}$-Halogenphenoxy-alkinyloxy, $C_8$-$C_{14}$-Phenoxy-halogenalkinyloxy, $C_8$-$C_{14}$-Halogenphenoxy-halogenalkinyloxy, $C_9$-$C_{16}$-Alkoxyphenoxy-alkinyloxy, $C_9$-$C_{16}$-Phenoxy-alkoxyalkinyloxy, $C_8$-$C_{14}$-Phenylalkinyloxy, $C_{14}$-$C_{20}$-Phenoxyphenyl-alkinyloxy, Carboxy, $C_2$-$C_8$-Alkoxycarbonyl, $C_2$-$C_8$-Alkylcarbonyloxy, $C_3$-$C_{12}$-Alkenyloxycarbonyl, $C_3$-$C_{12}$-Alkenylcarbonyloxy, $C_3$-$C_{12}$-Alkinyloxycarbonyl, $C_3$-$C_{12}$-Alkinylcarbonyloxy, Phenoxycarbonyl, Phenylcarbonyloxy, $C_3$-$C_{12}$-Alkoxycarbonyl-alkoxy, Phenoxy, $C_7$-$C_{14}$-Alkylphenoxy, Halogenphenoxy, Dihalogenphenoxy, Trihalogenphenoxy, $C_7$-$C_{14}$-Halogenalkyl-phenoxy, $C_7$-$C_{14}$-Alkoxyphenoxy, Phenoxy-phenoxy, $C_8$-$C_{14}$-Alkylendioxy-phenoxy, Phenylthio, $C_7$-$C_{14}$-Alkylphenylthio, Halogenphenylthio, Dihalogen-phenylthio, Trihalogen-phenylthio, $C_7$-$C_{14}$-Halogenalkyl-phenylthio, $C_7$-$C_{14}$-Alkoxyphenylthio, Phenoxy-phenylthio, $C_8$-$C_{14}$-Alkylendioxy-phenylthio, Phenyl, Phenoxy-phenyl, $C_7$-$C_{14}$-Phenylalkyl, $C_{13}$-$C_{20}$-Phenoxy-phenylalkyl, $C_8$-$C_{14}$-Alkylendioxy-phenylalkyloxy oder einen Rest ausgewählt aus

$-CH=N-OH$ ; 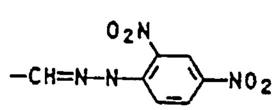 ; 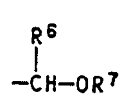 ; 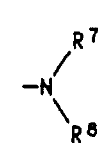 ;

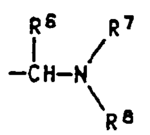 ; 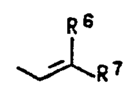 ; 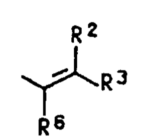 ; 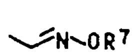 oder 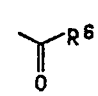 ,

$R^3$ Wasserstoff, $C_1$-$C_8$-Alkyl oder $C_1$-$C_2$-Halogenalkyl,
$R^4$, $R^5$ Wasserstoff, Fluor, Chlor, $C_1$-$C_3$-Alkyl, $C_1$-$C_3$-Alkoxy oder $C_1$-$C_3$-Halogenalkoxy,
$R^6$ Wasserstoff, $C_1$-$C_8$-Alkyl, $C_2$-$C_8$-Alkenyl, $C_2$-$C_8$-Alkinyl, Cyano, Phenyl, Halogenphenyl, $C_7$-$C_{14}$-Alkylphenyl oder $C_7$-$C_{14}$-Alkoxyphenyl,

$R^7$ Wasserstoff, $C_1$-$C_8$-Alkyl, $C_1$-$C_8$-Halogenalkyl, $C_2$-$C_8$-Alkoxy-alkyl, $C_2$-$C_8$-Alkenyl, $C_2$-$C_8$-Alkinyl, Aryl, Halogenphenyl, Dihalogenphenyl, Trihalogenphenyl, Cyanophenyl, $C_7$-$C_{14}$-Alkylphenyl, $C_7$-$C_{14}$-Alkoxyphenyl, $C_7$-$C_{14}$-Halogenalkyl-phenyl, $C_8$-$C_{16}$-Alkenylphenyl, $C_8$-$C_{16}$-Alkinylphenyl, Phenoxyphenyl, $C_8$-$C_{16}$-Methylendioxy-phenyl, $C_2$-$C_8$-Alkylcarbonyl, $C_2$-$C_8$-Halogenalkylcarbonyl, $C_2$-$C_8$-Alkoxycarbonyl, $C_5$-$C_{12}$-Alkylcyclopropyl-carbonyl, $C_7$-$C_{14}$-Alkenyl- und -alkylcyclopropyl-carbonyl, $C_6$-$C_{14}$-Halogenalkyl- und -alkylcyclopropyl-carbonyl, $C_7$-$C_{14}$-Halogenalkenyl- und -alkylcyclopropyl-carbonyl, $C_8$-$C_{16}$-Halogen- und -phenyl-alkylcarbonyl, $C_8$-$C_{16}$-Halogen- und -halogenphenylalkylcarbonyl, Benzoyl, Halogenbenzoyl, $C_8$-$C_{14}$-Alkylbenzoyl, $C_8$-$C_{14}$-Alkoxybenzoyl, $C_8$-$C_{14}$-Halogenalkyl-benzoyl, $C_8$-$C_{14}$-Halogenalkoxy-benzoyl, $C_8$-$C_{14}$-Phenylalkylcarbonyl, $C_8$-$C_{14}$-Halogenphenylalkylcarbonyl, $C_9$-$C_{16}$-Alkylphenylalkylcarbonyl, $C_9$-$C_{16}$-Alkoxyphenylalkylcarbonyl, $C_9$-$C_{16}$-Halogenalkylphenylalkylcarbonyl, $C_9$-$C_{16}$-Halogenalkoxyphenylalkylcarbonyl, $C_9$-$C_{16}$-Phenylalkenylcarbonyl, $C_9$-$C_{16}$-Halogenphenylalkenylcarbonyl, $C_{10}$-$C_{16}$-Alkylphenylalkenylcarbonyl, $C_{10}$-$C_{16}$-Alkoxyphenylalkenylcarbonyl, $C_{10}$-$C_{16}$-Halogenalkylphenylalkenylcarbonyl,$C_{10}$-$C_{16}$-Halogenalkoxyphenylalkenylcarbonyl, $C_9$-$C_{16}$-Phenylalkinylcarbonyl, $C_9$-$C_{16}$-Halogenphenylalkinylcarbonyl, $C_{10}$-$C_{16}$-Alkylphenylalkinylcarbonyl, $C_{10}$-$C_{16}$-Alkoxyphenylalkinylcarbonyl, $C_{10}$-$C_{16}$-Halogenalkylphenylalkinylcarbonyl, $C_{10}$-$C_{16}$-Halogenalkoxyphenylalkinylcarbonyl, $C_2$-$C_8$-Alkylaminocarbonyl, $C_2$-$C_8$-Halogenalkylaminocarbonyl, $C_3$-$C_{10}$-Alkylalkylaminocarbonyl, $C_3$-$C_{10}$-Alkoxyalkylaminocarbonyl, $C_3$-$C_{10}$-Halogenalkylalkylaminocarbonyl, $C_3$-$C_{10}$-Halogenalkoxyalkylaminocarbonyl, $C_3$-$C_{10}$-Dialkylaminocarbonyl, $C_3$-$C_8$-Halogendialkylaminocarbonyl, $C_4$-$C_{12}$-Alkyldialkylaminocarbonyl, $C_4$-$C_{12}$-Alkoxydialkylaminocarbonyl, $C_5$-$C_{20}$-Alkyl- und -alkoxy-aminocarbonyl, $C_4$-$C_{12}$-Halogenalkyldialkylaminocarbonyl, $C_4$-$C_{12}$-Halogenalkoxydialkylaminocarbonyl, Phenylaminocarbonyl, Halogenphenylaminocarbonyl, Dihalogenphenylaminocarbonyl, Trihalogenphenylaminocarbonyl, $C_8$-$C_{14}$-Alkylphenylaminocarbonyl, $C_8$-$C_{14}$-Alkoxyphenylaminocarbonyl, $C_8$-$C_{14}$-Halogenalkoxyphenylaminocarbonyl oder gegebenenfalls durch $C_1$-$C_4$-Alkyl substituierte Isoxazole,

$R^8$ Wasserstoff oder $C_1$-$C_8$-Alkyl und

X Halogen,

mit der Maßgabe, daß in Formel Ia $R^1$ nicht Phenyl bedeutet, wenn $R^2$, $R^3$, $R^4$, $R^5$ gleichzeitig für Wasserstoff stehen, und in Formel Ib $R^2$, $R^3$, $R^4$, $R^5$ nicht gleichzeitig Wasserstoff bedeuten, wenn $R^1$ für $C_4$-$C_8$-Alkyl, Phenyl, Tolyl oder Methoxyphenyl steht, sowie $R^1$ nicht Octyl bedeutet, wenn $R^2$ und $R^3$ für Wasserstoff und $R^4$ oder $R^5$ für Methyl, Methoxy oder Chlor stehen, sowie ferner mit der Maßgabe, daß in Formel Ib $R^1$ nicht n-Butyl bedeutet, wenn $R^2$ für Methyl und $R^3$, $R^4$ und $R^5$ für Wasserstoff stehen.

3. Verfahren zur Herstellung von Pyranderivaten der allgemeinen Formeln Ia, Ib oder Ic gemäß Anspruch 1, dadurch gekennzeichnet, daß man einen Vinylether der allgemeinen Formel II

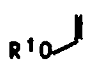

(II)

in Gegenwart eines Radikalinhibitors und einer basischen Verbindung bei Temperaturen von 130 bis 280°C und Drücken von 1 bis 300 bar in an sich bekannter Weise

a) mit einem monomer vorliegenden α,β-ungesättigten Aldehyd oder Keton III

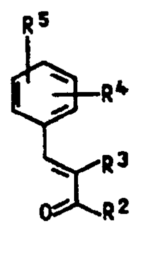

(III)

oder

b) mit einem dimer vorliegenden α,β-ungesättigten Aldehyd oder Keton IIIa

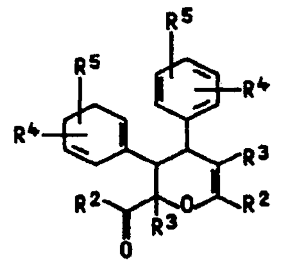

(IIIa)

umsetzt, und die Verbindungen Ib gegebenenfalls
c) zu den Verbindungen Ia hydriert, oder
d) für den Fall, daß $R^2$ = H ist, hydroformyliert, oder
e) für den Fall, daß $R^2$ und $R^3$ = H ist, mit einer Trihalogenmethanverbindung $CHX_3$ zu den Verbindungen Ic umsetzt.

4. Schälingsbekämpfungsmittel, enthaltend ein Pyranderivat Ia, Ib oder Ic gemäss Anspruch 1.

5. Schädlingsbekämpfungsmittel nach Anspruch 4, enthaltend ein Pyranderivat Ia, Ib oder Ic gemäß Anspruch 2, sowie hierfür übliche Trägerstoffe.

6. Schädlingsbekämpfungsmittel gemäß, einem der Ansprüche 4 oder 5, dadurch gekennzeichnet, daß es 0,1 bis 95 Gew.% eines Pyranderivates Ia, Ib oder Ic gemäss Anspruch 1 Gew.2 enthält.

7. Verfahren zur Bekämpfung von Schädlingen, dadurch gekennzeichnet, daß man die Schädlinge und-/oder die von Schädlingen freizuhaltenden Flächen und/oder Räume mit einer für Schädlinge wirksamen Menge eines Pyranderivates der Formeln Ia, Ib oder Ic gemäß Anspruch 1 Gew. mit einem Schädlingsbkämpfunps, mittel gemäss Anspruch 3 behandelt.

**Patentansprüche für den Vertragsstaat : ES**

1. Verfahren zur Herstellung von Pyranderivaten der allgemeinen Formeln Ia, Ib oder Ic

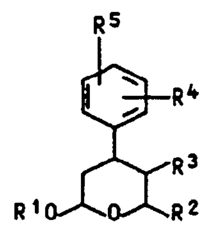

(Ia)

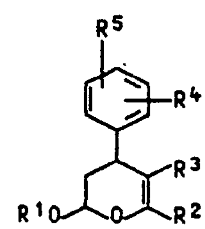

(Ib)

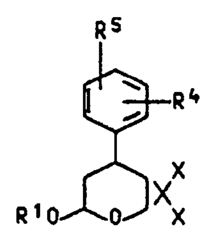

(Ic)

in denen die Substituenten folgente Bedeutung haben :

$R^1$ $C_1$-$C_{20}$-Alkyl, $C_2$-$C_{20}$-Alkenyl, $C_2$-$C_{20}$-Alkinyl, $C_3$-$C_{20}$-Cycloalkyl, Aryl, Biphenylyl, $C_7$-$C_{30}$-Arylalkyl, $C_8$-$C_{30}$-Arylalkenyl, $C_8$-$C_{30}$-Aryl-alkinyl, $C_2$-$C_{20}$-Alkoxy-alkyl, $C_3$-$C_{20}$-Alkoxy-alkoxy-alkyl, $C_4$-$C_{30}$-Alkoxy-alkoxy-alkoxy-alkyl, durch $C_7$-$C_{20}$-Alkyl-aryl, $C_8$-$C_{20}$-Dialkyl-aryl, $C_9$-$C_{20}$-Trialkyl-aryl, Aryloxy, Aryloxy-aryl, $C_3$-$C_{20}$-Cycloalkyl, $C_4$-$C_{20}$-Alkyl-cycloalkyl, $C_4$-$C_{20}$-Cyclo-alkenyl oder $C_5$-$C_{20}$-Alkyl-cycloalkenyl substituiertes $C_1$-$C_{20}$-Alkyl, $C_4$-$C_{20}$-Alkyl-cycloalkyl, $C_9$-$C_{20}$-Aryl-cycloalkyl, $C_9$-$C_{30}$-Aryloxy-cycloalkyl, $C_7$-$C_{20}$-Alkyl-aryl, $C_7$-$C_{20}$-Alkoxy-aryl, $C_8$-$C_{20}$-Dialkyl-aryl, $C_7$-$C_{20}$-Halogenalkyl-aryl, Halogenaryl, Dihalogenaryl, $C_7$-$C_{20}$-Alkyl-halogenaryl, $C_8$-$C_{20}$-Dialkyl-halogenaryl, Aryloxy-aryl, $C_{13}$-$C_{30}$-Aryloxy-arylalkyl, oder durch einen gesättigten oder ungesättigten 5-, 6-, 7- oder 8-gliedrigen Heterocyclus, mit einem, zwei oder drei gleichen oder verschiedenen Heteroatomen aus der Gruppe bestehend aus Sauerstoff, Schwefel und Stickstoff, der gegebenenfalls durch ein, zwei oder drei gleiche oder unterschiedliche Substituenten aus der Gruppe bestehend aus $C_1$-$C_{20}$-Alkyl, Aryl, $C_7$-$C_{20}$-Arylalkyl, Halogenaryl, $C_7$-$C_{20}$-Alkylaryl, $C_8$-$C_{20}$-Dialkyl-aryl, $C_9$-$C_{20}$-Trialkyl-aryl oder ein gegebenenfalls durch $C_1$-$C_8$-Alkyl substituertes $C_3$-$C_{20}$Spirocycloalkyl substituiert ist, subtituiertes $C_1$-$C_{20}$-Alkyl,

$R^2$ Wasserstoff, $C_1$-$C_{20}$-Alkyl, $C_1$-$C_{20}$-Halogenalkyl, gegebenenfalls durch Phenyl oder Halogenphenyl substituiert ; $C_1$-$C_{20}$-Alkoxy, $C_1$-$C_{20}$-Halogenalkoxy, $C_2$-$C_{20}$-Alkenyloxy, $C_2$-$C_{20}$-Halogenalkenyloxy, $C_3$-$C_{20}$-Alkoxy-alkenyloxy, $C_2$-$C_{20}$-Alkinyloxy, $C_2$-$C_{20}$-Halogenalkinyloxy, $C_3$-$C_{20}$-Alkoxy-alkinyloxy, $C_8$-$C_{20}$-Phenyl-alkinyloxy, $C_1$-$C_{20}$-Alkylthio, $C_1$-$C_{20}$-Halogenalkylthio, $C_2$-$C_{20}$-Alkoxy-alkylthio, Formyl,

Cyano, $C_7$-$C_{20}$-Phenoxy-alkoxy, $C_7$-$C_{20}$-Halogenphenoxy-alkoxy, $C_7$-$C_{20}$-Phenoxy-halogenalkoxy, $C_7$-$C_{20}$-Halogenphenoxy-halogenalkoxy, $C_8$-$C_{20}$-Alkoxy-phenoxyalkoxy, $C_8$-$C_{20}$-Phenoxy-alkoxyalkoxy, $C_7$-$C_{20}$-Phenoxy-alkenyloxy, $C_8$-$C_{20}$-Halogenphenoxy-alkenyl, $C_8$-$C_{20}$-Phenoxy-halogenalkenyloxy, $C_8$-$C_{20}$-Halogenphenoxy-halogenalkenyloxy, $C_9$-$C_{20}$-Alkoxyphenoxy-alkenyloxy, $C_9$-$C_{20}$-Phenoxy-alkoxy-alkenyloxy, $C_8$-$C_{20}$-Phenoxyhalogenalkinyloxy, $C_8$-$C_{20}$-Halogenphenoxy-halogenalkinyloxy, $C_9$-$C_{20}$-Alkoxy-phenoxy-alkinyloxy, $C_9$-$C_{20}$-Phenoxy-alkoxyalkinyloxy, $C_8$-$C_{20}$-Phenylalkinyloxy, $C_{14}$-$C_{30}$-Phenoxyphenyl-alkinyloxy, Carboxy, $C_2$-$C_{20}$-Alkoxycarbonyl, $C_2$-$C_{20}$-Alkylcarbonyloxy, $C_3$-$C_{20}$-Alkeyloxycarbonyl, $C_3$-$C_{20}$-Alkenylcarbonyloxy, $C_3$-$C_{20}$-Alkinyloxycarbonyl, $C_3$-$C_{20}$-Alkinylcarbonyloxy, Phenoxycarbonyl, Phenylcarbonyloxy, $C_3$-$C_{20}$-Alkoxycarbonyl-alkoxy, Phenoxy, $C_7$-$C_{20}$-Alkylphenoxy, Halogenphenoxy, Dihalogenphenoxy, Trihalogenphenoxy, $C_7$-$C_{20}$-Halogenalkyl-phenoxy, $C_7$-$C_{20}$-Alkoxy-phenoxy, Phenoxy-phenoxy, $C_8$-$C_{12}$-Alkylendioxy-phenoxy, Phenylthio, $C_7$-$C_{20}$-Alkylphenylthio, Halogen-phenylthio, Dihalogen-phenylthio, Trihalogen-phenylthio, $C_7$-$C_{20}$-Halogenalkyl-phenylthio, $C_7$-$C_{20}$-Alkoxyphenylthio, Phenoxy-phenylthio, $C_8$-$C_{12}$-Alkylendioxy-phenylthio, Phenyl, Phenoxy-phenyl, $C_7$-$C_{20}$-Phenylalkyl, $C_{13}$-$C_{30}$-Phenoxy-phenylalkyl, $C_8$-$C_{12}$-Alkylendioxy-phenylalkoxy oder einen Rest ausgewählt aus

$-CH=N-OH$ ; 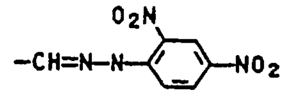 ; 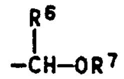 ; 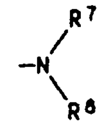 ;

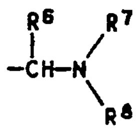 ; 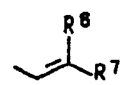 ; 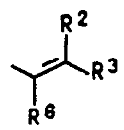 ; 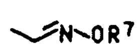 oder 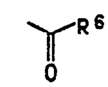 ,

$R^3$ Wasserstoff, $C_1$-$C_{20}$-Alkyl oder $C_1$-$C_3$-Halogenalkyl,

$R^4$, $R^5$ Wasserstoff, Fluor, Chlor, $C_1$-$C_3$-Alkyl, $C_1$-$C_3$-Alkoxy oder $C_1$-$C_3$-Halogenalkoxy,

$R^6$ Wasserstoff, $C_1$-$C_{20}$-Alkyl, $C_2$-$C_{20}$-Alkenyl, $C_2$-$C_{20}$-Alkinyl, Cyano, Phenyl, Halogenphenyl, $C_7$-$C_{20}$-Alkylphenyl oder $C_7$-$C_{20}$-Alkoxyphenyl,

$R^7$ Wasserstoff $C_1$-$C_{20}$-Alkyl, $C_1$-$C_{20}$-Halogenalkyl, $C_2$-$C_{20}$-Alkoxy-alkyl, $C_2$-$C_{20}$-Alkenyl, $C_2$-$C_{20}$-Alkinyl, Aryl, Halogenphenyl, Dihalogenphenyl, Trihalogenphenyl, Cyanophenyl, $C_7$-$C_{20}$-Alkylphenyl, $C_7$-$C_{20}$-Alkoxyphenyl, $C_7$-$C_{20}$-Halogenalkyl-phenyl, $C_8$-$C_{20}$-Alkenylphenyl, $C_8$-$C_{20}$-Alkinylphenyl, Phenoxyphenyl, $C_8$-$C_{12}$-Methylendioxy-phenyl, $C_2$-$C_{20}$-Alkylcarbonyl, $C_2$-$C_{20}$-Halogenalkylcarbonyl, $C_2$-$C_{20}$-Alkoxycarbonyl, $C_5$-$C_{20}$-Alkylcyclopropylcarbonyl, $C_7$-$C_{20}$-Alkenyl- und -alkylcyclopropyl-carbonyl, $C_6$-$C_{20}$-Halogenalkyl- und -alkylcyclopropyl-carbonyl, $C_7$-$C_{20}$-Halogenalkenyl- und -alkylcyclopropyl-carbonyl, $C_8$-$C_{20}$-Halogen- und -phenyl-alkylcarbonyl, $C_8$-$C_{20}$-Halogen- und -halogenphenylalkylcarbonyl, Benzoyl, Halogenbenzoyl, $C_8$-$C_{20}$-Alkylbenzoyl, $C_8$-$C_{20}$-Alkoxybenzoyl, $C_8$-$C_{20}$-Halogenalkyl-benzoyl, $C_8$-$C_{20}$-Halogenalkoxy-benzoyl, $C_8$-$C_{20}$-Phenylalkylcarbonyl, $C_8$-$C_{20}$-Halogenphenylalkylcarbonyl, $C_9$-$C_{20}$-Alkylphenylalkylcarbonyl, $C_9$-$C_{20}$-Alkoxyphenylalkulcarbonyl, $C_9$-$C_{20}$-Halogenalkylphenylalkylcarbonyl, $C_9$-$C_{20}$-Halogenalkoxyphenylalkylcarbonyl, $C_9$-$C_{20}$-Phenylalkenylcarbonyl, $C_9$-$C_{20}$-Halogenphenylalkenylcarbonyl, $C_{10}$-$C_{20}$-Alkylphenylalkenylcarbonyl, $C_{10}$-$C_{20}$-Alkoxyphenylalkenylcarbonyl, $C_{10}$-$C_{20}$-Halogenalkylphenylalkenylcarbonyl, $C_{10}$-$C_{20}$-Halogenalloxyphenylalkenylcarbonyl, $C_9$-$C_{20}$-Phenylalkinylcarbonyl, $C_9$-$C_{20}$-Halogenphenylalkinylcarbonyl, $C_{10}$-$C_{20}$-Alkylphenylalkinylcarbonyl, $C_{10}$-$C_{20}$-Alkoxyphenylalkinylcarbonyl, $C_{10}$-$C_{20}$-Halogenalkylphenylalkinylcarbonyl, $C_{10}$-$C_{20}$-Halogenalkoxyphenylalkinylcarbonyl, $C_2$-$C_{20}$-Alkylaminocarbonyl, $C_2$-$C_{20}$-Halogenalkylaminocarbonyl, $C_3$-$C_{20}$-Alkoxyalkylaminocarbonyl, $C_3$-$C_{20}$-Halogenalkylalkylaminocarbonyl, $C_3$-$C_{20}$-Halogenalkoxyalkylaminocarbonyl, $C_3$-C20-Dialkylaminocarbonyl, $C_3$-$C_{20}$-Halogendialkylaminocarbonyl, $C_4$-$C_{20}$-Alkoxydialkylamino-carbonyl, $C_5$-$C_{20}$-Alkyl- und -alkoxy-aminocarbonyl, $C_4$-$C_{20}$-Halogenalkyldiakylaminocarbonyl, $C_4$-$C_{20}$-Halogenalkoxydialkylaminocarbonyl, Phenylaminocarbonyl, Halogenphenylaminocarbonyl, Dihalogenphenylaminocarbonyl, Trihalogenphenylaminocarbonyl, $C_8$-$C_{20}$-Alkylphenylaminocarbonyl, $C_8$-$C_{20}$-Alkoxyphenylaminocarbonyl, $C_8$-$C_{20}$-Halogenalkoxyphenylaminocarbonyl oder gegebenenfalls durch $C_1$-$C_8$-Alkyl subtituierte Isoxazole,

$R^8$ Wasserstoff oder $C_1$-$C_{20}$-Alkyl und

X Halogen,

mit der Maßgabe, daß im Fall von Verbindung Ia $R^1$ nicht Phenyl bedeutet, wenn $R^2$ bis $R^5$ gleichzeitig für Wasserstoff stehen, und im Fall von Verbindung Ib $R^2$ bis $R^5$ nicht gleichzeitig Wasserstoff bedeuten, wenn $R^1$ für $C_4$-$C_8$-Alkyl, Phenyl, Tolyl oder Methoxyphenyl, sowie $R^1$ nicht Octyl bedeutet, wenn $R^2$ und $R^3$ für Wasserstoff und $R^4$ oder $R^5$ für Methyl, Methoxy oder Chlor stehen, sowie ferner mit der Maßgabe, daß in

Formel Ib $R^1$ nicht n-Butyl bedeutet, wenn $R^2$ für Methyl und $R^3$, $R^4$ und $R^5$ für Wasserstoff stehen, dadurch gekennzeichnet, daß man einen Vinylether der allgemeinen Formel II

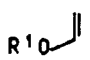

(II)

in Gegenwart eines Radikalinhibitors und einer basischen Verbindung bei Tempreraturen von 130 bis 280°C und Drücken von 1 bis 300 bar in an sich bekannter Weise

a) mit einem monomer vorliegenden α,β-ungesättigten Aldehyd oder Keton III

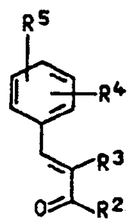

(III)

oder

b) mit einem dimer vorliegenden α,β-ungesättigten Aldehyd oder Keton IIIa

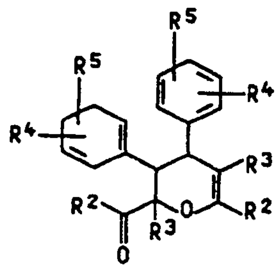

(IIIa)

umsetzt, und die Verbindungen Ib gegebenenfalls

c) zu den Verbindungen Ia hydriert, oder

d) für den Fall, daß $R^2$ = H ist, hydroformuliert oder

e) für den Fall, daß $R^2$ und $R^3$ = H ist, mit einer Trihalogenmethan-verbindung $CHX_3$ zu den Verbindungen Ia umsetzt.

2. Verfahren nach Anspruch 1 zur Herstellung von Pyranderivaten der Formeln Ia, Ib und Ic gemäß Anspruch 1, in denen die Substituenten die folgende Bedeutung haben :

$R^1$ $C_1$-$C_{12}$-Alkyl, $C_2$-$C_{14}$-Alkenyl, $C_2$-$C_8$-Alkinyl, $C_3$-$C_8$-Cycloalkyl, Phenyl, Naphthyl, Biphenylyl, $C_7$-$C_{14}$-Phenylalkyl, $C_8$-$C_{14}$-Phenylalkenyl, $C_8$-$C_{14}$-Phenylalkinyl, $C_2$-$C_8$-Alkoxy-alkyl, $C_3$-$C_{12}$-Alkoxy-alkoxy-alkyl, $C_4$-$C_{16}$-Alkoxy-alkoxy-alkoxy-alkyl, durch $c_7$-$C_{10}$-Alkyl-aryl, $C_8$-$C_{14}$-Dialkyl-phenyl, $C_9$-$C_{14}$-Trialkyl-aryl, Phenoxy, Phenoxy-phenyl, $C_3$-$C_8$-Cycloalkyl, $C_4$-$C_{12}$-Alkyl-cycloalkyl, $C_4$-$C_8$-Cycloalkenyl oder $C_5$-$C_{12}$-Alkyl-cycloalkenyl substituiertes $C_1$-$C_8$-Alkyl, $C_4$-$C_{12}$-Alkyl-cycloalkyl, $C_9$-$C_{14}$-Phenyl-cycloalkyl, $C_9$-$C_{14}$-Phenoxy-cycloalkyl, $C_7$-$C_{14}$-Alkyl-phenyl, $C_7$-$C_{14}$-Alkoxy-phenyl, $C_8$-$C_{16}$-Dialkyl-phenyl, $C_7$-$C_{14}$-Halogenalkyl-phenyl, Halogenphenyl, Dihalogenphenyl, $C_7$-$C_{14}$-Alkyl-halogenphenyl, $C_8$-$C_{14}$-Dialkyl-halogenphenyl, Phenoxy-phenyl, $C_{13}$-$C_{20}$-Phenoxy-phenylalkyl, oder durch einen gesättigten oder ungesättigten 5- oder 6-gliedrigen Heterocyclus, mit einem ein oder zwei gleichen oder verschiedenen Heteroatomen aus der Gruppe bestehend aus Sauerstoff und Stickstoff, der gegebenenfalls durch ein, zwei oder drei gleiche oder unterschiedliche Substituenten aus der Gruppe bestehend aus $C_1$-$C_8$-Alkyl, Phenyl, $c_7$-$C_{14}$-Phenylalkyl, Halogenphenyl, $C_7$-$C_{14}$-Alkulphenyl, $C_8$-$C_{16}$-Dialkyl-phenyl, $C_9$-$C_{16}$-Trialkyl-aryl oder durch ein gegenbenenfalls durch $C_1$-$C_4$-Alkyl substituiertes $C_3$-$C_8$-Spirocycloalkyl subtituiert ist, subtituiertes $C_1$-$C_8$-Alkyl,

$R^2$ Wasserstoff, $C_1$-$C_8$-Alkyl, $C_1$-$C_8$-Halogenalkyl, gegebenenfalls durch Phenyl oder Halogenphenyl subtituiert ; $C_1$-$C_8$-Alkoxy, $C_1$-$C_8$-Halogenalkoxy, $C_2$-$C_8$-Alkenyloxy, $C_2$-$C_8$-Halogenalkenyloxy, $C_3$-$C_{12}$-Alkoxy-

alkenyloxy, $C_2$-$C_8$-Alkinyloxy, $C_2$-$C_8$-Halogenalkinyloxy, $C_3$-$C_{12}$-Alkoxy-alkinyloxy, $C_8$-$C_{16}$-Phenyl-alkinyloxy, $C_1$-$C_8$-Alkylthio, $C_1$-$C_8$-Halogenalkylthio, $C_2$-$C_{12}$-Alkoxy-alkylthio, Formyl, Cyano, $C_7$-$C_{14}$-Phenoxy-alkoxy, $C_7$-$C_{14}$-Halogenphenoxy-alkoxy, $C_7$-$C_{14}$-Phenoxy-halogenalkoxy, $C_7$-$C_{14}$-Halogenphenoxy-halogenalkoxy, $C_8$-$C_{16}$-Alkoxyphenoxyalkoxy, $C_8$-$C_{16}$-Phenoxy-alkoxyalkoxy, $C_7$-$C_{16}$-Phenoxy-alkenyloxy, $C_8$-$C_{16}$-Halogenphenoxy-alkenyl, $C_8$-$C_{16}$-Phenoxy-halogenalkenyloxy, $C_8$-$C_{16}$-Halogenphenoxy-halogenalkenyloxy, $C_9$-$C_{16}$-Alkoxyphenoxy-alkenyloxy, $C_9$-$C_{16}$-Phenoxy-alkoxyalkenyloxy, $C_8$-$C_{14}$-Phenoxy-alkinyloxy, $C_8$-$C_{14}$-Halogenphenoxy-alkinyloxy, $C_8$-$C_{14}$-Phenoxy-halogenalkinyloxy, $C_8$-$C_{14}$-Halogenphenoxy-halogenalkinyloxy, $C_9$-$C_{16}$-Alkoxyphenoxy-alkinyloxy, $C_9$-$C_{16}$-Phenoxy-alkoxyalkinyloxy, $C_8$-$C_{14}$-Phenylalkinyloxy, $C_{14}$-$C_{20}$-Phenoxyphenyl-alkinyloxy, Carboxy, $C_2$-$C_8$-Alkoxycarbonyl, $C_2$-$C_8$-Alkylcarbonyloxy, $C_3$-$C_{12}$-Alkenyloxycarbonyl, $C_3$-$C_{12}$-Alkenylcarbonyloxy, $C_3$-$C_{12}$-Alkinyloxycarbonyl, $C_3$-$C_{12}$-Alkinylcarbonyloxy, Phenoxycarbonyl, Phenylcarbonyloxy, $C_3$-$C_{12}$-Alkoxycarbonyl-alkoxy, Phenoxy $C_7$-$C_{14}$-Alkylphenoxy, Halogenphenoxy, Dihalogenphenoxy, Trihalogenphenoxy, $C_7$-$C_{14}$-Halogenalkyl-phenoxy, $C_7$-$C_{14}$-Alkoxyphenoxy, Phenoxy-phenoxy, $C_8$-$C_{14}$-Alkylendioxy-phenoxy, Phenylthio, $C_7$-$C_{14}$-Alkylphenylthio, Halogen-phenylthio, Dihalogen-phenylthio, Trihalogen-phenylthio, $C_7$-$C_{14}$-Halogenalkyl-phenylthio, $C_7$-$C_{14}$-Alkoxyphenylthio, Phenoxy-phenylthio, $C_8$-$C_{14}$-Alkylendioxy-phenylthio, Phenyl, Phenoxy-phenyl, $C_7$-$C_{14}$-Phenylalkyl, $C_{13}$-$C_{20}$-Phenoxy-phenylalkyl, $C_8$-$C_{14}$-Alkylendioxy-phenylalkyloxy oder einen Rest ausgewählt aus

$-CH{=}N{-}OH$ ; 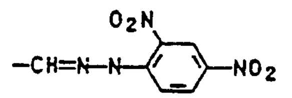 ; 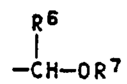 ; 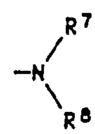 ;

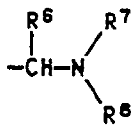 ; 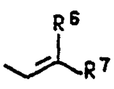 ; 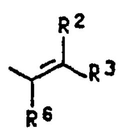 ; 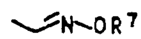 oder 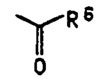

$R^3$ Wasserstoff, $C_1$-$C_8$-Alkyl oder $C_1$-$C_2$-Halogenalkyl,
$R^4$, $R^5$ Wasserstoff, Fluor, Chlor, $C_1$-$C_3$-Alkyl, $C_1$-$C_3$-Alkoxy oder $C_1$-$C_3$-Halogenalkoxy,
$R^6$ Wasserstoff, $C_1$-$C_8$-Alkyl, $C_2$-$C_8$-Alkenyl, $C_2$-$C_8$-Alkinyl, Cyano, Phenyl, Halogenphenyl, $C_7$-$C_{14}$Alkylphenyl oder $C_7$-$C_{14}$-Alkoxyphenyl,
$R^7$ Wasserstoff, $C_1$-$C_8$-Alkyl, $C_1$-$C_8$-Halogenalkyl, $C_2$-$C_8$-Alkoxy-alkyl, $C_2$-$C_8$-Alkenyl, $C_2$-$C_8$-Alkinyl, Aryl, Halogenphenyl, Dihalogenphenyl, Trihalogenphenyl, Cyanophenyl, $C_7$-$C_{14}$-Alkylphenyl, $C_7$-$C_{14}$-Alkoxyphenyl, $C_7$-$C_{14}$-Halogenalkyl-phenyl, $C_8$-$C_{16}$-Alkenylphenyl, $C_8$-$C_{16}$-Alkinylphenyl, Phenoxyphenyl, $C_8$-$C_{16}$-Methylendioxy-phenyl, $C_2$-$C_8$-Alkylcarbonyl, $C_2$-$C_8$-Halogenalkylcarbonyl, $C_2$-$C_8$-Alkoxycarbonyl, $C_5$-$C_{12}$-Alkylcyclopropyl-carbonyl, $C_7$-$C_{14}$-Alkenyl- und -alkylcyclopropyl-carbonyl, $C_6$-$C_{14}$-Halogenalkyl- und -alkylcyclopropyl-carbonyl, $C_7$-$C_{14}$-Halogenalkenyl- und -alkylcyclopropyl-carbonyl, $C_8$-$C_{16}$-Halogen- und -phenyl-alkylcarbonyl, $C_8$-$C_{16}$-Halogen- und -halogenphenylalkylcarbonyl, Benzoyl, Halogenbenzoyl, $C_8$-$C_{14}$-Alkylbenzoyl, $C_8$-$C_{14}$-Alkoxybenzoyl, $C_8$-$C_{14}$-Halogenalkyl-benzoyl, $C_8$-$C_{14}$-Halogenalkoxy-benzoyl, $C_8$-$C_{14}$-Phenylakylcarbonyl, $C_8$-$C_{14}$-Halogenphenylalkylcarbonyl, $C_9$-$C_{16}$-Alkylphenylalkylcarbonyl, $C_9$-$C_{16}$-Alkoxyphenylalkylcarbonyl, $C_9$-$C_{16}$-Halogenalkylphenylalkylcarbonyl, $C_9$-$C_{16}$-Halogenalkoxyphenylalkylcarbonyl, $C_9$-$C_{16}$-Phenylalkenylcarbonyl, $C_9$-$C_{16}$-Halogenphenylalkenylcarbonyl, $C_{10}$-$C_{16}$-Alkylphenylalkenylcarbonyl, $C_{10}$-$C_{16}$-Alkoxyphenylalkenylcarbonyl, $C_{10}$-$C_{16}$-Halogenalkylphenylalkenylcarbonyl, $C_{10}$-$C_{16}$-Halogenalkoxyphenylalkenylcarbonyl, $C_9$-$C_{16}$-Phenylalkinylcarbonyl, $C_9$-$C_{16}$-Halogenphenylalkinylcarbonyl, $C_{10}$-$C_{16}$-Alkylphenylalkinylcarbonyl, $C_{10}$-$C_{16}$-Alkoxyphenylalkinylcarbonyl, $C_{10}$-$C_{16}$-Halogenalkylphenylcarbonyl, $C_{10}$-$C_{16}$-Halogenalkoxyphenylalkinylcarbonyl, $C_2$-$C_8$-Alkylaminocarbonyl, $C_2$-$C_8$-Halogenalkylaminocarbonyl, $C_3$-$C_{10}$-Alkylalkylaminocarbonyl, $C_3$-$C_{10}$-Alkoxyalkylaminocarbonyl, $C_3$-$C_{10}$-Halogenalkylalkylaminocarbonyl, $C_3$-$C_{10}$-Halogenalkoxyalkylaminocarbonyl, $C_3$-$C_{10}$-Dialkylaminocarbonyl, $C_3$-$C_8$-Halogendialkylaminocarbonyl, $C_4$-$C_{12}$-Alkyldialkylaminocarbonyl, $C_4$-$C_{12}$-Alkoxydialkylaminocarbonyl, $C_5$-$C_{20}$-Alkyl- und -alkoxy-aminocarbonyl, $C_4$-$C_{12}$-Halogenalkyldialkylaminocarnonyl, $C_4$-$C_{12}$-Halogenalkoxydialkylaminocarbonyl, Phenylaminocarbonyl, Halogenphenylaminocarbonyl, Dihalogenphenylaminocarbonyl, Trihalogenphenylaminocarbonyl, $C_8$-$C_{14}$-Alkylphenylaminocarbonyl, $C_8$-$C_{14}$-Alkoxyphenylaminocarbonyl, $C_8$-$C_{14}$-Halogenalkoxyphenylaminocarbonyl oder gegebenenfalls durch $C_1$-$C_4$-Alkyl substituierte Isoxazole,
$R^8$ Wasserstoff oder $C_1$-$C_8$-Alkyl und
X Halogen
mit der Maßgabe, daß in Formel Ia $R^1$ nicht Phenyl bedeutet, wenn $R^2$, $R^3$, $R^4$, $R^5$ gleichzeitig für Wasserstoff

stehen, und in Formel Ib $R^2$, $R^3$, $R^4$, $R^5$, nicht gleichzeitig Wasserstoff bedeuten, wenn $R^1$ für $C_4$-$C_8$-Alkyl, Phenyl, Tolyl oder Methoxyphenyl steht, sowie $R^1$ nicht Octyl bedeutet, wenn $R^2$ und $R^3$ für Wasserstoff und $R^4$ oder $R^5$ für Methyl, Methoxy oder Chlor stehen, sowie ferner mit der Maßgabe, daß in Formel Ib $R^1$ nicht n-Butyl bedeutet, wenn $R^2$ für Methyl und $R^3$, $R^4$ und $R^5$ für Wasserstoff stehen.

3. Schädlingsbekämpfungsmittel, enthaltend ein Pyranderivat Ia, Ib oder Ic

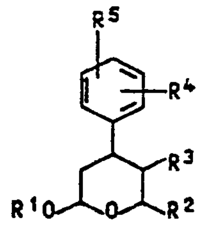
(Ia)

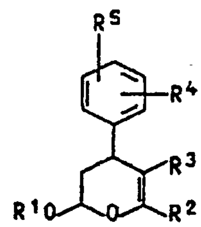
(Ib)

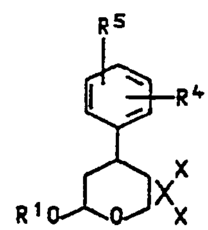
(Ic)

in denen die Substituenten folgende Bedeutung haben :

$R^1$ $C_1$-$C_{20}$-Alkyl, $C_2$-$C_{20}$-Alkenyl, $C_2$-$C_{20}$-Alkinyl, $C_3$-$C_{20}$Cycloalkyl, Aryl, Biphenylyl, $C_7$-$C_{30}$-Arylalkyl, $C_8$-$C_{30}$-Arylalkenyl, $C_8$-$C_{30}$-Arylalkinyl, $C_2$-$C_{20}$-Alkoxy-alkyl, $C_3$-$C_{20}$-Alkoxy-alkoxy-alkyl, $C_4$-$C_{30}$-Alkoxy-alkoxy-alkoxy-alkyl, durch $C_7$-$C_{20}$-Alkyl-aryl, $C_8$-$C_{20}$-Dialkyl-aryl, $C_9$-$C_{20}$-Trialkyl-aryl, Aryloxy, Aryloxy-aryl, $C_3$-$C_{20}$-Cycloalkyl, $C_4$-$C_{20}$-Alkyl-cycloalkyl, $C_4$-$C_{20}$-Cycloalkenyl oder $C_5$-$C_{20}$-Alkyl-cycloalkenyl substituiertes $C_1$-$C_{20}$-Alkyl, $C_4$-$C_{20}$-Alkyl-cycloalkyl, $C_9$-$C_{20}$-Aryl-cycloalkyl, $C_9$-$C_{30}$-Aryloxy-cycloalkyl, $C_7$-$C_{20}$-Alkyl-aryl, $C_7$-$C_{20}$-Alkoxy-aryl, $C_8$-$C_{20}$-Dialkyl-aryl, $C_7$-$C_{20}$-Halogenalkyl-aryl, Halogenaryl, Dihalogenaryl, $C_7$-$C_{20}$-Alkyl-halogenaryl, $C_8$-$C_{20}$-Dialkyl-halogenaryl, Aryloxy-aryl, $C_{13}$-$C_{30}$-Aryloxy-arylalkyl, oder durch einen gesättigten oder ungesättigten 5-, 6-, 7- oder 8-gliedrigen Heterocyclus, mit einem, zwei oder drei gleichen oder verschiedenen Heteroatomen aus der Gruppe Bestehend aus Sauerstoff, Schwefel und Stickstoff, der gegebenenfalls durch ein, zwei oder drei gleiche oder unterschiedliche Substituenten aus der Gruppe bestehend aus $C_1$-$C_{20}$-Alkyl, Aryl, $C_7$-$C_{20}$-Arylalkyl, Halogenaryl, $C_7$-$C_{20}$-Alkylaryl, $C_8$-$C_{20}$-Dialkylaryl, $C_9$-$C_{20}$-Trialkyl-aryl oder ein gegebenenfalls durch $C_1$-$C_8$-Alkyl substituiertes $C_3$-$C_{20}$-Spirocycloalkyl substituiert ist, subtituiertes $C_1$-$C_{20}$-Alkyl,

$R^2$ Wasserstoff, $C_1$-$C_{20}$-Alkyl, $C_1$-$C_{20}$-Halogenalkyl, gegebenenfalls durch Phenyl oder Halogenphenyl substituiert ; $C_7$-$C_{20}$-Halogenphenyl-halogenalkyl, $C_1$-$C_{20}$-Alkoxy, $C_1$-$C_{20}$-Halogenalkoxy, $C_2$-$C_{20}$-Alkenyloxy, $C_2$-$C_{20}$-Halogenalkenyloxy, $C_3$-$C_{20}$-Alkoxy-alkenyloxy, $C_2$-$C_{20}$-Alkinyloxy, $C_2$-$C_{20}$-Halogenalkinyloxy, $C_3$-$C_{20}$-Alkoxy-alkinyloxy, $C_8$-$C_{20}$-Phenyl-alkinyloxy, $C_1$-$C_{20}$-Alkylthio, $C_1$-$C_{20}$-Halogenalkylthio, $C_2$-$C_{20}$-Alkoxy-alkylthio, Formyl, Cyano, $C_7$-$C_{20}$-Phenoxy-alkoxy, $C_7$-$C_{20}$-Halogenphenoxy-alkoxy, $C_7$-$C_{20}$-Phenoxy-halogenalkoxy, $C_7$-$C_{20}$Halogenphenoxy-halogenalkoxy, $C_8$-$C_{20}$-Alkoxy-phenoxyalkoxy, $C_8$-$C_{20}$-Phenoxy-alkoxyalkoxy, $C_7$-$C_{20}$-Phenoxy-alkenyloxy, $C_8$-$C_{20}$-Halogenphenoxy-alkenyl, $C_8$-$C_{20}$-Phenoxy-Halogenalkenyloxy, $C_8$-$C_{20}$-Halogenphenoxy-halogenalkenyloxy, $C_9$-$C_{20}$-Alkoxyphenoxy-alkenyloxy, $C_9$-$C_{20}$-Phenoxy-alkoxyalkenyloxy, $C_8$-$C_{20}$-Phenoxy-alkinyloxy, $C_8$-$C_{20}$-Halogenphenoxy-alkinyloxy, $C_8$-$C_{20}$-Phenoxy-halogenalkinyloxy, $C_8$-$C_{20}$-Halogenphenoxy-halogenalkinyloxy, $C_9$-$C_{20}$-Alkoxy-phenoxy-alkinyloxy, $C_9$-$C_{20}$-Phenoxy-alkoxyalkinyloxy, $C_8$-$C_{20}$-Phenylalkinyloxy, $C_{14}$-$C_{30}$-Phenoxyphenyl-alkinyloxy, Carboxy, $C_2$-$C_{20}$-Alkoxycarbonyl, $C_2$-$C_{20}$-Alkylcarbonyloxy, $C_3$-$C_{20}$-Alkenyloxycarbonyl, $C_3$-$C_{20}$-Alkenylcarbonyloxy, $C_3$-$C_{20}$-Alkinyloxycarbonyl, $C_3$-$C_{20}$-Alkinylcarbonyloxy, Phenoxycarbonyl, Phenylcarbonyloxy, $C_3$-$C_{20}$-Alkoxycarbonyl-alkoxy, Phenoxy, $C_7$-$C_{20}$-Alkylphenoxy, Halogenphenoxy, Dihalogenphenoxy, Trihalogenphenoxy, $C_7$-$C_{20}$-Halogenalkyl-phenoxy, $C_7$-$C_{20}$-Alkoxyphenoxy, Phenoxy-phenoxy, $C_8$-$C_{12}$-Alkylendioxy-phenoxy, Phenylthio, $C_7$-$C_{20}$-Alkylphenylthio, Halogen-phenylthio, Dihalogen-phenylthio, Trihalogen-phenylthio, $C_7$-$C_{20}$-Halogenalkyl-phenylthio, $C_7$-$C_{20}$-Alkoxyphenylthio, Phenoxy-phenylthio, $C_8$-$C_{12}$-Alkylendioxy-phenylthio, Phenyl, Phenoxy-phenyl, $C_7$-$C_{20}$-Phenylalkyl, $C_{13}$-$C_{30}$-Phenoxy-phenylalkyl, $C_8$-$C_{12}$-Alkylendioxy-phenylalkoxy oder einen Rest ausgewählt aus

—CH=N—OH ; 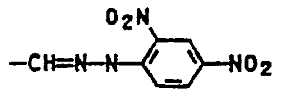 ; 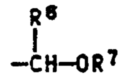 ; 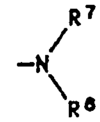 ;

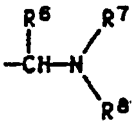 ; 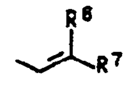 ; 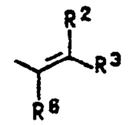 ; 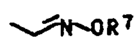 oder 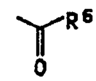 ,

$R^3$ Wasserstoff, $C_1$-$C_{20}$-Alkyl oder $C_1$-$C_3$-Halogenalkyl,

$R^4$, $R^5$ Wasserstoff, Fluor, Chlor, $C_1$-$C_3$-Alkyl, $C_1$-$C_3$-Alkoxy oder $C_1$-$C_3$-Halogenalkoxy,

$R^6$ Wasserstoff, $C_1$-$C_{20}$-Alkyl, $C_2$-$C_{20}$-Alkenyl, $C_2$-$C_{20}$-Alkinyl, Cyano, Phenyl, Halogenphenyl, $C_7$-$C_{20}$-Alkylphenyl oder $C_7$-$C_{20}$-Alkoxyphenyl,

$R^7$ Wasserstoff, $C_1$-$C_{20}$-Alkyl, $C_1$-$C_{20}$-Halogenalkyl, $C_2$-$C_{20}$-Alkoxy-alkyl, $C_2$-$C_{20}$-Alkenyl, $C_2$-$C_{20}$-Alkinyl, Aryl, Halogenphenyl, Dihalogenphenyl, Trihalogenphenyl, Cyanophenyl, $C_7$-$C_{20}$-Alkylphenyl, $C_7$-$C_{20}$-Alkoxyphenyl, $C_7$-$C_{20}$-Halogenalkyl-phenyl, $C_8$-$C_{20}$-Alkenylphenyl, $C_8$-$C_{20}$-Alkinylphenyl, Phenoxyphenyl, $C_8$-$C_{12}$-Methylendioxy-phenyl, $C_2$-$C_{20}$-Alkylcarbonyl, $C_2$-$C_{20}$-Halogenalkylcarbonyl, $C_2$-$C_{20}$-Alkoxycarbonyl, $C_5$-$C_{20}$-Alkylcyclopropyl-carbonyl, $C_7$-$C_{20}$-Alkenyl- und -alkylcyclopropyl-carbonyl, $C_6$-$C_{20}$-Halogenalkyl- und -alkylcyclopropyl-carbonyl, $C_7$-$C_{20}$-Halogenalkenyl- und -alkylcyclopropyl-carbonyl, $C_8$-$C_{20}$-Halogen- und -phenyl-alkylcarbonyl, $C_8$-$C_{20}$-Halogen- und -halogenphenylalkylcarbonyl, Benzoyl, Halogenbenzoyl, $C_8$-$C_{20}$-Alkylbenzoyl, $C_8$-$C_{20}$-Alkoxybenzoyl, $C_8$-$C_{20}$-Halogenalkyl-benzoyl, $C_8$-$C_{20}$-Halogenalkoxy-benzoyl, $C_8$-$C_{20}$-Phenylalkylcarbonyl, $C_8$-$C_{20}$-Halogenphenylalkylcarbonyl, $C_9$-$C_{20}$-Alkylphenylalkylcarbonyl, $C_9$-$C_{20}$-Alkoxyphenylalkylcarbonyl, $C_9$-$C_{20}$-Halogenalkylphenylalkylcarbonyl, $C_9$-$C_{20}$-Halogenalkoxyphenylalkylcarbonyl, $C_9$-$C_{20}$-Phenylalkenylcarbonyl, $C_9$-$C_{20}$-Halogenphenylalkenylcarbonyl, $C_{10}$-$C_{20}$-Alkylphenylalkenylcarbonyl, $C_{10}$-$C_{20}$-Alkoxyphenylalkenylcarbonyl, $C_{10}$-$C_{20}$-Halogenalkylphenylalkenylcarbonyl, $C_{10}$-$C_{20}$-Halogenalkoxyphenylalkenylcarbonyl, $C_9$-$C_{20}$-Phenylalkinylcarbonyl, $C_9$-$C_{20}$-Halogenphenylalkinylcarbonyl, $C_{10}$-$C_{20}$-Alkylphenylalkinylcarbonyl, $C_{10}$-$C_{20}$-Alkoxyphenylalkinylcarbonyl, $C_{10}$-$C_{20}$-Halogenalkylphenylalkinylcarbonyl, $C_{10}$-$C_{20}$-Halogenalkoxyphenylalkinylcarbonyl, $C_2$-$C_{20}$-Alkylaminocarbonyl, $C_2$-$C_{20}$-Halogenalkylaminocarbonyl, $C_3$-$C_{20}$-Alkoxyalkylaminocarbonyl, $C_3$-$C_{20}$-Halogenalkylalkylaminocarbonyl, $C_3$-$C_{20}$-Halogenalkoxyalkylaminocarbonyl, $C_3$-$C_{20}$-Dialkylaminocarbonyl, $C_3$-$C_{20}$-Halogendialkylaminocarbonyl, $C_4$-$C_{20}$-Alkoxydialkylaminocarbonyl, $C_5$-$C_{20}$-Alkyl- und -alkoxy-aminocarbonyl, $C_4$-$C_{20}$-Halogenalkyldialkylaminocarbonyl, $C_4$-$C_{20}$-Halogenalkoxydialkylaminocarbonyl, Phenylaminocarbonyl, Halogenphenylaminocarbonyl, Dihalogenphenylaminocarbonyl, Trihalogenphenylaminocarbonyl, $C_8$-$C_{20}$-Alkylphenylaminocarbonyl, $C_8$-$C_{20}$-Alkoxyphenylaminocarbonyl, $C_8$-$C_{20}$-Halogenalkoxyphenylaminocarbonyl oder gegebenenfalls durch $C_1$-$C_8$-Alkyl substituierte Isoxazole,

$R^8$ Wasserstoff oder $C_1$-$C_{20}$-Alkyl und

X Halogen,

mit der Maßgabe, daß im Fall von Verbindung Ia $R^1$ nicht Phenyl bedeutet, wenn $R^2$ bis $R^5$ gleichzeitig für Wasserstoff stehen, und im Fall von Verbindung Ib $R^2$ bis $R^5$ nicht gleichzeitig Wasserstoff bedeuten, wenn $R^1$ für $C_4$-$C_8$-Alkyl, Phenyl, Tolyl oder Methoxyphenyl, sowie $R^1$ nicht Octyl bedeutet, wenn $R^2$ und $R^3$ für Wasserstoff und $R^4$ oder $R^5$ für Methyl, Methoxy oder Chlor stehen, sowie ferner mit der Maßgabe, daß in Formel Ib $R^1$ nicht n-Butyl bedeutet, wenn $R^2$ für Methyl und $R^3$, $R^4$ und $R^5$ für Wasserstoff stehen.

4. Schädlingsbekämpfungsmittel nach Anspruch 3, enthaltend ein Pyranderivat Ia, Ib oder Ic gemäß Anspruch 3, wobei die Substituenten die folgende Beudeutung haben :

$R^1$ $C_1$-$C_{12}$-Alkyl, $C_2$-$C_{14}$-Alkenyl, $C_2$-$C_8$-Alkinyl, $C_3$-$C_8$-Cycloalkyl, Phenyl, Naphthyl, Biphenylyl, $C_7$-$C_{14}$-Phenylalkyl, $C_8$-$C_{14}$-Phenylalkenyl, $C_8$-$C_{14}$-Phenylalkinyl, $C_2$-$C_8$-Alkoxy-alkyl, $C_3$-$C_{12}$-Alkoxy-alkoxy-alkyl, $C_4$-$C_{16}$-Alkoxy-alkoxy-alkoxy-alkyl, durch $C_7$-$C_{10}$-Alkyl-aryl, $C_8$-$C_{14}$-Dialkyl-phenyl, $C_9$-$C_{14}$-Trialkyl-aryl, Phenoxy, Phenoxy-phenyl, $C_3$-$C_8$-Cycloalkyl, $C_4$-$C_{12}$-Alkyl-cycloalkyl, $C_4$-$C_8$-Cycloalkenyl oder $C_5$-$C_{12}$-Alkyl-cycloalkenyl substituiertes $C_1$-$C_8$-Alkyl, $C_4$-$C_{12}$-Alkyl-cycloalkyl, $C_9$-$C_{14}$-Phenyl-cycloalkyl, $C_9$-$C_{14}$-Phenoxy-cycloalkyl, $C_7$-$C_{14}$-Alkyl-phenyl, $C_7$-$C_{14}$-Alkoxy-phenyl, $C_8$-$C_{16}$-Dialkyl-phenyl, $C_7$-$C_{14}$-Halogenalkyl-phenyl, Halogenphenyl, Dihalogenphenyl, $C_7$-$C_{14}$-Alkyl-halogenphenyl, $C_8$-$C_{14}$-Dialkyl-halogenphenyl, Phenoxy-phenyl, $C_{13}$-$C_{20}$-Phenoxy-phenylalkyl, oder durch einen gesättigten oder ungesättigten 5- oder 6-gliedrigen Heterocyclus, mit einem ein oder zwei gleichen oder verschiedenen Heteroatomen aus der Gruppe bestehend aus Sauerstoff und Stickstoff, der gegebenenfalls durch ein, zwei oder drei gleiche oder unterschiedliche Substituenten aus der Gruppe bestehend aus $C_1$-$C_8$-Alkyl, Phenyl, $C_7$-$C_{14}$-Phenylalkyl, Halogenphenyl, $C_7$-$C_{14}$-Alkylphenyl, $C_8$-$C_{16}$-Dialkyl-phenyl, $C_9$-$C_{16}$-Trialkyl-aryl oder durch ein gegebenenfalls durch $C_1$-$C_4$-Alkyl substituiertes $C_3$-$C_8$-Spirocycloalkyl substituiert ist, substituiertes $C_1$-

$C_8$-Alkyl,

$R^2$ Wasserstoff, $C_1$-$C_8$-Alkyl, $C_1$-$C_8$-Halogenalkyl, gegebenenfalls durch Phenyl oder Halogenphenyl substituiert ; $C_1$-$C_8$-Alkoxy, $C_1$-$C_8$-Halogenalkoxy, $C_2$-$C_8$-Alkenyloxy, $C_2$-$C_8$-Halogenalkenyloxy, $C_3$-$C_{12}$-Alkoxy-alkenyloxy, $C_2$-$C_8$-Alkinyloxy, $C_2$-$C_8$-Halogenalkinyloxy, $C_3$-$C_{12}$-Alkoxy-alkinyloxy, $C_8$-$C_{16}$-Phenyl-alkinyloxy, $C_1$-$C_8$-Alkylthio, $C_1$-$C_8$-Halogenalkylthio, $C_2$-$C_{12}$-Alkoxy-alkylthio, Formyl, Cyano, $C_7$-$C_{14}$-Phenoxy-alkoxy, $C_7$-$C_{14}$-Halogenphenoxy-alkoxy, $C_7$-$C_{14}$-Phenoxy-halogenalkoxy, $C_7$-$C_{14}$-Halogenphenoxy-halogenalkoxy, $C_8$-$C_{16}$-Alkoxyphenoxyalkoxy, $C_8$-$C_{16}$-Phenoxy-alkoxyalkoxy, $C_7$-$C_{16}$-Phenoxy-alkenyloxy, $C_8$-$C_{16}$-Halogenphenoxy-alkenyl, $C_8$-$C_{16}$-Phenoxy-halogenalkenyloxy, $C_8$-$C_{16}$-Halogenphenoxy-halogenalkenyloxy, $C_9$-$C_{16}$-Alkoxyphenoxy-alkenyloxy, $C_9$-$C_{18}$-Phenoxy-alkoxy-alkenyloxy, $C_8$-$C_{14}$-Phenoxy-alkinyloxy,$C_8$-$C_{14}$-Halogenphenoxy-alkinyloxy, $C_8$-$C_{14}$-Phenoxy-halogenalkinyloxy, $C_8$-$C_{14}$-Halogenphenoxy-halogenalkinyloxy, $C_9$-$C_{16}$-Alkoxyphenoxy-alkinyloxy, $C_9$-$C_{16}$-Phenoxy-alkoxyalkinyloxy, $C_8$-$C_{14}$-Phenylalkinyloxy, $C_{14}$-$C_{20}$-Phenoxyphenyl-alkinyloxy, Carboxy, $C_2$-$C_8$-Alkoxycarbonyl, $C_2$-$C_8$-Alkylcarbonyloxy, $C_3$-$C_{12}$-Alkenyloxycarbonyl, $C_3$-$C_{12}$-Alkenylcarbonyloxy, $C_3$-$C_{12}$-Alkinyloxycarbonyl, $C_3$-$C_{12}$-Alkinylcarbonyloxy, Phenoxycarbonyl, Phenylcarbonyloxy, $C_3$-$C_{12}$-Alkoxycarbonyl-alkoxy, Phenoxy, $C_7$-$C_{14}$-Alkylphenoxy, Halogenphenoxy, Dihalogenphenoxy, Trihalogenphenoxy, $C_7$-$C_{14}$-Halogenalkyl-phenoxy, $C_7$-$C_{14}$-Alkoxyphenoxy, Phenoxy-phenoxy, $C_8$-$C_{14}$-Alkylendioxy-phenoxy, Phenylthio, $C_7$-$C_{14}$-Alkylphenylthio, Halogen-phenylthio, Dihalogen-phenylthio, Trihalogen-phenylthio, $C_7$-$C_{14}$-Halogenalkyl-phenylthio, $C_7$-$C_{14}$-Alkoxyphenylthio, Phenoxy-phenylthio, $C_8$-$C_{14}$-Alkylendioxy-phenylthio, Phenyl, Phenoxy-phenyl, $C_7$-$C_{14}$-Phenylalkyl, $C_{13}$-$C_{20}$-Phenoxy-phenylalkyl, $C_8$-$C_{14}$-Alkylendioxy-phenylalkyloxy oder einen Rest ausgewählt aus

$-CH=N-OH$ ; 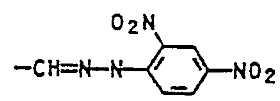 ; 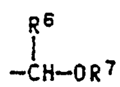 ; 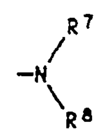 ;

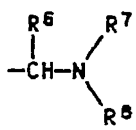 ; 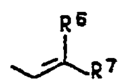 ; 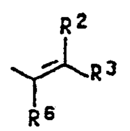 ; 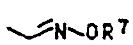 oder 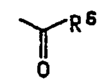 ,

$R^3$ Wasserstoff, $C_1$-$C_8$-Alkyl oder $C_1$-$C_2$-Halogenalkyl,

$R^4$, $R^5$ Wasserstoff, Fluor, Chlor, $C_1$-$C_3$-Alkyl, $C_1$-$C_3$-Alkoxy oder $C_1$-$C_3$-Halogenalkoxy,

$R^6$ Wasserstoff, $C_1$-$C_8$-Alkyl, $C_2$-$C_8$-Alkenyl, $C_2$-$C_8$-Alkinyl, Cyano, Phenyl, Halogenphenyl, $C_7$-$C_{14}$-Alkylphenyl oder $C_7$-$C_{14}$-Alkoxyphenyl,

$R^7$ Wasserstoff, $C_1$-$C_8$-Alkyl, $C_1$-$C_8$-Halogenalkyl, $C_2$-$C_8$-Alkoxy-alkyl, $C_2$-$C_8$-ALkenyl, $C_2$-$C_8$-Alkinyl, Aryl, Halogenphenyl, Dihalogenphenyl, Trihalogenphenyl, Cyanophenyl, $C_7$-$C_{14}$-Alkylphenyl, $C_7$-$C_{14}$-Alkoxyphenyl, $C_7$-$C_{14}$-Halogenalkyl-phenyl, $C_8$-$C_{16}$-Alkenylphenyl, $C_8$-$C_{16}$-ALkinylphenyl, Phenoxyphenyl, $C_8$-$C_{16}$-Methylendioxy-phenyl, $C_2$-$C_8$-Alkylcarbonyl, $C_2$-$C_8$-Halogenalkylcarbonyl, $C_2$-$C_8$-Alkoxycarbonyl, $C_5$-$C_{12}$-ALkylcyclopropyl-carbonyl, $C_7$-$C_{14}$-Alkenyl- und -alkylcycloporpyl-carbonyl, $C_6$-$C_{14}$-Halogenalkyl- und -alkylcyclopropyl-carbonyl, $C_7$-$C_{14}$-Halogenalkenyl- und -alkylcyclopropyl-carbonyl, $C_8$-$C_{16}$-Halogen- und -phenyl-alkylcarbonyl, $C_8$-$C_{16}$-Halogen- und -halogenphenylalkylcarbonyl, Benzoyl, Halogenbenzoyl, $C_8$-$C_{14}$-Alkylbenzoyl, $C_8$-$C_{14}$-Alkoxybenzoyl, $C_8$-$C_{14}$-Halogenalkyl-benzoyl, $C_8$-$C_{14}$-Halogenalkoxy-benzoyl, $C_8$-$C_{14}$-Phenylalkylcarbonyl, $C_8$-$C_{14}$-Halogenphenylalkylcarbonyl, $C_9$-$C_{16}$-ALkylphenylalkylcarbonyl, $C_9$-$C_{16}$-ALkoxyphenylalkylcarbonyl, $C_9$-$C_{16}$-Halogenalkylphenylalkylcarbonyl, $C_9$-$C_{16}$-Halogenalkoxyphenylalkylcarbonyl, $C_9$-$C_{18}$-Phenylalkenylcarbonyl, $C_9$-$C_{16}$-Halogenphenylalkenylcarbonyl, $C_{10}$-$C_{16}$-Alkylphenylalkenylcarbonyl, $C_{10}$-$C_{16}$-Alkoxyphenylalkenylcarbonyl, $C_{10}$-$C_{16}$-Halogenalkylphenylalkenylcarbonyl, $C_{10}$-$C_{18}$-Halogenalkoxyphenylalkenylcarbonyl, $C_9$-$C_{16}$-Phenylalkinylcarbonyl, $C_9$-$C_{16}$-Halogenphenylalkinylcarbonyl, $C_{10}$-$C_{16}$-Alkylohenylalkinylcarbonyl, $C_{10}$-$C_{16}$-Alkoxyphenylalkinylcarbonyl, $C_{10}$-$C_{16}$-Halogenalkylphenylalkinylcarbonyl, $C_{10}$-$C_{16}$-Halogenalkoxyphenylalkinylcarbonyl, $C_2$-$C_8$-Alkylaminocarbonyl, $C_2$-$C_8$-Halogenalkylaminocarbonyl, $C_3$-$C_{10}$-Alkylalkylaminocarbonyl, $C_3$-$C_{10}$-Alkoxyalkylaminocarbonyl, $C_3$-$C_{10}$-Halogenalkylalkylaminocarbonyl, $C_3$-$C_{10}$-Halogenalkoxyalkylaminocarbonyl, $C_3$-$C_{10}$-Dialkylaminocarbonyl, $C_3$-$C_8$-Halogendialkylaminocarbonyl, $C_4$-$C_{12}$-Alkyldialkylaminocarbonyl, $C_4$-$C_{12}$-Alkoxydialkylaminocarbonyl, $C_5$-$C_{20}$-Alkyl- und -alkoxy-aminocarbonyl, $C_4$-$C_{12}$-Halogenalkyldialkylaminocarbonyl, $C_4$-$C_{12}$-Halogenalkoxydialkylaminocarbonyl, Phenylaminocarbonyl, Halogenphenylaminocarbonyl, Dihalogenphenylaminocarbonyl, Trihalogenphenylaminocarbonyl, $C_8$-$C_{14}$-Alkylphenylaminocarbonyl, $C_8$-$C_{14}$-Alkoxyphenylaminocarbonyl, $C_8$-$C_{14}$-Halogenalkoxyphenylaminocarbonyl oder gegebenenfalls durch $C_1$-$C_4$-Alkyl substituierte Isoxazole,

$R^8$ Wasserstoff oder $C_1$-$C_8$-Alkyl und
X Halogen,
mit der Maßgabe, daß in Formel Ia $R^1$ nicht Phenyl bedeutet, wenn $R^2$, $R^3$, $R^4$, $R^5$ gleichzeitig für Wasserstoff stehen, und in Formel Ib $R^2$, $R^3$, $R^4$, $R^5$ nicht gleichzeitig Wasserstoff bedeutet, wenn $R^1$ für $C_4$-$C_8$-Alkyl, Phenyl, Tolyl oder Methoxyphenyl steht, sowie $R^1$ nicht Octyl bedeutet, wenn $R^2$ und $R^3$ für Wasserstoff und $R^4$ oder $R^5$ für Methyl, Methoxy oder Chlor stehen, sowie ferner mit der Maßgabe, daß in Formel Ib $R^1$ nicht n-Butyl bedeutet, wenn $R^2$ für Methyl und $R^3$, $R^4$ und $R^5$ für Wasserstoff stehen,
neben hierfür üblichen Trägerstoffen.

5. Schädlingsbekämpfungsmittel gemäß Anspruch 3, dadurch gekennzeichnet, daß es 0,1 bis 95 Gew.% eines Pyranderivates Ia, Ib oder Ic enthält.

6. Verfahren zur Bekämpfung von Schädlingen, dadurch gekennzeichnet, daß man die Schädlinge und/oder die von Schädlingen freizuhaltenden Flächen und/oder Räume mit einer für Schädlinge wirksamen Menge eines Mittels gemän einem der Ansprüche 3 oder 4 behandelt.

## Claims

### Claims for the Contracting States AT, BE, CH, DE, FR, GB, GR, IT, LI, NL, SE

1. A pyran derivative of the general formula Ia, Ib or Ic

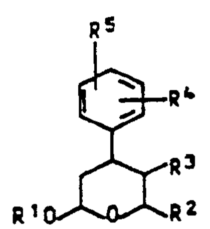
(Ia)

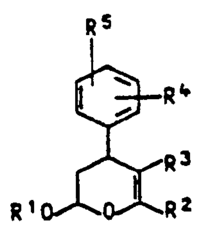
(Ib)

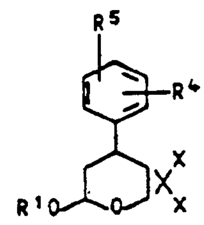
(Ic)

where
$R^1$ is $C_1$-$C_{20}$-alkyl, $C_2$-$C_{20}$-alkenyl, $C_2$-$C_{20}$-alkynyl, $C_3$-$C_{20}$-cycloalkyl, aryl, biphenylyl, $C_7$-$C_{30}$-aralkyl, $C_8$-$C_{30}$-arylalkenyl, $C_8$-$C_{30}$-arylalkynyl, $C_2$-$C_{20}$-alkoxyalkyl, $C_3$-$C_{20}$-alkoxyalkoxyalkyl, $C_4$-$C_{30}$-alkoxyalkoxyalkoxyalkyl, $C_1$-$C_{20}$-alkyl which is substituted by $C_7$-$C_{20}$-alkylaryl, $C_8$-$C_{20}$-dialkylaryl, $C_9$-$C_{20}$-trialkylaryl, aryloxy, aryloxyaryl, $C_3$-$C_{20}$-cycloalkyl, $C_4$-$C_{20}$-alkyl-cycloalkyl, $C_4$-$C_{20}$-cycloalkenyl or $C_5$-$C_{20}$-alkylcycloalkenyl, $C_4$-$C_{20}$-alkylcycloalkyl, $C_9$-$C_{20}$-arylcycloalkyl, $C_9$-$C_{30}$-aryloxycycloalkyl, $C_7$-$C_{20}$-alkylaryl, $C_7$-$C_{20}$-alkoxyaryl, $C_8$-$C_{20}$-dialkylaryl, $C_7$-$C_{20}$-haloalkylaryl, haloaryl, dihaloaryl, $C_7$-$C_{20}$-alkylhaloaryl, $C_8$-$C_{20}$-dialkylhaloaryl, aryloxyaryl or $C_{13}$-$C_{30}$-aryloxyaryl-alkyl, or is $C_1$-$C_{20}$-alkyl which is substituted by a saturated or unsaturated 5-membered, 6-membered, 7-membered or 8-membered heterocyclic structure which has one, two or three identical or different hetero atoms from the group consisting of oxygen, sulfur and nitrogen and is unsubstituted or substituted by one, two or three identical or different substituents from the group consisting of $C_1$-$C_{20}$-alkyl, aryl, $C_7$-$C_{20}$-aralkyl, haloaryl, $C_7$-$C_{20}$-alkylaryl, $C_8$-$C_{20}$-dialkylaryl, $C_9$-$C_{20}$-trialkylaryl or unsubstituted or $C_1$-$C_8$-alkyl-substituted $C_3$-$C_{20}$-spirocycloalkyl,
$R^2$ is hydrogen, $C_1$-$C_{20}$-alkyl, unsubstituted, phenyl-substituted or halophenyl-substituted $C_1$-$C_{20}$-haloalkyl; $C_7$-$C_{20}$-halophenylhaloalkyl, $C_1$-$C_{20}$-alkoxy, $C_1$-$C_{20}$-haloalkoxy, $C_2$-$C_{20}$-alkenyloxy, $C_2$-$C_{20}$-haloalkenyloxy, $C_3$-$C_{20}$-alkoxyalkenyloxy, $C_2$-$C_{20}$-alkynyloxy, $C_2$-$C_{20}$-haloalkynyloxy, $C_3$-$C_{20}$-alkoxyalkynyloxy, $C_8$-$C_{20}$-phenylalkynyloxy, $C_1$-$C_{20}$-alkylthio, $C_1$-$C_{20}$-haloalkylthio, $C_2$-$C_{20}$-alkoxyalkylthio, formyl, cyano, $C_7$-$C_{20}$-phenoxyalkoxy, $C_7$-$C_{20}$-halophenoxyalkoxy, $C_7$-$C_{20}$-phenoxyhaloalkoxy, $C_7$-$C_{20}$-halophenoxyhaloalkoxy, $C_8$-$C_{20}$-alkoxyphenoxyalkoxy, $C_8$-$C_{20}$-phenoxyalkoxyalkoxy, $C_7$-$C_{20}$-phenoxyalkenyloxy, $C_8$-$C_{20}$-halophenoxyalkenyl, $C_8$-$C_{20}$-phenoxyhaloalkenyloxy, $C_8$-$C_{20}$-halophenoxyhaloalkenyloxy, $C_9$-$C_{20}$-alkoxyphenoxyalkenyloxy, $C_9$-$C_{20}$-phenoxyalkoxyalkenyloxy, $C_8$-$C_{20}$-phenoxyalkynyloxy, $C_8$-$C_{20}$-halophenoxyalkynyloxy, $C_8$-$C_{20}$-phenoxyhaloalkynyloxy, $C_8$-$C_{20}$-halophenoxyhaloalkynyloxy, $C_9$-$C_{20}$-alkoxyphenoxyalkynyloxy, $C_9$-$C_{20}$-phenoxyalkoxyalkynyloxy, $C_8$-$C_{20}$-phenylalkynyloxy, $C_{14}$-$C_{30}$-phenoxyphenylalkynyloxy, carboxyl, $C_2$-$C_{20}$-alkoxycarbonyl, $C_2$-$C_{20}$-alkylcarbonyloxy, $C_3$-$C_{20}$-alkenyloxycarbonyl, $C_3$-$C_{20}$-alkenylcarbonyloxy, $C_3$-$C_{20}$-alkynyloxycarbonyl, $C_3$-$C_{20}$-alkynylcarbonyloxy, phenoxycarbonyl, phenylcarbonyloxy, $C_3$-$C_{20}$-alkoxycarbonylalkoxy, phenoxy, $C_7$-$C_{20}$-alkylphenoxy, halophenoxy, dihalophenoxy, trihalophenoxy, $C_7$-$C_{20}$-haloalkylphenoxy, $C_7$-$C_{20}$-alkoxyphenoxy,

phenoxyphenoxy, $C_8$-$C_{12}$-alkylenedioxyphenoxy, phenylthio, $C_7$-$C_{20}$-alkylphenylthio, halophenylthio, dihalophenylthio, trihalophenylthio, $C_7$-$C_{20}$-haloalkylphenylthio, $C_7$-$C_{20}$-alkoxyphenylthio, phenoxyphenylthio, $C_8$-$C_{12}$-alkylenedioxyphenylthio, phenyl, phenoxyphenyl, $C_7$-$C_{20}$-phenylalkyl, $C_{13}$-$C_{30}$-phenoxyphenylalkyl, $C_8$-$C_{12}$-alkylenedioxyphenylalkoxy or a radical selected from

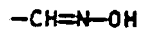 ; 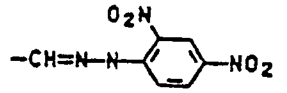 ; 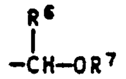 ; 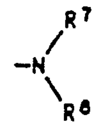 ;

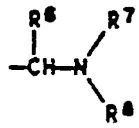 ; 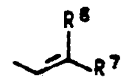 ; 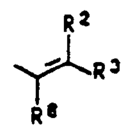 ; 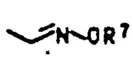 and 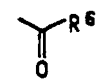 .

$R^3$ is hydrogen, $C_1$-$C_{20}$-alkyl or $C_1$-$C_3$-haloalkyl,

$R^4$ and $R^5$ are each hydrogen, fluorine, chlorine, $C_1$-$C_3$-alkyl, $C_1$-$C_3$-alkoxy or $C_1$-$C_3$-haloalkoxy,

$R^6$ is hydrogen, $C_1$-$C_{20}$-alkyl, $C_2$-$C_{20}$-alkenyl, $C_2$-$C_{20}$-alkynyl, cyano, phenyl, halophenyl, $C_7$-$C_{20}$-alkylphenyl or $C_7$-$C_{20}$-alkoxyphenyl,

$R^7$ is hydrogen, $C_1$-$C_{20}$-alkyl, $C_1$-$C_{20}$-haloalkyl, $C_2$-$C_{20}$-alkoxyalkyl, $C_2$-$C_{20}$-alkenyl, $C_2$-$C_{20}$-alkynyl, aryl, halophenyl, dihalophenyl, trihalophenyl, cyanophenyl, $C_7$-$C_{20}$-alkylphenyl, $C_7$-$C_{20}$-alkoxyphenyl, $C_7$-$C_{20}$-haloalkylphenyl, $C_8$-$C_{20}$-alkenylphenyl, $C_8$-$C_{20}$-alkynyphenyl, phenoxyphenyl, $C_8$-$C_{12}$-methylenedioxyphenyl, $C_2$-$C_{20}$-alkylcarbonyl, $C_2$-$C_{20}$-haloalkylcarbonyl, $C_2$-$C_{20}$-alkoxycarbonyl, $C_5$-$C_{20}$-alkylcyclopropylcarbonyl, $C_7$-$C_{20}$-alkenyl- and -alkylcyclopropylcarbonyl, $C_6$-$C_{20}$-haloalkyl- and -alkylcyclopropylcarbonyl, $C_7$-$C_{20}$-haloalkenyl- and -alkylcyclopropylcarbonyl, $C_8$-$C_{20}$-halo- and -phenylalkylcarbonyl, $C_8$-$C_{20}$-halo- and -halophenylalkylcarbonyl, benzoyl, halobenzoyl, $C_8$-$C_{20}$-alkylbenzoyl, $C_8$-$C_{20}$-alkoxybenzoyl, $C_8$-$C_{20}$-haloalkylbenzoyl, $C_8$-$C_{20}$-haloalkoxybenzoyl, $C_8$-$C_{20}$-phenylalkylcarbonyl, $C_8$-$C_{20}$-halophenylalkylcarbonyl, $C_9$-$C_{20}$-alkylphenylalkylcarbonyl, $C_9$-$C_{20}$-alkoxyphenylalkylcarbonyl, $C_9$-$C_{20}$-haloalkylphenylalkylcarbonyl, $C_9$-$C_{20}$-haloalkoxyphenylalkylcarbonyl, $C_9$-$C_{20}$-phenylalkenylcarbonyl, $C_9$-$C_{20}$-halophenylalkenylcarbonyl, $C_{10}$-$C_{20}$-alkylphenylalkenylcarbonyl, $C_{10}$-$C_{20}$-alkoxyphenylalkenylcarbonyl, $C_{10}$-$C_{20}$-haloalkylphenylalkenylcarbonyl, $C_{10}$-$C_{20}$-haloalkoxyphenylalkenylcarbonyl, $C_9$-$C_{20}$-phenylalkynylcarbonyl, $C_9$-$C_{20}$-halophenylalkynylcarbonyl, $C_{10}$-$C_{20}$-alkylphenylalkynylcarbonyl, $C_{10}$-$C_{20}$-alkoxyphenylalkynylcarbonyl, $C_{10}$-$C_{20}$-haloalkylphenylalkynylcarbonyl, $C_{10}$-$C_{20}$-haloalkoxyphenylalkynylcarbonyl, $C_2$-$C_{20}$-alkylaminocarbonyl, $C_2$-$C_{20}$-haloalkylaminocarbonyl, $C_3$-$C_{20}$-alkoxyalkylaminocarbonyl, $C_3$-$C_{20}$-haloalkylalkylaminocarbonyl, $C_3$-$C_{20}$-haloalkoxyalkylaminocarbonyl, $C_3$-$C_{20}$-dialkylaminocarbonyl, $C_3$-$C_{20}$-halodialkylaminocarbonyl, $C_4$-$C_{20}$-alkoxydialkylaminocarbonyl, $C_5$-$C_{20}$-alkyl- and -alkoxyaminocarbonyl, $C_4$-$C_{20}$-haloalkyldialkylaminocarbonyl, $C_4$-$C_{20}$-haloalkoxydialkylaminocarbonyl, phenylaminocarbonyl, halophenylaminocarbonyl, dihalophenylaminocarbonyl, trihalophenylaminocarbonyl, $C_8$-$C_{20}$-alkylphenylaminocarbonyl, $C_8$-$C_{20}$-alkoxyphenylaminocarbonyl, $C_8$-$C_{20}$-haloalkoxyphenylaminocarbonyl or an unsubstituted or $C_1$-$C_8$-alkyl-substituted isoxazole,

$R^8$ is hydrogen or $C_1$-$C_{20}$-alkyl and

X is halogen,

with the proviso that, in formula Ia, $R^1$ is not phenyl when $R^2$, $R^3$, $R^4$ and $R^5$ are simultaneously hydrogen, and, in formula Ib, $R^2$, $R^3$, $R^4$ and $R^5$ are not simultaneously hydrogen when $R^1$ is $C_4$-$C_8$-alkyl, phenyl, tolyl or methoxyphenyl, and $R^1$ is not octyl when $R^2$ and $R^3$ are each hydrogen and $R^4$ or $R^5$ is methyl, methoxy or chlorine, and with the further proviso that, in formula Ib, $R^1$ is not n-butyl when $R^2$ is methyl and $R^3$, $R^4$ and $R^5$ are each hydrogen.

2. A pyran derivative of the general formula Ia, Ib or Ic as claimed in claim 1, where

$R^1$ is $C_1$-$C_{12}$-alkyl, $C_2$-$C_{14}$-alkenyl, $C_2$-$C_8$-alkynyl, $C_3$-$C_8$-cycloalkyl, phenyl, naphthyl, biphenylyl, $C_7$-$C_{14}$-phenylalkyl, $C_8$-$C_{14}$-phenylalkenyl, $C_8$-$C_{14}$-phenylalkynyl, $C_2$-$C_8$-alkoxyalkyl, $C_3$-$C_{12}$-alkoxyalkoxyalkyl, $C_4$-$C_{16}$-alkoxyalkoxyalkoxyalkyl, $C_1$-$C_8$-alkyl which is substituted by $C_7$-$C_{10}$-alkylaryl, $C_8$-$C_{14}$-dialkylphenyl, $C_9$-$C_{14}$-trialkylaryl, phenoxy, phenoxyphenyl, $C_3$-$C_8$-cycloalkyl, $C_4$-$C_{12}$-alkylcycloalkyl, $C_4$-$C_8$-cycloalkenyl or $C_5$-$C_{12}$-alkylcycloalkenyl, $C_4$-$C_{12}$-alkylcycloalkyl, $C_9$-$C_{14}$-phenylcycloalkyl, $C_9$-$C_{14}$-phenoxycycloalkyl, $C_7$-$C_{14}$-alkylphenyl, $C_7$-$C_{14}$-alkoxyphenyl, $C_8$-$C_{16}$-dialkylphenyl, $C_7$-$C_{14}$-haloalkylphenyl, halophenyl, dihalophenyl, $C_7$-$C_{14}$-alkylhalophenyl, $C_8$-$C_{14}$-dialkylhalophenyl, phenoxyphenyl or $C_{13}$-$C_{20}$-phenoxyphenylalkyl, or is $C_1$-$C_8$-alkyl which is substituted by a saturated or unsaturated 5-membered or 6-membered heterocyclic structure which has one or two identical or different hetero atoms from the group consisting of oxygen and nitrogen and is unsubstituted or substituted by one, two or three identical or different substituents from the group consisting of $C_1$-$C_8$-alkyl, phenyl, $C_7$-$C_{14}$-phenylalkyl, halophenyl, $C_7$-$C_{14}$-alkylphenyl, $C_8$-$C_{16}$-dialkylphenyl, $C_9$-$C_{16}$-trialkylaryl or an unsubstituted or $C_1$-$C_4$-alkyl-substituted $C_3$-$C_8$-spirocycloalkyl,

$R^2$ is hydrogen, $C_1$-$C_8$-alkyl, $C_1$-$C_8$-haloalkyl, which is unsubstituted or substituted by phenyl or halophenyl; $C_1$-$C_8$-alkoxy, $C_1$-$C_8$-haloalkoxy, $C_2$-$C_8$-alkenyloxy, $C_2$-$C_8$-haloalkenyloxy, $C_3$-$C_{12}$-alkoxyalkenyloxy, $C_2$-$C_8$-alkynyloxy, $C_2$-$C_8$-haloalkynyloxy, $C_3$-$C_{12}$-alkoxyalkynyloxy, $C_8$-$C_{16}$-phenylalkynyloxy, $C_1$-$C_8$-alkylthio, $C_1$-$C_8$-haloalkylthio, $C_2$-$C_{12}$-alkoxy-alkylthio, formyl, cyano, $C_7$-$C_{14}$-phenoxyalkoxy, $C_7$-C14-halophenoxyalkoxy, $C_7$-$C_{14}$-phenoxyhaloalkoxy, $C_7$-$C_{14}$-halophenoxyhaloalkoxy, $C_8$-$C_{16}$-alkoxyphenoxyalkoxy, $C_8$-$C_{16}$-phenoxyalkoxyalkoxy, $C_7$-$C_{16}$-phenoxyalkenyloxy, $C_8$-$C_{16}$-halophenoxyalkenyl, $C_8$-$C_{16}$-phenoxyhaloalkenyloxy, $C_8$-$C_{16}$-halophenoxyhaloalkenyloxy, $C_9$-$C_{16}$-alkoxyphenoxyalkenyloxy, $C_9$-$C_{16}$-phenoxyalkoxyalkenyloxy, $C_8$-$C_{14}$-phenoxyalkynyloxy, $C_8$-$C_{14}$-halophenoxyalkynyloxy, $C_8$-$C_{14}$-phenoxyhaloalkynyloxy, $C_8$-$C_{14}$-halophenoxyhaloalkynyloxy, $C_9$-$C_{16}$-alkoxyphenoxyalkynyloxy, $C_9$-$C_{16}$-phenoxyalkoxyalkynyloxy, $C_8$-$C_{14}$-phenylalkynyloxy, $C_{14}$-$C_{20}$-phenoxyphenylalkynyloxy, carboxyl, $C_2$-$C_8$-alkoxycarbonyl, $C_2$-$C_8$-alkylcarbonyloxy, $C_3$-$C_{12}$-alkenyloxycarbonyl, $C_3$-$C_{12}$-alkenylcarbonyloxy, $C_3$-$C_{12}$-alkynyloxycarbonyl, $C_3$-$C_{12}$-alkynylcarbonyloxy, phenoxycarbonyl, phenylcarbonyloxy, $C_3$-$C_{12}$-alkoxycarbonylalkoxy, phenoxy, $C_7$-$C_{14}$-alkylphenoxy, halophenoxy, dihalophenoxy, trihalophenoxy, $C_7$-$C_{14}$-haloalkylphenoxy, $C_7$-$C_{14}$-alkoxyphenoxy, phenoxyphenoxy, $C_8$-$C_{14}$-alkylenedioxyphenoxy, phenylthio, $C_7$-$C_{14}$-alkylphenylthio, halophenylthio, dihalophenylthio, trihalophenylthio, $C_7$-$C_{14}$-haloalkylphenylthio, $C_7$-$C_{14}$-alkoxyphenylthio, phenoxyphenylthio, $C_8$-$C_{14}$-alkylenedioxyphenylthio, phenyl, phenoxyphenyl, $C_7$-$C_{14}$-phenylalkyl, $C_{13}$-$C_{20}$-phenoxyphenylalkyl, $C_8$-$C_{14}$-alkylenedioxyphenylalkyloxy or a radical selected from

$-CH=N-OH$ ; 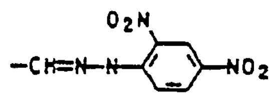 ; 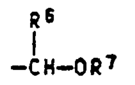 ; 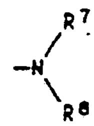 ;

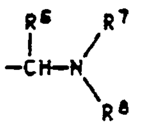 ; 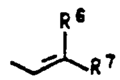 ; 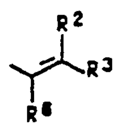 ; 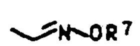 and 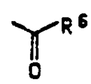 .

$R^3$ is hydrogen, $C_1$-$C_8$-alkyl or $C_1$-$C_2$-haloalkyl,

$R^4$ and $R_5$ are each hydrogen, fluorine, chlorine, $C_1$-$C_3$-alkyl, $C_1$-$C_3$-alkoxy or $C_1$-$C_3$-haloalkoxy,

$R^6$ is hydrogen, $C_1$-$C_8$-alkyl, $C_2$-$C_8$-alkenyl, $C_2$-$C_8$-alkynyl, cyano, phenyl, halophenyl, $C_7$-$C_{14}$-alkylphenyl or $C_7$-$C_4$-alkoxyphenyl,

$R^7$ is hydrogen, $C_1$-$C_8$-alkyl, $C_1$-$C_8$-haloalkyl, $C_2$-$C_8$-alkoxyalkyl, $C_2$-$C_8$-alkenyl, $C_2$-$C_8$-alkynyl, aryl, halophenyl, dihalophenyl, trihalophenyl, cyanophenyl, $C_7$-$C_{14}$-alkylphenyl, $C_7$-$C_{14}$-alkoxyphenyl, $C_7$-$C_{14}$-haloalkylphenyl, $C_8$-$C_{16}$-alkenylphenyl, $C_8$-$C_{16}$-alkynylphenyl, phenoxyphenyl, $C_8$-$C_{16}$-methylenedioxyphenyl, $C_2$-$C_8$-alkylcarbonyl, $C_2$-$C_8$-haloalkylcarbonyl, $C_2$-$C_8$-alkoxycarbonyl, $C_5$-$C_{12}$-alkylcyclopropylcarbonyl, $C_7$-$C_{14}$-alkenyl- and -alkylcyclopropylcarbonyl, $C_6$-$C_{14}$-haloalkyl- and -alkylcyclopropylcarbonyl, $C_7$-$C_{14}$-haloalkenyl- and -alkylcyclopropylcarbonyl, $C_8$-$C_{16}$-halo- and -phenylalkylcarbonyl, $C_8$-$C_{16}$-halo- and -halophenylalkylcarbonyl, benzoyl, halobenzoyl, $C_8$-$C_{14}$-alkylbenzoyl, $C_8$-$C_{14}$-alkoxybenzoyl, $C_8$-$C_{14}$-haloalkylbenzoyl, $C_8$-$C_{14}$-haloalkoxybenzoyl, $C_8$-$C_{14}$-phenylalkylcarbonyl, $C_8$-$C_{14}$-halophenylalkylcarbonyl, $C_9$-$C_{16}$-alkylphenylalkylcarbonyl, $C_9$-$C_{16}$-alkoxyphenylalkylcarbonyl, $C_9$-$C_{16}$-haloalkylphenylalkylcarbonyl, $C_9$-$C_{16}$-haloalkoxyphenylalkylcarbonyl, $C_9$-$C_{16}$-phenylalkenylcarbonyl, $C_9$-$C_{16}$-halophenylalkenylcarbonyl, $C_{10}$-$C_{16}$-alkylphenylalkenylcarbonyl, $C_{10}$-$C_{16}$-alkoxyphenylalkenylcarbonyl, $C_{10}$-$C_{16}$-haloalkylphenylalkenylcarbonyl, $C_{10}$-$C_{16}$-haloalkoxyphenylalkenylcarbonyl, $C_9$-$C_{16}$-phenylalkynylcarbonyl, $C_9$-$C_{16}$-halophenylalkynylcarbonyl, $C_{10}$-$C_{16}$-alkylphenylalkynylcarbonyl, $C_{10}$-$C_{16}$-alkoxyphenylalkynylcarbonyl, $C_{10}$-$C_{16}$-haloalkylphenylalkynylcarbonyl, $C_{10}$-$C_{16}$-haloalkoxyphenylalkynylcarbonyl, $C_2$-$C_8$-alkylaminocarbonyl, $C_2$-$C_8$-haloalkylaminocarbonyl, $C_3$-$C_{10}$-alkylalkylaminocarbonyl, $C_3$-$C_{10}$-alkoxyalkylaminocarbonyl, $C_3$-$C_{10}$-haloalkylalkylaminocarbonyl, $C_3$-$C_{10}$-haloalkoxyalkylaminocarbonyl, $C_3$-$C_{10}$-dialkylaminocarbonyl, $C_3$-$C_8$-halodialkylaminocarbonyl, $C_4$-$C_{12}$-alkyldialkylaminocarbonyl, $C_4$-$C_{12}$-alkoxydialkylaminocarbonyl, $C_5$-$C_{20}$-alkyl- and -alkoxyaminocarbonyl, $C_4$-$C_{12}$-haloalkyldialkylaminocarbonyl, $C_4$-$C_{12}$-haloalkoxydialkylaminocarbonyl, phenylaminocarbonyl, halophenylaminocarbonyl, dihalophenylaminocarbonyl, trihalophenylaminocarbonyl, $C_8$-$C_{14}$-alkylphenylaminocarbonyl, $C_8$-$C_{14}$-alkoxyphenylaminocarbonyl, $C_8$-$C_{14}$-haloalkoxyphenylaminocarbonyl or an unsubstituted or $C_1$-$C_4$-alkyl-substituted isoxazole,

$R^8$ is hydrogen or $C_1$-$C_8$-alkyl and
X is halogen,
with the proviso that, in formula Ia, $R^1$ is not phenyl when $R^2$, $R^3$, $R^4$ and $R^5$ are simultaneously hydrogen, and, in formula Ib, $R^2$, $R^3$, $R^4$ and $R^5$ are not simultaneously hydrogen when $R^1$ is $C_4$-$C_8$-alkyl, phenyl, tolyl or methoxyphenyl, and $R^1$ is not octyl when $R^2$ and $R^3$ are each hydrogen and $R^4$ or $R^5$ is methyl, methoxy or chlorine, and with the further proviso that, in formula Ib, $R^1$ is not n-butyl when $R^2$ is methyl and $R^3$, $R^4$ and $R^5$ are each hydrogen.

3. A process for preparing a pyran derivative of the general formula Ia, Ib or Ic as claimed in claim 1, wherein a vinyl ether of the general formula II

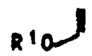

(II)

is reacted in a conventional manner in the presence of a free radical inhibitor and a basic compound at from 130 to 280°C and from 1 to 300 bar

a) with a monomeric α,β-unsaturated aldehyde or ketone III

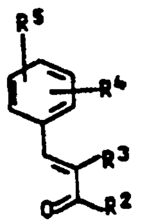

(III)

or

b) with a dimeric α,β-unsaturated aldehyde or ketone IIIa

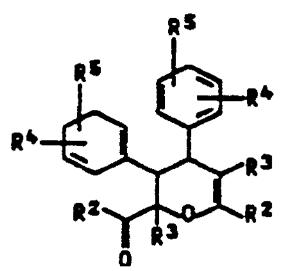

(IIIa)

and the compound Ib is if necessary

c) hydrogenated to give a compound Ia, or
d) if $R^2$ = H, hydroformylated, or
e) if $R^2$ and $R^3$ are each H, reacted with a trihalomethane compound $CHX_3$ to give a compound Ic.

4. A pesticide containing a pyran derivative Ia, Ib or Ic as claimed in claim 1.

5. A pesticide as claimed in claim 4 containing a pyran derivative Ia, Ib or Ic as claimed in claim 2, and conventional carriers therefor.

6. A pesticide as claimed in either of claims 4 and 5 containing from 0.1 to 95% by weight of a pyran derivative Ia, Ib or Ic as claimed in claim 1 or 2.

7. A process for controlling pests, wherein the pests or the areas or spaces to be kept free of pests are treated with a pesticidally effective amount of a pyran derivative of the formula Ia, Ib or Ic as claimed in claim 1 or with a pesticide as claimed in claim 3.

**Claims for the Contracting State ES**

1. A process for preparing a pyran derivative of the general formula Ia, Ib or Ic

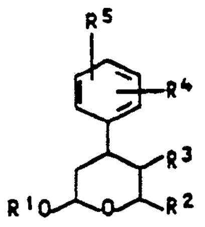 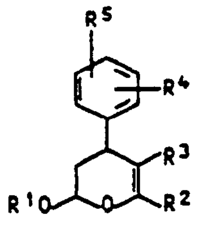 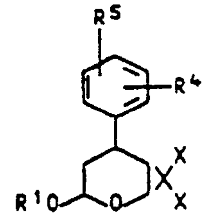

(Ia) (Ib) (Ic)

where

$R^1$ is $C_1$-$C_{20}$-alkyl, $C_2$-$C_{20}$-alkenyl, $C_2$-$C_{20}$-alkynyl, $C_3$-$C_{20}$-cycloalkyl, aryl, biphenylyl, $C_7$-$C_{30}$-aralkyl, $C_8$-$C_{30}$-arylalkenyl, $C_8$-$C_{30}$-arylalkynyl, $C_2$-$C_{20}$-alkoxyalkyl, $C_3$-$C_{20}$-alkoxyalkoxyalkyl, $C_4$-$C_{30}$-alkoxyalkoxyalkoxyalkyl, $C_1$-$C_{20}$-alkyl which is substituted by $C_7$-$C_{20}$-alkylaryl, $C_8$-$C_{20}$-dialkylaryl, $C_9$-$C_{20}$-trialkylaryl, aryloxy, aryloxyaryl, $C_3$-$C_{20}$-cycloalkyl, $C_4$-$C_{20}$-alkylcycloalkyl, $C_4$-$C_{20}$-cycloalkenyl or $C_5$-$C_{20}$-alkylcycloalkenyl, $C_4$-$C_{20}$-alkylcycloalkyl, $C_9$-$C_{20}$-arylcycloalkyl, $C_9$-$C_{30}$-aryloxycycloalkyl, $C_7$-$C_{20}$-alkylaryl, $C_7$-$C_{20}$-alkoxyaryl, $C_8$-$C_{20}$-dialkylaryl, $C_7$-$C_{20}$-haloalkylaryl, haloaryl, dihaloaryl, $C_7$-$C_{20}$-alkylhaloaryl, $C_8$-$C_{20}$-dialkylhaloaryl, aryloxyaryl or $C_{13}$-$C_{30}$-aryloxyarylalkyl, or is $C_1$-$C_{20}$-alkyl which is substituted by a saturated or unsaturated 5-membered, 6-membered, 7membered or 8-membered heterocyclic structure which has one, two or three identical or different hetero atoms from the group consisting of oxygen, sulfur and nitrogen and is unsubstituted or substituted by one, two or three identical or different substituents from the group consisting of $C_1$-$C_{20}$-alkyl, aryl, $C_7$-$C_{20}$-aralkyl, haloaryl, $C_7$-$C_{20}$-alkylaryl, $C_8$-$C_{20}$-dialkylaryl, $C_9$-$C_{20}$-trialkylaryl or an unsubstituted or $C_1$-$C_8$-alkyl-substituted $C_3$-$C_{20}$-spirocycloalkyl,

$R^2$ is hydrogen, $C_1$-$C_{20}$-alkyl, unsubstituted, phenyl-substituted or halophenyl-substituted $C_1$-$C_{20}$-haloalkyl; $C_1$-$C_{20}$-alkoxy, $C_1$-$C_{20}$-haloalkoxy, $C_2$-$C_{20}$-alkenyloxy, $C_2$-$C_{20}$-haloalkenyloxy, $C_3$-$C_{20}$-alkoxyalkenyloxy, $C_2$-$C_{20}$-alkynyloxy, $C_2$-$C_{20}$-haloalkynyloxy, $C_3$-$C_{20}$-alkoxy-alkynyloxy, $C_8$-$C_{20}$-phenylalkynyloxy, $C_1$-$C_{20}$-alkylthio, $C_1$-$C_{20}$-haloalkylthio, $C_2$-$C_{20}$-alkoxyalkylthio, formyl, cyano, $C_7$-$C_{20}$-phenoxyalkoxy, $C_7$-$C_{20}$-halophenoxyalkoxy, $C_7$-$C_{20}$-phenoxyhaloalkoxy, $C_7$-$C_{20}$-halophenoxyhaloalkoxy, $C_8$-$C_{20}$alkoxyphenoxyalkoxy, $C_8$-$C_{20}$-phenoxyalkoxyalkoxy, $C_7$-$C_{20}$-phenoxyalkenyloxy, $C_8$-$C_{20}$-halophenoxyalkenyl, $C_8$-$C_{20}$-phenoxyhaloalkenyloxy, $C_8$-$C_{20}$-halophenoxyhaloalkenyloxy, $C_9$-$C_{20}$-alkoxyphenoxyalkenyloxy, $C_9$-$C_{20}$-phenoxyalkoxyalkenyloxy, $C_8$-$C_{20}$-phenoxyalkynyloxy, $C_8$-$C_{20}$-halophenoxyalkynyloxy, $C_8$-$C_{20}$-phenoxyhaloalkynyloxy, $C_8$-$C_{20}$-halophenoxyhaloalkynyloxy, $C_9$-$C_{20}$-alkoxyphenoxyalkynyloxy, $C_9$-$C_{20}$-phenoxyalkoxyalkynyloxy, $C_8$-$C_{20}$-phenylalkynyloxy, $C_{14}$-$C_{30}$-phenoxyphenylalkynyloxy, carboxyl, $C_2$-$C_{20}$-alkoxycarbonyl, $C_2$-$C_{20}$-alkylcarbonyloxy, $C_3$-$C_{20}$-alkenyloxycarbonyl, $C_3$-$C_{20}$-alkenylcarbonyloxy, $C_3$-$C_{20}$-alkynyloxycarbonyl, $C_3$-$C_{20}$-alkynylcarbonyloxy, phenoxycarbonyl, phenylcarbonyloxy, $C_3$-$C_{20}$-alkoxycarbonylalkoxy, phenoxy, $C_7$-$C_{20}$-alkylphenoxy, halophenoxy, dihalophenoxy, trihalophenoxy, $C_7$-$C_{20}$-haloalkylphenoxy, $C_7$-$C_{20}$-alkoxy-phenoxy, phenoxyphenoxy, $C_8$-$C_{12}$-alkylenedioxyphenoxy, phenylthio, $C_7$-$C_{20}$-alkylphenylthio, halophenylthio, dihalophenylthio, trihalophenylthio, $C_7$-$C_{20}$-haloalkylphenylthio, $C_7$-$C_{20}$-alkoxyphenylthio, phenoxyphenylthio, $C_8$-$C_{12}$-alkylenedioxyphenylthio, phenyl, phenoxyphenyl, $C_7$-$C_{20}$-phenylalkyl, $C_{13}$-$C_{30}$-phenoxyphenylalkyl, $C_8$-$C_{12}$-alkylenedioxyphenylalkoxy or a radical selected from

$-CH=N-OH$ ; 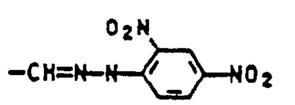 ; 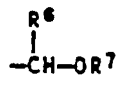 ; 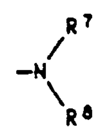 ;

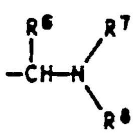 ; 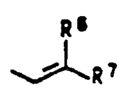 ; 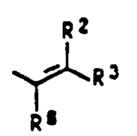 ; 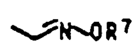 and 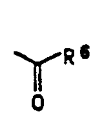 .

$R^3$ is hydrogen, $C_1$-$C_{20}$-alkyl or $C_1$-$C_3$-haloalkyl,

$R^4$ and $R^5$ are each hydrogen, fluorine, chlorine, $C_1$-$C_3$-alkyl, $C_1$-$C_3$-alkoxy or $C_1$-$C_3$-haloalkoxy,

$R^6$ is hydrogen, $C_1$-$C_{20}$-alkyl, $C_2$-$C_{20}$-alkenyl, $C_2$-$C_{20}$-alkynyl, cyano, phenyl, halophenyl, $C_7$-$C_{20}$-alkylphenyl or $C_7$-$C_{20}$-alkoxyphenyl,

$R^7$ is hydrogen, $C_1$-$C_{20}$-alkyl, $C_1$-$C_{20}$-haloalkyl, $C_2$-$C_{20}$ alkoxyalkyl, $C_2$-$C_{20}$-alkenyl, $C_2$-$C_{20}$-alkynyl, aryl, halophenyl, dihalophenyl, trihalophenyl, cyanophenyl, $C_7$-$C_{20}$-alkylphenyl, $C_7$-$C_{20}$-alkoxyphenyl, $C_7$-$C_{20}$-

haloalkylphenyl, $C_8$-$C_{20}$-alkenylphenyl, $C_8$-$C_{20}$-alkynylphenyl, phenoxyphenyl, $C_8$-$C_{12}$-methylenedioxyphenyl, $C_2$-$C_{20}$-alkylcarbonyl, $C_2$-$C_{20}$-haloalkylcarbonyl, $C_2$-$C_{20}$-alkoxycarbonyl, $C_5$-$C_{20}$-alkylcyclopropylcarbonyl, $C_7$-$C_{20}$-alkenyl- and -alkylcyclopropylcarbonyl, $C_6$-$C_{20}$-haloalkyl- and -alkylcyclopropylcarbonyl, $C_7$-$C_{20}$-haloalkenyl- and -alkylcyclopropylcarbonyl, $C_8$-$C_{20}$-halo- and -phenylalkylcarbonyl, $C_8$-$C_{20}$-halo- and -halophenylalkylcarbonyl, benzoyl, halobenzoyl, $C_8$-$C_{20}$-alkylbenzoyl, $C_8$-$C_{20}$-alkoxybenzoyl, $C_8$-$C_{20}$-haloalkylbenzoyl, $C_8$-$C_{20}$-haloalkoxybenzoyl, $C_8$-$C_{20}$-phenylalkylcarbonyl, $C_8$-$C_{20}$-halophenylalkylcarbonyl, $C_9$-$C_{20}$-alkylphenylalkylcarbonyl, $C_9$-$C_{20}$-alkoxyphenylalkylcarbonyl, $C_9$-$C_{20}$-haloalkylphenylalkylcarbonyl, $C_9$-$C_{20}$-haloalkoxyphenylalkylcarbonyl, $C_9$-$C_{20}$-phenylalkenylcarbonyl, $C_9$-$C_{20}$-halophenylalkenylcarbonyl, $C_{10}$-$C_{20}$-alkylphenylalkenylcarbonyl, $C_{10}$-$C_{20}$-alkoxyphenylalkenylcarbonyl, $C_{10}$-$C_{20}$-haloalkylphenylalkenylcarbonyl, $C_{10}$-$C_{20}$-haloalkoxyphenylalkenylcarbonyl, $C_9$-$C_{20}$-phenylalkynylcarbonyl, $C_9$-$C_{20}$-halophenylalkynylcarbonyl, $C_{10}$-$C_{20}$-alkylphenylalkynylcarbonyl, $C_{10}$-$C_{20}$-alkoxyphenylalkynylcarbonyl, $C_{10}$-$C_{20}$-haloalkylphenylalkynylcarbonyl, $C_{10}$-$C_{20}$-haloalkoxyphenyl-alkynylcarbonyl, $C_2$-$C_{20}$-alkylaminocarbonyl, $C_2$-$C_{20}$-haloalkylaminocarbonyl, $C_3$-$C_{20}$-alkoxyalkylaminocarbonyl, $C_3$-$C_{20}$-haloalkylalkylaminocarbonyl, $C_3$-$C_{20}$-haloalkoxyalkylaminocarbonyl, $C_3$-$C_{20}$-dialkylaminocarbonyl, $C_3$-$C_{20}$-halodialkylaminocarbonyl, $C_4$-$C_{20}$-alkoxydialkylaminocarbonyl, $C_5$-$C_{20}$-alkyl- and -alkoxyaminocarbonyl, $C_4$-$C_{20}$-haloalkyldialkylaminocarbonyl, $C_4$-$C_{20}$-haloalkoxydialkylaminocarbonyl, phenylaminocarbonyl, halophenylaminocarbonyl, dihalophenylaminocarbonyl, trihalophenylaminocarbonyl, $C_8$-$C_{20}$-alkylphenylaminocarbonyl, $C_8$-$C_{20}$-alkoxyphenylaminocarbonyl, $C_8$-$C_{20}$-haloalkoxyphenylaminocarbonyl or an unsubstituted or $C_1$-$C_8$-alkyl-substituted isoxazole,

$R^8$ is hydrogen or $C_1$-$C_{20}$-alkyl and

X is halogen,

with the proviso that, in the case of compound Ia, $R^1$ is not phenyl when $R^2$, $R^3$, $R^4$ and $R^5$ are simultaneously hydrogen, and, in the case of compound Ib, $R^2$, $R^3$, $R^4$ and $R^5$ are not simultaneously hydrogen when $R^1$ is $C_4$-$C_8$-alkyl, phenyl, tolyl or methoxyphenyl, and $R^1$ is not octyl when $R^2$ and $R^3$ are each hydrogen and $R^4$ or $R^5$ is methyl, methoxy or chlorine, and with the further proviso that, in formula Ib, $R^1$ is not n-butyl when $R^2$ is methyl and $R^3$, $R^4$ and $R^5$ are each hydrogen, wherein a vinyl ether of the general formula II

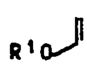

(II)

is reacted in a conventional manner in the presence of a free radical inhibitor and a basic compound at from 130 to 280°C and from 1 to 300 bar

a) with a monomeric α,β-unsaturated aldehyde or ketone III

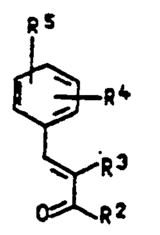

(III)

or

b) with a dimeric α,β-unsaturated aldehyde or ketone IIIa

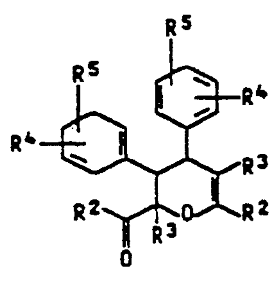

(IIIa)

and the compound Ib is if necessary

c) hydrogenated to give a compound Ia, or

d) if $R^2$ = H, hydroformylated, or

e) if $R^2$ and $R^3$ are each H, reacted with a trihalomethane compound $CHX_3$ to give a compound Ic.

2. A process as claimed in claim 1 for preparing a pyran derivative of the formula Ia, Ib or Ic as claimed in claim 1 where

$R^1$ is $C_1$-$C_{12}$-alkyl, $C_2$-$C_{14}$-alkenyl, $C_2$-$C_8$-alkynyl, $C_3$-$C_8$-cycloalkyl, phenyl, naphthyl, biphenylyl, $C_7$-$C_{14}$-phenylalkyl, $C_8$-$C_{14}$-phenylalkenyl, $C_8$-$C_{14}$-phenyl-alkynyl, $C_2$-$C_8$-alkoxyalkyl, $C_3$-$C_{12}$-alkoxyalkoxyalkyl, $C_4$-$C_{16}$-alkoxyalkoxyalkoxyalkyl, $C_1$-$C_8$-alkyl which is substituted by $C_7$-$C_{10}$-alkylaryl, $C_8$-$C_{14}$-dialkylphenyl, $C_9$-$C_{14}$-trialkylaryl, phenoxy, phenoxyphenyl, $C_3$-$C_8$-cycloalkyl, $C_4$-$C_{12}$-alkylcycloalkyl, $C_4$-$C_8$-cycloalkenyl or $C_5$-$C_{12}$-alkylcycloalkenyl, $C_4$-$C_{12}$-alkylcycloalkyl, $C_9$-$C_{14}$-phenylcycloalkyl, $C_9$-$C_{14}$-phenoxycycloalkyl, $C_7$-$C_{14}$-alkylphenyl, $C_7$-$C_{14}$-alkoxyphenyl, $C_8$-$C_{16}$-dialkylphenyl, $C_7$-$C_{14}$-haloalkylphenyl, halophenyl, dihalophenyl, $C_7$-$C_{14}$-alkylhalophenyl, $C_8$-$C_{14}$-dialkylhalophenyl, phenoxyphenyl or $C_{13}$-$C_{20}$-phenoxyphenylalkyl, or is $C_1$-$C_8$-alkyl which is substituted by a saturated or unsaturated 5-membered or 6-membered heterocyclic structure which has one or two identical or different hetero atoms from the group consisting of oxygen and nitrogen and is unsubstituted or substituted by one, two or three identical or different substituents from the group consisting of $C_1$-$C_8$-alkyl, phenyl, $C_7$-$C_{14}$-phenylalkyl, halophenyl, $C_7$-$C_{14}$-alkylphenyl, $C_8$-$C_{16}$-dialkylphenyl, $C_9$-$C_{16}$-trialkylaryl or an unsubstituted or $C_1$-$C_4$-alkyl-substituted $C_3$-$C_8$-spirocycloalkyl,

$R^2$ is hydrogen, $C_1$-$C_8$-alkyl, $C_1$-$C_8$-haloalkyl, which is unsubstituted or substituted by phenyl or halophenyl; $C_1$-$C_8$-alkoxy, $C_1$-$C_8$-haloalkoxy, $C_2$-$C_8$-alkenyloxy, $C_2$-$C_8$-haloalkenyloxy, $C_3$-$C_{12}$-alkoxyalkenyloxy, $C_2$-$C_8$-alkynyloxy, $C_2$-$C_8$-haloalkynyloxy, $C_3$-$C_{12}$-alkoxyalkynyloxy, $C_8$-$C_{16}$-phenylalkynyloxy, $C_1$-$C_8$-alkylthio, $C_1$-$C_8$-haloalkylthio, $C_2$-$C_{12}$-alkoxyalkylthio, formyl, cyano, $C_7$-$C_{14}$-phenoxyalkoxy, $C_7$-$C_{14}$-halophenoxyalkoxy, $C_7$-$C_{14}$-phenoxyhaloalkoxy, $C_7$-$C_{14}$-halophenoxyhaloalkoxy, $C_8$-$C_{16}$-alkoxyphenoxyalkoxy, $C_8$-$C_{16}$-phenoxyalkoxyalkoxy, $C_7$-$C_{16}$-phenoxyalkenyloxy, $C_8$-$C_{16}$-halophenoxyalkenyl, $C_8$-$C_{16}$-phenoxyhaloalkenyloxy, $C_8$-$C_{16}$-halophenoxyhaloalkenyloxy, $C_9$-$C_{16}$-alkoxyphenoxyalkenyloxy, $C_9$-$C_{16}$-phenoxyalkoxyalkenyloxy, $C_8$-$C_{14}$-phenoxyalkynyloxy, $C_8$-$C_{14}$-halophenoxyalkynyloxy, $C_8$-$C_{14}$-phenoxyhaloalkynyloxy, $C_8$-$C_{14}$-halophenoxyhaloalkynyloxy, $C_9$-$C_{16}$-alkoxyphenoxyalkynyloxy, $C_9$-$C_{16}$-phenoxyalkoxyalkynyloxy, $C_8$-$C_{14}$-phenylalkynyloxy, $C_{14}$-$C_{20}$-phenoxy-phenylalkynyloxy, carboxyl, $C_2$-$C_8$-alkoxycarbonyl, $C_2$-$C_8$-alkylcarbonyloxy, $C_3$-$C_{12}$-alkenyloxycarbonyl, $C_3$-$C_{12}$-alkenylcarbonyloxy, $C_3$-$C_{12}$-alkyloxycarbonyl, $C_3$-$C_{12}$-alkynylcarbonyloxy, phenoxycarbonyl, phenylcarbonyloxy, $C_3$-$C_{12}$-alkoxycarbonylalkoxy, phenoxy, $C_7$-$C_{14}$-alkylphenoxy, halophenoxy, dihalophenoxy, trihalophenoxy, $C_7$-$C_{14}$-haloalkylphenoxy, $C_7$-$C_{14}$-alkoxyphenoxy, phenoxyphenoxy, $C_8$-$C_{14}$-alkylenedioxyphenoxy, phenylthio, $C_7$-$C_4$-alkylphenylthio, halophenylthio, dihalophenylthio, trihalophenylthio, $C_7$-$C_{14}$-haloalkylphenylthio, $C_7$-$C_{14}$-alkoxyphenylthio, phenoxyphenylthio, $C_8$-$C_{14}$-alkylenedioxyphenylthio, phenyl, phenoxyphenyl, $C_7$-$C_{14}$-phenylalkyl, $C_{13}$-$C_{20}$-phenoxyphenylalkyl, $C_8$-$C_{14}$-alkylenedioxyphenylalkyloxy or a radical selected from

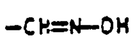 ; 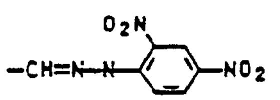 : 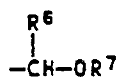 ; 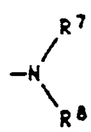 ;

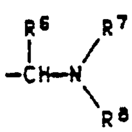 ; 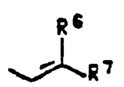 ; 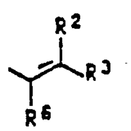 ; 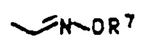 and 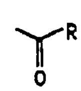 .

$R^3$ is hydrogen, $C_1$-$C_8$-alkyl or $C_1$-$C_2$-haloalkyl,

$R^4$ and $R^5$ are each hydrogen, fluorine, chlorine, $C_1$-$C_3$-alkyl, $C_1$-$C_3$-alkoxy or $C_1$-$C_3$-haloalkoxy,

$R^6$ is hydrogen, $C_1$-$C_8$-alkyl, $C_2$-$C_8$-alkenyl, $C_2$-$C_8$-alkynyl, cyano, phenyl, halophenyl, $C_7$-$C_{14}$-alkylphenyl or $C_7$-$C_{14}$-alkoxyphenyl,

$R^7$ is hydrogen, $C_1$-$C_8$-alkyl, $C_1$-$C_8$-haloalkyl, $C_2$-$C_8$-alkoxyalkyl, $C_2$-$C_8$-alkenyl, $C_2$-$C_8$-alkynyl, aryl, halophenyl, dihalophenyl, trihalophenyl, cyanophenyl, $C_7$-$C_{14}$-alkylphenyl, $C_7$-$C_{14}$-alkoxyphenyl, $C_7$-$C_{14}$-haloalkylphenyl, $C_8$-$C_{16}$-alkenylphenyl, $C_8$-$C_{16}$-alkynylphenyl, phenoxyphenyl, $C_8$-$C_{16}$-methylenedioxyphenyl, $C_2$-$C_8$-alkylcarbonyl, $C_2$-$C_8$-haloalkylcarbonyl, $C_2$-$C_8$-alkoxycarbonyl, $C_5$-$C_{12}$-alkylcyclopropylcarbonyl, $C_7$-$C_{14}$-alkenyl- and -alkylcyclopropylcarbonyl, $C_6$-$C_{14}$-haloalkyl- and -alkylcyclopropylcarbonyl, $C_7$-$C_{14}$-haloalkenyl- and -alkylcyclopropylcarbonyl, $C_8$-$C_{16}$-halo- and -phenylalkylcarbonyl, $C_8$-$C_{16}$-halo- and -halophenylalkylcarbonyl, benzoyl, halobenzoyl, $C_8$-$C_{14}$-alkylbenzoyl, $C_8$-$C_{14}$-alkoxybenzoyl, $C_8$-$C_{14}$-haloalkylbenzoyl, $C_8$-$C_{14}$-haloalkoxybenzoyl, $C_8$-$C_{14}$-phenylalkylcarbonyl, $C_8$-$C_{14}$-halophenylalkyl-

carbonyl, $C_9$-$C_{16}$-alkylphenylalkylcarbonyl, $C_9$-$C_{16}$-alkoxyphenylalkylcarbonyl, $C_9$-$C_{16}$-haloalkylphenylalkylcarbonyl, $C_9$-$C_{16}$-haloalkoxyphenylalkylcarbonyl, $C_9$-$C_{16}$-phenylalkenylcarbonyl, $C_9$-$C_{16}$-halophenylalkenylcarbonyl, $C_{10}$-$C_{16}$-alkylphenylalkenylcarbonyl, $C_{10}$-$C_{16}$-alkoxyphenylalkenylcarbonyl, $C_{10}$-$C_{16}$-haloalkylphenylalkenylcarbonyl, $C_{10}$-$C_{16}$-haloalkoxyphenylalkenylcarbonyl, $C_9$-$C_{16}$-phenylalkynylcarbonyl, $C_9$-$C_{16}$-halophenylalkynylcarbonyl, $C_{10}$-$C_{16}$-alkylphenylalkynylcarbonyl, $C_{10}$-$C_{16}$-alkoxyphenylalkynylcarbonyl, $C_{10}$-$C_{16}$-haloalkylphenylalkynylcarbonyl, $C_{10}$-$C_{16}$-haloalkoxyphenylalkynylcarbonyl, $C_2$-$C_8$-alkylaminocarbonyl, $C_2$-$C_8$-haloalkylaminocarbonyl, $C_3$-$C_{10}$-alkylalkylaminocarbonyl, $C_3$-$C_{10}$-alkoxyalkylaminocarbonyl, $C_3$-$C_{10}$-haloalkylalkylaminocarbonyl, $C_3$-$C_{10}$-haloalkoxyalkylaminocarbonyl, $C_3$-$C_{10}$-dialkylaminocarbonyl, $C_3$-$C_8$-halodialkylaminocarbonyl, $C_4$-$C_{12}$-alkyldialkylaminocarbonyl, $C_4$-$C_{12}$-alkoxydialkylaminocarbonyl, $C_5$-$C_{20}$-alkyl- and -alkoxyaminocarbonyl, $C_4$-$C_{12}$-haloalkyldialkylaminocarbonyl, $C_4$-$C_{12}$-haloalkoxydialkylaminocarbonyl, phenylaminocarbonyl, halophenylaminocarbonyl, dihalophenylaminocarbonyl, trihalophenylaminocarbonyl, $C_8$-$C_{14}$-alkylphenylaminocarbonyl, $C_8$-$C_{14}$-alkoxyphenylaminocarbonyl, $C_8$-$C_{14}$-haloalkoxyphenylaminocarbonyl or an unsubstituted or $C_1$-$C_4$-alkyl-substituted isoxazole,

$R^8$ is hydrogen or $C_1$-$C_8$-alkyl and

X is halogen,

with the proviso that, in formula Ia, $R^1$ is not phenyl when $R^2$, $R^3$, $R^4$ and $R^5$ are simultaneously hydrogen, and, in formula Ib, $R^2$, $R^3$, $R^4$ and $R^5$ are not simultaneously hydrogen when $R^1$ is $C_4$-$C_8$-alkyl, phenyl, tolyl or methoxyphenyl, and $R^1$ is not octyl when $R^2$ and $R^3$ are each hydrogen and $R^4$ or $R^5$ is methyl, methoxy or chlorine, and with the further proviso that, in formula Ib, $R^1$ is not n-butyl when $R^2$ is methyl and $R^3$, $R^4$ and $R^5$ are each hydrogen.

3. A pesticide containing a pyran derivative Ia, Ib or Ic

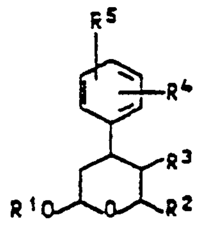

(Ia)

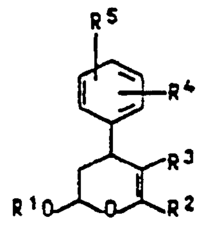

(Ib)

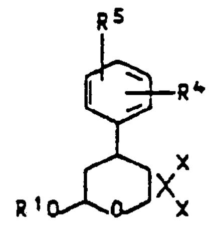

(Ic)

where

$R^1$ is $C_1$-$C_{20}$-alkyl, $C_2$-$C_{20}$-alkenyl, $C_2$-$C_{20}$-alkynyl, $C_3$-$C_{20}$-cycloalkyl, aryl, biphenylyl, $C_7$-$C_{30}$-aralkyl, $C_8$-$C_{30}$-arylalkenyl, $C_8$-$C_{30}$-arylalkynyl, $C_2$-$C_{20}$-alkoxyalkyl, $C_3$-$C_{20}$-alkoxyalkoxyalkyl, $C_4$-$C_{30}$-alkoxyalkoxyalkoxyalkyl, $C_1$-$C_{20}$-alkyl which is substituted by $C_7$-$C_{20}$-alkylaryl, $C_8$-$C_{20}$-dialkylaryl, $C_9$-$C_{20}$-trialkylaryl, aryloxy, aryloxyaryl, $C_3$-$C_{20}$-cycloalkyl, $C_4$-$C_{20}$-alkylcycloalkyl, $C_4$-$C_{20}$-cycloalkenyl or $C_5$-$C_{20}$-alkylcycloalkenyl, $C_4$-$C_{20}$-alkylcycloalkyl, $C_9$-$C_{20}$-arylcycloalkyl, $C_9$-$C_{30}$-aryloxycycloalkyl, $C_7$-$C_{20}$-alkylaryl, $C_7$-$C_{20}$-alkoxyaryl, $C_8$-$C_{20}$-dialkylaryl, $C_7$-$C_{20}$-haloalkylaryl, haloaryl, dihaloaryl, $C_7$-$C_{20}$-alkylhaloaryl, $C_8$-$C_{20}$-dialkylhaloaryl, aryloxyaryl or $C_{13}$-$C_{30}$-aryloxyaryl-alkyl, or is $C_1$-$C_{20}$-alkyl which is substituted by a saturated or unsaturated 5-membered, 6-membered, 7membered or 8-membered heterocyclic structure which has one, two or three identical or different hetero atoms from the group consisting of oxygen, sulfur and nitrogen and is unsubstituted or substituted by one, two or three identical or different substituents from the group consisting of $C_1$-$C_{20}$-alkyl, aryl, $C_7$-$C_{20}$-aralkyl, haloaryl, $C_7$-$C_{20}$-alkylaryl, $C_8$-$C_{20}$-dialkylaryl, $C_9$-$C_{20}$-trialkylaryl or an unsubstituted or $C_1$-$C_8$-alkyl-substituted $C_3$-$C_{20}$-spirocycloalkyl,

$R^2$ is hydrogen, $C_1$-$C_{20}$-alkyl, unsubstituted, phenyl-substituted or halophenyl-substituted $C_1$-$C_{20}$-haloalkyl; $C_7$-$C_{20}$-halophenylhaloalkyl, $C_1$-$C_{20}$-alkoxy, $C_1$-$C_{20}$-haloalkoxy, $C_2$-$C_{20}$-alkenyloxy, $C_2$-$C_{20}$-haloalkenyloxy, $C_3$-$C_{20}$-alkoxyalkenyloxy, $C_2$-$C_{20}$-alkynyloxy, $C_2$-$C_{20}$-haloalkynyloxy, $C_3$-$C_{20}$-alkoxyalkynyloxy, $C_8$-$C_{20}$-phenylalkynyloxy, $C_1$-$C_{20}$-alkylthio, $C_1$-$C_{20}$-haloalkylthio, $C_2$-$C_{20}$-alkoxyalkylthio, formyl, cyano, $C_7$-$C_{20}$-phenoxyalkoxy, $C_7$-$C_{20}$-halophenoxyalkoxy, $C_7$-$C_{20}$-phenoxyhaloalkoxy, $C_7$-$C_{20}$-halophenoxyhaloalkoxy, $C_8$-$C_{20}$-alkoxyphenoxyalkoxy, $C_8$-$C_{20}$-phenoxyalkoxyalkoxy, $C_7$-$C_{20}$-phenoxyalkenyloxy, $C_8$-$C_{20}$-halophenoxyalkenyl, $C_8$-$C_{20}$-phenoxyhaloalkenyloxy, $C_8$-$C_{20}$-halophenoxyhaloalkenyloxy, $C_9$-$C_{20}$-alkoxyphenoxyalkenyloxy, $C_9$-$C_{20}$-phenoxyalkoxyalkenyloxy, $C_8$-$C_{20}$-phenoxyalkynyloxy, $C_8$-$C_{20}$-halophenoxyalkynyloxy, $C_8$-$C_{20}$-phenoxyhaloalkynyloxy, $C_8$-$C_{20}$-halophenoxyhaloalkynyloxy, $C_9$-$C_{20}$ alkoxyphenoxyalkynyloxy, $C_9$-$C_{20}$-phenoxyalkoxyalkynyloxy, $C_8$-$C_{20}$-phenylalkynyloxy, $C_{14}$-$C_{30}$-phenoxy-phenylalkynyloxy, carboxyl, $C_2$-$C_{20}$-alkoxycarbonyl, $C_2$-$C_{20}$-alkylcarbonyloxy, $C_3$-$C_{20}$-alkenyloxycarbonyl, $C_3$-$C_{20}$-alkenylcarbonyloxy, $C_3$-$C_{20}$-alkynyloxycarbonyl, $C_3$-$C_{20}$-alkynylcar-

bonyloxy, phenoxycarbonyl, phenylcarbonyloxy, $C_3$-$C_{20}$-alkoxycarbonylalkoxy, phenoxy, $C_7$-$C_{20}$-alkylphenoxy, halophenoxy, dihalophenoxy, trihalophenoxy, $C_7$-$C_{20}$-haloalkylphenoxy, $C_7$-$C_{20}$-alkoxyphenoxy, phenoxyphenoxy, $C_8$-$C_{12}$-alkylenedioxyphenoxy, phenylthio, $C_7$-$C_{20}$-alkylphenylthio, halophenylthio, dihalophenylthio, trihalophenylthio, $C_7$-$C_{20}$-haloalkylphenylthio, $C_7$-$C_{20}$-alkoxyphenylthio, phenoxyphenylthio, $C_8$-$C_{12}$-alkylenedioxyphenylthio, phenyl, phenoxyphenyl, $C_7$-$C_{20}$-phenylalkyl, $C_{13}$-$C_{30}$-phenoxyphenylalkyl, $C_8$-$C_{12}$-alkylenedioxyphenylalkoxy or a radical selected from

$-CH=N-OH$ ; 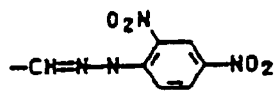 ; 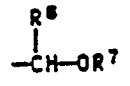 ; 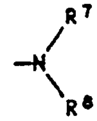 ;

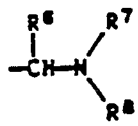 ; 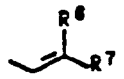 ; 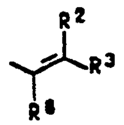 ; 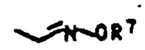 and 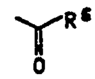 ,

$R^3$ is hydrogen, $C_1$-$C_{20}$-alkyl or $C_1$-$C_3$-haloalkyl,

$R^4$ and $R^5$ are each hydrogen, fluorine, chlorine, $C_1$-$C_3$-alkyl, $C_1$-$C_3$-alkoxy or $C_1$-$C_3$-haloalkoxy,

$R^6$ is hydrogen, $C_1$-$C_{20}$-alkyl, $C_2$-$C_{20}$-alkenyl, $C_2$-$C_{20}$-alkynyl, cyano, phenyl, halophenyl, $C_7$-$C_{20}$-alkylphenyl or $C_7$-$C_{20}$-alkoxyphenyl,

$R^7$ is hydrogen, $C_1$-$C_{20}$-alkyl, $C_1$-$C_{20}$-haloalkyl, $C_2$-$C_{20}$-alkoxyalkyl, $C_2$-$C_{20}$-alkenyl, $C_2$-$C_{20}$-alkynyl, aryl, halophenyl, dihalophenyl, trihalophenyl, cyanophenyl, $C_7$-$C_{20}$-alkylphenyl, $C_7$-$C_{20}$-alkoxyphenyl, $C_7$-$C_{20}$-haloalkylphenyl, $C_8$-$C_{20}$-alkenylphenyl, $C_8$-$C_{20}$-alkynylphenyl, phenoxyphenyl, $C_8$-$C_{12}$-methylenedioxyphenyl, $C_2$-$C_{20}$-alkylcarbonyl, $C_2$-$C_{20}$-haloalkylcarbonyl, $C_2$-$C_{20}$-alkoxycarbonyl, $C_5$-$C_{20}$-alkylcyclopropylcarbonyl, $C_7$-$C_{20}$-alkenyl- and -alkylcyclopropylcarbonyl, $C_6$-$C_{20}$-haloalkyl- and -alkylcyclopropylcarbonyl, $C_7$-$C_{20}$-haloalkenyl- and -alkylcyclopropylcarbonyl, $C_8$-$C_{20}$-halo- and -phenylalkylcarbonyl, $C_8$-$C_{20}$-halo- and -halophenylalkylcarbonyl, benzoyl, halobenzoyl, $C_8$-$C_{20}$-alkylbenzoyl, $C_8$-$C_{20}$-alkoxybenzoyl, $C_8$-$C_{20}$-haloalkylbenzoyl, $C_8$-$C_{20}$-haloalkoxybenzoyl, $C_8$-$C_{20}$-phenylalkylcarbonyl, $C_8$-$C_{20}$-halophenylalkylcarbonyl, $C_9$-$C_{20}$-alkylphenylalkylcarbonyl, $C_9$-$C_{20}$-alkoxyphenylalkylcarbonyl, $C_9$-$C_{20}$-haloalkylphenylalkylcarbonyl, $C_9$-$C_{20}$-haloalkoxyphenylalkylcarbonyl, $C_9$-$C_{20}$-phenylalkenylcarbonyl, $C_9$-$C_{20}$-halophenylalkenylcarbonyl, $C_{10}$-$C_{20}$-alkylphenylalkenylcarbonyl, $C_{10}$-$C_{20}$-alkoxyphenylalkenylcarbonyl, $C_{10}$-$C_{20}$-haloalkylphenylalkenylcarbonyl, $C_{10}$-$C_{20}$-haloalkoxyphenylalkenylcarbonyl, $C_9$-$C_{20}$-phenylalkynylcarbonyl, $C_9$-$C_{20}$-halophenylalkynylcarbonyl, $C_{10}$-$C_{20}$-alkylphenylalkynylcarbonyl, $C_{10}$-$C_{20}$-alkoxyphenylalkynylcarbonyl, $C_{10}$-$C_{20}$-haloalkylphenylalkynylcarbonyl, $C_{10}$-$C_{20}$-haloalkoxyphenylalkynylcarbonyl, $C_2$-$C_{20}$-alkylaminocarbonyl, $C_2$-$C_{20}$-haloalkylaminocarbonyl, $C_3$-$C_{20}$-alkoxyalkylaminocarbonyl, $C_3$-$C_{20}$-haloalkylalkylaminocarbonyl, $C_3$-$C_{20}$-haloalkoxyalkylaminocarbonyl, $C_3$-$C_{20}$-dialkylaminocarbonyl, $C_3$-$C_{20}$-halodialkylaminocarbonyl, $C_4$-$C_{20}$-alkoxydialkylaminocarbonyl, $C_5$-$C_{20}$-alkyl- and -alkoxyaminocarbonyl, $C_4$-$C_{20}$-haloalkyldialkylaminocarbonyl, $C_4$-$C_{20}$-haloalkoxydialkylaminocarbonyl, phenylaminocarbonyl, halophenylaminocarbonyl, dihalophenylaminocarbonyl, trihalophenylaminocarbonyl, $C_8$-$C_{20}$-alkylphenylaminocarbonyl, $C_8$-$C_{20}$-alkoxyphenylaminocarbonyl, $C_8$-$C_{20}$-haloalkoxyphenylaminocarbonyl or an unsubstituted or $C_1$-$C_8$-alkyl-substituted isoxazole,

$R^8$ is hydrogen or $C_1$-$C_{20}$-alkyl and

X is halogen,

with the proviso that, in the case of compound Ia, $R^1$ is not phenyl when $R^2$, $R^3$, $R^4$ and $R^5$ are simultaneously hydrogen, and, in the case of compound Ib, $R^2$, $R^3$, $R^4$ and $R^5$ are not simultaneously hydrogen when $R^1$ is $C_4$-$C_8$-alkyl, phenyl, tolyl or methoxyphenyl, and $R^1$ is not octyl when $R^2$ and $R^3$ are each hydrogen and $R^4$ or $R^5$ is methyl, methoxy or chlorine, and with the further proviso that, in formula Ib, $R^1$ is not n-butyl when $R^2$ is methyl and $R^3$, $R^4$ and $R^5$ are each hydrogen.

4. A pesticide as claimed in claim 3 containing a pyran derivative Ia, Ib or Ic as set forth in claim 3, where $R^1$ is $C_1$-$C_{12}$-alkyl, $C_2$-$C_{14}$-alkenyl, $C_2$-$C_8$-alkynyl, $C_3$-$C_8$-cycloalkyl, phenyl, naphthyl, biphenylyl, $C_7$-$C_{14}$-phenylalkyl, $C_8$-$C_{14}$-phenylalkenyl, $C_8$-$C_{14}$-phenylalkynyl, $C_2$-$C_8$-alkoxyalkyl, $C_3$-$C_{12}$-alkoxyalkoxyalkyl, $C_4$-$C_{16}$-alkoxyalkoxyalkoxyalkyl, $C_1$-$C_8$-alkyl which is substituted by $C_7$-$C_{10}$-alkylaryl, $C_8$-$C_{14}$-dialkylphenyl, $C_9$-$C_{14}$-trialkylaryl, phenoxy, phenoxyphenyl, $C_3$-$C_8$-cycloalkyl, $C_4$-$C_{12}$-alkylcycloalkyl, $C_4$-$C_8$-cycloalkenyl or $C_5$-$C_{12}$-alkylcycloalkenyl, $C_4$-$C_{12}$-alkylcycloalkyl, $C_9$-$C_{14}$-phenylcycloalkyl, $C_9$-$C_{14}$-phenoxycycloalkyl, $C_7$-$C_{14}$-alkylphenyl, $C_7$-$C_{14}$-alkoxyphenyl, $C_8$-$C_{16}$-dialkylphenyl, $C_7$-$C_{14}$-haloalkylphenyl, halophenyl, dihalophenyl, $C_7$-$C_{14}$-alkylhalophenyl, $C_8$-$C_{14}$-dialkylhalophenyl, phenoxyphenyl or $C_{13}$-$C_{20}$-phenoxyphenylalkyl, or is $C_1$-$C_8$-alkyl which is substituted by a saturated or unsaturated 5-membered or 6-

membered heterocyclic structure which has one or two identical or different hetero atoms from the group consisting of oxygen and nitrogen and is unsubstituted or substituted by one, two or three identical or different substituents from the group consisting of $C_1$-$C_8$--alkyl, phenyl, $C_7$-$C_{14}$-phenylalkyl, halophenyl, $C_7$-$C_{14}$-alkylphenyl, $C_8$-$C_{16}$-dialkylphenyl, $C_9$-$C_{16}$-trialkylaryl or an unsubstituted or $C_1$-$C_4$-alkylsubstituted $C_3$-$C_8$-spirocycloalkyl,

$R^2$ is hydrogen, $C_1$-$C_8$-alkyl, $C_1$-$C_8$-haloalkyl, which is unsubstituted or substituted by phenyl or halophenyl; $C_1$-$C_8$-alkoxy, $C_1$-$C_8$-haloalkoxy, $C_2$-$C_8$-alkenyloxy, $C_2$-$C_8$-haloalkenyloxy, $C_3$-$C_{12}$-alkoxyalkenyloxy, $C_2$-$C_8$-alkynyloxy, $C_2$-$C_8$-haloalkynyloxy, $C_3$-$C_{12}$-alkoxyalkynyloxy, $C_8$-$C_{16}$-phenylalkynyloxy, $C_1$-$C_8$-alkylthio, $C_1$-$C_8$-haloalkylthio, $C_2$-$C_{12}$-alkoxyalkylthio, formyl, cyano, $C_7$-$C_{14}$-phenoxyalkoxy, $C_7$-$C_{14}$-halophenoxyalkoxy, $C_7$-$C_{14}$-phenoxyalkoxy, $C_7$-$C_{14}$-halophenoxyhaloalkoxy, $C_8$-$C_{16}$-alkoxyphenoxyalkoxy, $C_8$-$C_{16}$-phenoxyalkoxyalkoxy, $C_7$-$C_{16}$-phenoxyalkenyloxy, $C_8$-$C_{16}$-halophenoxyalkenyl, $C_8$-$C_{16}$-phenoxyhaloalkenyloxy, $C_8$-$C_{16}$-halophenoxyhaloalkenyloxy, $C_9$-$C_{16}$-alkoxyphenoxyalkenyloxy, $C_9$-$C_{16}$-phenoxyalkoxyalkenyloxy, $C_8$-$C_{14}$-phenoxyalkynyloxy, $C_8$-$C_{14}$-halophenoxyalkynyloxy, $C_8$-$C_{14}$-phenoxyhaloalkynyloxy, $C_8$-$C_{14}$-halophenoxyhaloalkynyloxy, $C_9$-$C_{16}$-alkoxyphenoxyalkynyloxy, $C_9$-$C_{16}$-phenoxyalkoxyalkynyloxy, $C_8$-$C_{14}$-phenylalkynyloxy, $C_{14}$-$C_{20}$-phenoxyphenylalkynyloxy, carboxyl, $C_2$-$C_8$-alkoxycarbonyl, $C_2$-$C_8$-alkylcarbonyloxy, $C_3$-$C_{12}$-alkenyloxycarbonyl, $C_3$-$C_{12}$-alkenylcarbonyloxy, $C_3$-$C_{12}$-alkynyloxycarbonyl, $C_3$-$C_{12}$-alkynylcarbonyloxy, phenoxycarbonyl, phenylcarbonyloxy, $C_3$-$C_{12}$-alkoxycarbonylalkoxy, phenoxy, $C_7$-$C_{14}$-alkylphenoxy, halophenoxy, dihalophenoxy, trihalophenoxy, $C_7$-$C_{14}$-haloalkylphenoxy, $C_7$-$C_{14}$-alkoxyphenoxy, phenoxyphenoxy, $C_8$-$C_{14}$-alkylenedioxyphenoxy, phenylthio, $C_7$-$C_{14}$-alkylphenylthio, halophenylthio, dihalophenylthio, trihalophenylthio, $C_7$-$C_{14}$-haloalkylphenylthio, $C_7$-$C_{14}$-alkoxyphenylthio, phenoxyphenylthio, $C_8$-$C_{14}$-alkylenedioxyphenylthio, phenyl, phenoxyphenyl, $C_7$-$C_{14}$-phenylalkyl, $C_{13}$-$C_{20}$-phenoxyphenylalkyl, $C_8$-$C_{14}$-alkylenedioxyphenylalkyloxy or a radical selected from

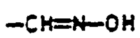 ; 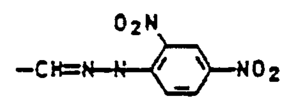 ; 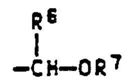 ; 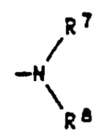 ;

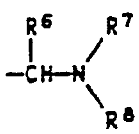 ; 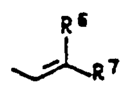 ; 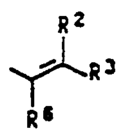 ; 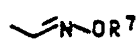 and 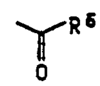 .

$R^3$ is hydrogen, $C_1$-$C_8$-alkyl or $C_1$-$C_2$-haloalkyl,

$R^4$ and $R^5$ are each hydrogen, fluorine, chlorine, $C_1$-$C_3$-alkyl, $C_1$-$C_3$-alkoxy or $C_1$-$C_3$-haloalkoxy,

$R^6$ is hydrogen, $C_1$-$C_8$-alkyl, $C_2$-$C_8$-alkenyl, $C_2$-$C_8$-alkynyl, cyano, phenyl, halophenyl, $C_7$-$C_{14}$-alkylphenyl or $C_7$-$C_{14}$-alkoxyphenyl,

$R^7$ is hydrogen, $C_1$-$C_8$-alkyl, $C_1$-$C_8$-haloalkyl, $C_2$-$C_8$-alkoxyalkyl, $C_2$-$C_8$-alkenyl, $C_2$-$C_8$-alkynyl, aryl, halophenyl, dihalophenyl, trihalophenyl, cyanophenyl, $C_7$-$C_{14}$-alkylphenyl, $C_7$-$C_{14}$-alkoxyphenyl, $C_7$-$C_{14}$-haloalkylphenyl, $C_8$-$C_{16}$-alkenylphenyl, $C_8$-$C_{16}$-alkynylphenyl, phenoxyphenyl, $C_8$-$C_{16}$-methylenedioxyphenyl, $C_2$-$C_8$-alkylcarbonyl, $C_2$-$C_8$-haloalkylcarbonyl, $C_2$-$C_8$-alkoxycarbonyl, $C_5$-$C_{12}$-alkylcyclopropylcarbonyl, $C_7$-$C_{14}$-alkenyl- and -alkylcyclopropylcarbonyl, $C_6$-$C_{14}$-haloalkyl- and -alkylcyclopropylcarbonyl, $C_7$-$C_{14}$-haloalkenyl- and -alkylcyclopropylcarbonyl, $C_8$-$C_{16}$-halo- and -phenylalkylcarbonyl, $C_8$-$C_{16}$-halo- and -halophenylalkylcarbonyl, benzoyl, halobenzoyl, $C_8$-$C_{14}$-alkylbenzoyl, $C_8$-$C_{14}$-alkoxybenzoyl, $C_8$-$C_{14}$-haloalkylbenzoyl, $C_8$-$C_{14}$-haloalkoxybenzoyl, $C_8$-$C_{14}$-phenylalkylcarbonyl, $C_8$-$C_{14}$-halophenylalkylcarbonyl, $C_9$-$C_{16}$-alkylphenylalkylcarbonyl, $C_9$-$C_{16}$-alkoxyphenylalkylcarbonyl, $C_9$-$C_{16}$-haloalkylphenylalkylcarbonyl, $C_9$-$C_{16}$-haloalkoxyphenylalkylcarbonyl, $C_9$-$C_{16}$-phenylalkenylcarbonyl, $C_9$-$C_{16}$-halophenylalkenylcarbonyl, $C_{10}$-$C_{16}$-alkylphenylalkenylcarbonyl, $C_{10}$-$C_{16}$-alkoxyphenylalkenylcarbonyl, $C_{10}$-$C_{16}$-haloalkylphenylalkenylcarbonyl, $C_{10}$-$C_{16}$-haloalkoxyphenylalkenylcarbonyl, $C_9$-$C_{16}$-phenylalkynylcarbonyl, $C_9$-$C_{16}$-halophenylalkynylcarbonyl, $C_{10}$-$C_{16}$-alkylphenylalkynylcarbonyl, $C_{10}$-$C_{16}$-alkoxyphenylalkynylcarbonyl, $C_{10}$-$C_{16}$-haloalkylphenylalkynylcarbonyl, $C_{10}$-$C_{16}$-haloalkoxyphenylalkynylcarbonyl, $C_2$-$C_8$-alkylaminocarbonyl, $C_2$-$C_8$-haloalkylaminocarbonyl, $C_3$-$C_{10}$-alkylalkylaminocarbonyl, $C_3$-$C_{10}$-alkoxyalkylaminocarbonyl, $C_3$-$C_{10}$-haloalkylalkylaminocarbonyl, $C_3$-$C_{10}$-haloalkoxyalkylaminocarbonyl, $C_3$-$C_{10}$-dialkylaminocarbonyl, $C_3$-$C_8$-halodialkylaminocarbonyl, $C_4$-$C_{12}$-alkyldialkylaminocarbonyl, $C_4$-$C_{12}$-alkoxydialkylaminocarbonyl, $C_5$-$C_{20}$-alkyl- and -alkoxyaminocarbonyl, $C_4$-$C_{12}$-haloalkyldialkylaminocarbonyl, $C_4$-$C_{12}$-haloalkoxydialkylaminocarbonyl, phenylaminocarbonyl, halophenylaminocarbonyl, dihalophenylaminocarbonyl, trihalophenylaminocarbonyl, $C_8$-$C_{14}$-alkylphenylaminocarbonyl, $C_8$-$C_{14}$-alkoxyphenylaminocarbonyl, $C_8$-$C_{14}$-haloal-

koxyphenylaminocarbonyl or an unsubstituted or $C_1$-$C_4$-alkyl-substituted isoxazole,
$R^8$ is hydrogen or $C_1$-$C_8$-alkyl and
X is halogen,
with the proviso that, in formula Ia, $R^1$ is not phenyl when $R^2$, $R^3$, $R^4$ and $R^5$ are simultaneously hydrogen, and, in formula Ib, $R^2$, $R^3$, $R^4$ or $R^5$ are not simultaneously hydrogen when $R^1$ is $C_4$-$C_8$-alkyl, phenyl, tolyl or methoxyphenyl, and $R^1$ is not octyl when $R^2$ and $R^3$ are each hydrogen and $R^4$ or $R^5$ is methyl, methoxy or chlorine, and with the further proviso that, in formula Ib, $R^1$ is not n-butyl when $R^2$ is methyl and $R^3$, $R^4$ and $R^5$ are each hydrogen, in addition to conventional carriers therefor.

5. A pesticide as claimed in claim 3 containing 0.1 to 95% by weight of a pyran derivative Ia, Ib or Ic.

6. A process for controlling pests, wherein the pests or the areas or spaces to be kept free of pests are treated with an effective amount of a pesticide as claimed in either of claims 3 and 4.

## Revendications

### Revendications pour les Etats Contractants : AT-SE

1. Dérivés de pyrane des formules générales Ia, Ib ou Ic

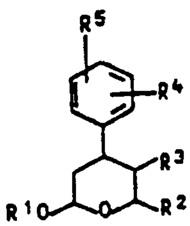
(Ia)

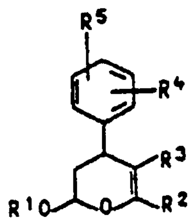
(Ib)

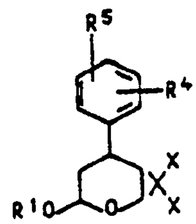
(Ic)

dans lesquelles les substituants ont les significations suivantes :
$R^1$ alkyle en $C_1$-$C_{20}$, alcényle en $C_2$-$C_{20}$, alcynyle en $C_2$-$C_{20}$, cycloalkyle en $C_3$-$C_{20}$, aryle, biphénylyle, arylalkyle en $C_7$-$C_{30}$, arylalcényle en $C_8$-$C_{30}$, arylalcynyle en $C_8$-$C_{30}$, alcoxy-alkyle en $C_2$-$C_{20}$, alcoxy-alcoxy-alkyle en $C_3$-$C_{20}$, alcoxy-alcoxy-alcoxy-alkyle en $C_4$-$C_{30}$, alkyle en $C_1$-$C_{20}$ substitué par alkyle-aryle en $C_7$-$C_{20}$, dialkyle-aryle en $C_8$-$C_{20}$, trialkyl-aryle en $C_9$-$C_{20}$, aryloxy, aryloxy-aryle, cycloalkyle en $C_3$-$C_{20}$, alkyl-cycloalkyle en $C_4$-$C_{20}$, cycloalcényle en $C_4$-$C_{20}$ ou alkylcycloalcényle en $C_5$-$C_{20}$, alkyl-cycloalkyle en $C_4$-$C_{20}$, arylcycloalkyle en $C_9$-$C_{20}$, aryloxycycloalkyle en $C_9$-$C_{30}$, alkyl-aryle en $C_7$-$C_{20}$, alcoxy-aryle en $C_7$-$C_{20}$, dialkyl-aryle en $C_8$-$C_{20}$, halogénalkyl-aryle en $C_7$-$C_{20}$, halogénaryle, dihalogénaryle, alkyl-halogénaryle en $C_7$-$C_{20}$, dialkyl-halogénaryle en $C_8$-$C_{20}$, aryloxy-aryle, aryloxy-arylalkyle en $C_{13}$-$C_{30}$, ou alkyle en $C_1$-$C_{20}$ substitué par un hétérocycle saturé ou insaturé, de 5-, 6-, 7- ou 8 chaînons, à un, deux ou trois hétéroatomes identiques ou différents du groupe constitué par oxygène, soufre et azote, qui est éventuellement substitué par un, deux ou trois substituants identiques ou différents du groupe constitué par alkyle en $C_1$-$C_{20}$, aryle, arylalkyle en $C_7$-$C_{20}$, halogénaryle, alkylaryle en $C_7$-$C_{20}$, dialkyl-aryle en $C_8$-$C_{20}$, trialkyl-aryle en $C_9$-$C_{20}$, ou par un spiro-cycloalkyle en $C_3$-$C_{20}$ éventuellement substitué par alkyle en $C_1$-$C_8$
$R^2$, hydrogène, alkyle en $C_1$-$C_{20}$, halogénoalkyle en $C_1$-$C_{20}$, éventuellement substitués par phényle ou halogénophényle ; halogèn-phényl-halogénalkyle en $C_7$-$C_{20}$, alcoxy en $C_1$-$C_{20}$, halogénalcoxy en $C_1$-$C_{20}$, alcényloxy en $C_2$-$C_{20}$, halogénalcényloxy en $C_2$-$C_{20}$, alkoxy-alcényloxy en $C_3$-$C_{20}$, alcynyloxy en $C_2$-$C_{20}$, halogénalcynyloxy en $C_2$-$C_{20}$, alcoxy -alcynyloxy en $C_3$-$C_{20}$, phényl-alcynyloxy en $C_8$-$C_{20}$, alkylthio en $C_1$-$C_{20}$, halogénalkylthio en $C_1$-$C_{20}$, alcoxyalkylthio en $C_2$-$C_{20}$, formyle, cyano, phénoxy-alcoxy en $C_7$-$C_{20}$, halogénphenoxy-alcoxy en $C_7$-$C_{20}$, phénoxy-halogénalcoxy en $C_7$-$C_{20}$, halogénphénoxyhalogénalcoxy en $C_7$-$C_{20}$, alcoxy-phénoxyalcoxy en $C_8$-$C_{20}$, phénoxy-alcoxyalcoxy en $C_8$-$C_{20}$, phénoxyalcényloxy en $C_7$-$C_{20}$, halogénphénoxy-alcynyle en $C_8$-$C_{20}$, phénoxy-halogénalcényloxy en $C_8$-$C_{20}$, halogénphénoxy-halogénalcényloxy en $C_8$-$C_{20}$, alcoxyphénoxy-alcényloxy en $C_9$-$C_{20}$, phénoxyalcoxyalcényloxy en $C_9$-$C_{20}$, phénoxy-alcynyloxy en $C_8$-$C_{20}$, halogénphénoxy-alcynyle en $C_8$-$C_{20}$, phénoxyhalogénalcynyloxy en $C_8$-$C_{20}$, halogénphénoxy-halogénalcynyloxy en $C_8$-$C_{20}$, alcoxyphénoxy-alcynyloxy en $C_9$-$C_{20}$, phénoxy-alcoxyalcynyloxy en $C_9$-$C_{20}$, phénylalcynyloxy en $C_{8-20}$, phénoxyphénylalcynyloxy en $C_{14}$-$C_{30}$, carboxy, alcoxycarbonyle en $C_2$-$C_{20}$, alkylcarbonyloxy en $C_2$-$C_{20}$, alcényloxycarbonyle en $C_3$-$C_{20}$, alcénylcarbonyloxy en $C_3$-$C_{20}$, alcynyloxy-carbonyle en $C_3$-$C_{20}$, alcynylcarbonyloxy en $C_3$-$C_{20}$, phénoxycarbonyle, phényl-carbonyloxy, alcoxy-carbonyl-alcoxy en $C_3$-$C_{20}$, phénoxy, alkylphénoxy en $C_7$-$C_{20}$, halogénophénoxy, dihalogénophénoxy,

trihalogénophénoxy, halogénalkyl-phénoxy en $C_7$-$C_{20}$, alcoxyphénoxy en $C_7$-$C_{20}$, phénoxy-phénoxy, alkylendioxy-phénoxy en $C_8$-$C_{12}$, phénylthio, alkylphénylthio en $C_7$-$C_{20}$, halogèn-phénlylthio, dihalogèn-phénylthio, trihalogén-phénylthio, halogénalkyl-phénylthio en $C_7$-$C_{20}$, alcoxyphénylthio en $C_7$-$C_{20}$, phénoxy-phénylthio, alkylendioxyphénylthio en $C_8$-$C_{12}$, phényle, phénoxy-phényle, phénylalkyle en $C_7$-$C_{20}$, phénoxy-phénylalkyle en $C_{13}$-$C_{30}$, alkylendioxy-phénylalcoxy en $C_8$-$C_{12}$ ou un reste choisi parmi

$-CH{=}N-OH$ ; 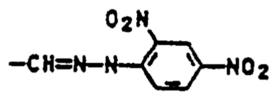 ; 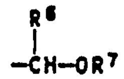 ; 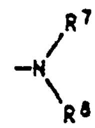 ;

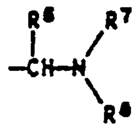 ; 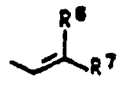 ; 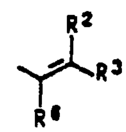 ; 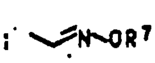 ou 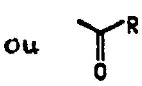

$R^3$, hydrogène, alkyle en $C_1$-$C_{20}$ ou halogénoalkyle en $C_1$-$C_3$

$R^4$, $R^5$, hydrogène, fluor, chlore, alkyle en $C_1$-$C_3$, alcoxy en $C_1$-$C_3$ ou halogénalcoxy en $C_1$-$C_3$,

$R^6$, hydrogene, alkyle en $C_1$-$C_{20}$ alcényle en $C_2$-$C_{20}$, alcynyle en $C_2$-$C_{20}$, cyano, phényle, halogénophényle, alkylphényle en $C_7$-$C_{20}$ ou alcoxyphényle en $C_7$-$C_{20}$,

$R^7$, hydrogène, alkyle en $C_1$-$C_{20}$, halogénalkyle en $C_1$-$C_{20}$, alcoxyalkyle en $C_2$-$C_{20}$, alcényle en $C_2$-$C_{20}$, alcynyle en $C_2$-$C_{20}$, aryle, halogénophényle, dihalogénophényle, trihalogénophényle, cyanophényle, alkyl-phényle en $C_7$-$C_{20}$, alcoxyphényle en $C_7$-$C_{20}$-halogénalkyl-phényle en $C_7$-$C_{20}$, alcénylphényle en $C_8$-$C_{20}$, alcynylphényle en $C_8$-$C_{20}$, phénoxyphényle, méthylendioxy-phényle en $C_8$-$C_{12}$, alkylcarbonyle en $C_2$-$C_{20}$, halogénalkyl-carbonyle en $C_2$-$C_{20}$, alcoxycarbonyle en $C_2$-$C_{20}$, alkylcyclopropyl-carbonyle en $C_5$-$C_{20}$, alcényle et alkylcyclopropyl-carbonyle en $C_7$-$C_{20}$, halogénoalkyle et alkylcyclopropyl-carbonyle en $C_8$-$C_{20}$, halogénalcényle et alkylcyclopropyl-carbonyle en $C_7$-$C_{20}$, halogèno et phényl-alkylcarbonyle en $C_8$-$C_{20}$ et halogèno et -halogénophénylalkylcarbonyle en $C_8$-$C_{20}$, benzoyle, halogénobenzoyle, alkylbenzoyle en $C_8$-$C_{20}$, alcoxybenzoyle en $C_8$-$C_{20}$, halogénalkylebenzoyle en $C_8$-$C_{20}$, halogénalcoxy-benzoyle en $C_8$-$C_{20}$, phénylalkylcarbonyle en $C_8$-$C_{20}$, halogénophénylalkylcarbonyle en $C_8$-$C_{20}$, alkylphénylalkylcarbonyle en $C_9$-$C_{20}$ alcoxyphénylalkylcarbonyle en $C_9$-$C_{20}$, halogénalkylphénylalkylcarbonyle en $C_9$-$C_{20}$, halogénalcoxyphénylalkylcarbonyle en $C_9$-$C_{20}$, phénylalcénylcarbonyle en $C_9$-$C_{20}$, halogénophénylalcényle-carbonyle en $C_9$-$C_{20}$, alkylphénylalcénylcarbonyle en $C_{10}$-$C_{20}$, alcoxyphénylalcénylcarbonyle en $C_{10}$-$C_{20}$, halogénalkylphénylalcényl-carbonyle en $C_{10}$-$C_{20}$, halogénalcoxyphénylalcénylcarbonyle en $C_{10}$-$C_{20}$, phénylalcynylcarbonyle en $C_9$-$C_{20}$, halogénophénylalcynylcarbonyle en $C_9$-$C_{20}$, alkylphénylalcynylcarbonyle en $C_{10}$-$C_{20}$, alcoxyphénylalcynyl-carbonyle en $C_{10}$-$C_{20}$, halogénalkylphénylalcynylcarbonyle en $C_{10}$-$C_{20}$, halogénalcoxyphénylalcynylcarbonyle en $C_{10}$-$C_{20}$, alkylaminocarbonyle en $C_2$-$C_{20}$, halogénalkylaminocarbonyle en $C_2$-$C_{20}$, alcoxyalkylamino-carbonyle en $C_3$-$C_{20}$, halogénalkylalkylaminocarbonyle en $C_3$-$C_{20}$, halogénalcoxyalkylaminocarbonyle en $C_3$-$C_{20}$, dialkylaminocarbonyle en $C_3$-$C_{20}$, halogéndialkylaminocarbonyle en $C_3$-$C_{20}$, alcoxydialkylaminocarbonyle en $C_4$-$C_{20}$, alkyle et alcoxy-aminocarbonyle en $C_5$-$C_{20}$, halogénalkyldialkylaminocarbonyle en $C_4$-$C_{20}$ halogénalcoxydialkylaminocarbonyle en $C_4$-$C_{20}$, phénylaminocarbonyle, halogénophénylaminocarbonyle, dihalogénophénylaminocarbonyle, trihalogénophénylaminocarbonyle, alkylphénylaminocarbonyle en $C_8$-C20, alcoxyphénylaminocarbonyle en $C_8$-$C_{20}$, halogénalcoxyphénylaminocarbonyle en $C_8$-$C_{20}$ ou isoxazole éventuellement substitué par alkyle en $C_1$-$C_8$,

$R^8$, hydrogène ou alkyle en $C_1$-$C_{20}$ et

X, halogène

sous réserve que, dans la formule $I_a$, $R^1$ ne représente pas phényle quand $R^2$, $R^3$, $R^4$, $R^5$ sont mis simultanément pour hydrogène et que dans la formule $I_b$, $R^2$, $R^3$, $R^4$, $R^5$ ne représentent pas simultanément hydrogène quand $R^1$ est mis pour alkyle en $C_4$-$C_8$, phényle, tolyle ou méthoxyphényle, ainsi que $R^1$ ne représente pas octyle quand $R^2$ et $R^3$ sont mis pour hydrogène et $R^4$ ou $R^5$ pour méthyle, méthoxy ou chlore, et sous réserve encore que, dans la formule $I_b$, $R^1$ ne représente pas n-butyle quand $R^2$ est mis pour méthyle et $R^3$, $R^4$ et $R^5$ pour hydrogène.

2. Dérivés de pyranne des formules générales $I_a$, Ib et $I_c$ selon la revendication 1, dans lesquelles les substituants ont les significations suivantes,

$R^1$, alkyle en $C_1$-$C_{12}$, alcényle en $C_2$-$C_{14}$, alcynyle en $C_2$-$C_8$, cycloalkyle en $C_3$-$C_8$, phényle, naphtyle, biphénylyle, phénylalkyle en $C_7$-$C_{14}$, phénylalcényle en $C_8$-$C_{14}$, phénylalcynyle en $C_8$-$C_{14}$, alcoxy-alkyle en $C_2$-$C_8$, alcoxy-alcoxy-alkyle en $C_3$-$C_{12}$, alcoxyalcoxy-alcoxy-alkyle en $C_4$-$C_{18}$, alkyle en $C_1$-$C_8$ substitué par alkylaryle en $C_7$-$C_{10}$, dialkylephényle en $C_8$-$C_{14}$, trialkyl-aryle en $C_9$-$C_{14}$, phénoxy, phénoxy-phényle, cycloalkyle

en $C_3$-$C_8$, alkyl-cycloalkyle en $C_4$-$C_{12}$, cycloalcényle en $C_4$-$C_8$ ou alkyle-cycloalcényle en $C_5$-$C_{12}$, alkylecycloalkyle en $C_4$-$C_{12}$, phényl-cycloalkyle en $C_9$-$C_{14}$, phénoxy-cycloalkyle en $C_9$-$C_{14}$, alkyle-phényle en $C_7$-$C_{14}$, alcoxy-phényle en $C_7$-$C_{14}$, dialkylphényle en $C_8$-$C_{16}$, halogénalkyle-phényle en $C_7$-$C_{14}$, halogénophényle, dihalogénophényle, alkylhalogénophényle en $C_7$-$C_{14}$, dialkyl-halogénophényle en $C_8$-$C_{14}$, phénoxy-phényle, phénoxy-phénylalkyle en $C_{13}$-$C_{20}$.ou alkyle en $C_1$-$C_8$ substitué par un hétérocycle saturé ou insaturé, de 5 à 6 chaînons à un ou deux hétéroatomes identiques ou différents du groupe constitué par oxygène et azote, qui est éventuellement substitué par un deux ou trois substituants identiques ou différents du groupe constitué par alkyle en $C_1$-$C_8$, phényle, phénylalkyle en $C_7$-$C_{14}$, halogénphényle, alkylphényle en $C_7$-$C_{14}$, dialkyle-phényle en $C_8$-$C_{16}$ trialkyl-aryle en $C_9$-$C_{16}$ ou par un spirocycloalkyle en $C_3$-$C_8$ éventuellement substitué par alkyle en $C_1$-$C_4$,

$R_2$, hydrogène, alkyle en $C_1$-$C_8$, halogénoalkyle en $C_1$-$C_8$, éventuellement substitués par phényle ou halogénophényle, alcoxy en $C_1$-$C_8$, halogènalcoxy en $C_1$-$C_8$, alcényloxy en $C_2$-$C_8$, halogénalcényloxy en $C_2$-$C_8$, alcoxy-alcényloxy en $C_3$-$C_{12}$, alcynyloxy en $C_2$-$C_8$, halogénalcynyloxy en $C_2$-$C_8$ alcoxyalcynyloxy en $C_3$-$C_{12}$, phényl-alcynyloxy en $C_8$-$C_{16}$, alkylthio en $C_1$-$C_8$, halogénalkylthio en $C_1$-$C_8$, alcoxy-alkylthio en $C_2$-$C_{12}$, formyle, cyano, phénoxy-alcoxy en $C_7$-$C_{14}$, halogénphénoxy-alcoxy en $C_7$-$C_{14}$, phénoxy-halogénalcoxy en $C_7$-$C_{14}$, halogénphénoxy-halogénalcoxy en $C_7$-$C_{14}$, alcoxyphénoxyalcoxy en $C_8$-$C_{16}$, phénoxy-alcoxyalcoxy en $C_8$-$C_{16}$, phénoxy-alcényloxy en $C_7$-$C_{16}$, halogénphénoxy-alcényle en $C_8$-$C_{16}$, phénoxy-halogénalcényloxy en $C_8$-$C_{16}$, halogénphénoxy-halogénalcényloxy en $C_8$-$C_{16}$, alcoxyphénoxy-alcényloxy en $C_9$-$C_{16}$, phénoxyalcoxyalcényloxy en $C_9$-$C_{16}$, phénoxy-alcynyloxy en $C_8$-$C_{14}$, halogénphénoxy-alcynyloxy en $C_8$-$C_{14}$, phénoxy-halogénalcynyloxy en $C_8$-$C_{14}$, halogénphénoxy-halogénalcynyloxy en $C_8$-$C_{14}$, alcoxyphénoxy-alcynyloxy en $C_9$-$C_{16}$, phénoxyalcoxyalcynyloxy en $C_9$-$C_{16}$, phénylalcynyloxy en $C_8$-$C_{14}$, phénoxyphénylalcynyloxy en $C_{14}$-$C_{20}$, carboxy, alcoxycarbonyle en $C_2$-$C_8$, alkyl-carbonyloxy en $C_2$-$C_8$, alcénylocxycarbonyle en $C_3$-$C_{12}$, alcénylcarbonyloxy en $C_3$-$C_{12}$, alcinyloxycarbonyle en $C_3$-$C_{12}$, alcynylcarbonyloxy en $C_3$-$C_{12}$, phénoxycarbonyle, phénylcarbonyloxy, alcoxycarbonyl-alcoxy en $C_3$-$C_{12}$, phénoxy, alkylphénoxy en $C_7$-$C_{14}$, halogénophénoxy, dihalogénophénoxy, trihalogénophénoxy, halogénalkyl-phénoxy en $C_7$-$C_{14}$, alcoxyphénoxy en $C_7$-$C_{14}$, phénoxy-phénoxy, alkylendioxy-phénoxy en $C_8$-$C_{14}$, phénylthio, alkylphénylthio en $C_7$-$C_{14}$, halogén-phénylthio, dihalogén-phénylthio, trihalogén-phénylthio, halogénalkylphénylthio en $C_7$-$C_{14}$, alcoxyphénylthio en $C_7$-$C_{14}$, phénoxy-phénylthio, alkylendioxyphénylthio en $C_8$-$C_{14}$, phényle, phénoxy-phényle, phénylalkyle en $C_7$-$C_{14}$, phénoxy-phénylalkyle en $C_{13}$-$C_{20}$, alkylendioxy-phénylalkyloxy en $C_8$-$C_{14}$ ou un reste choisi parmi :

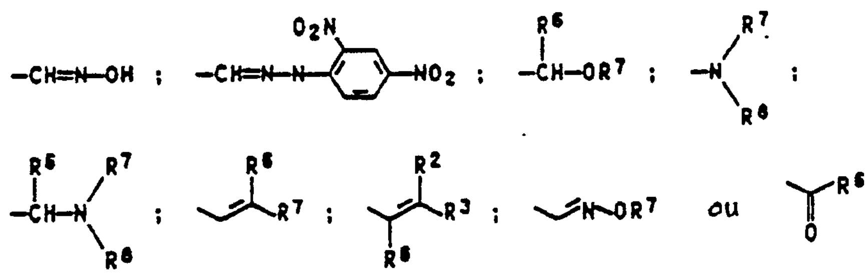

$R^3$, hydrogène, alkyle en $C_1$-$C_8$ ou halogénalkyle en $C_1$-$C_2$

$R^4$, $R^5$, hydrogène, fluor, chlore, alkyle en $C_1$-$C_3$, alcoxy en $C_1$-$C_3$ ou halogénalcoxy en $C_1$-$C_3$,

$R^6$, hydrogène, alkyle en $C_1$-$C_8$, alcényle en $C_2$-$C_8$, alcynyle en $C_2$-$C_8$, cyano, phényle, halogénophényle, alkylphényle en $C_7$-$C_{14}$ ou alcoxyphényle en $C_7$-$C_{14}$

$R^7$, hydrogène, alkyle en $C_1$-$C_8$, halogénalkyle en $C_1$-$C_8$, alcoxy-alkyle en $C_2$-$C_8$, alcényle en $C_2$-$C_8$, alcynyle en $C_2$-$C_8$, aryle, halogénophényle, dihalogénophényle, trihalogénophényle, cyanophényle, alkylphényle en $C_7$-$C_{14}$, alcoxy-phényle en $C_7$-$C_{14}$, halogénalkyl-phényle en $C_7$-$C_{14}$, alcénylphényle en $C_8$-$C_{16}$, alcynylphényle en $C_8$-$C_{16}$, phénoxyphényle, méthylendioxy-phényle en $C_8$-$C_{16}$, alkylcarbonyle en $C_2$-$C_8$, halogénalkylcarbonyle en $C_2$-$C_8$, alcoxycarbonyle en $C_2$-$C_8$, alcylcyclopropyle-carbonyle en $C_5$-$C_{12}$, alcényle en $C_7$-$C_{14}$ et alkylcyclopropyle-carbonyle, halogénalkyle et alkylcyclopropyl-carbonyle en $C_6$-$C_{14}$, halogénalcényl- et alcylcyclopropyl-carbonyle en $C_7$-$C_{14}$, halogèno et phényl-alkylcarbonyle en $C_8$-$C_{16}$, halogèno et halogènphénylalcylcarbonyle en $C_8$-$C_{16}$, benzoyle, halogénbenzoyle, alkylbenzoyle en $C_8$-$C_{14}$, alcoxybenzoyle en $C_8$-$C_{14}$, halogénalkylbenzoyle en $C_8$-$C_{14}$, halogén-alcoxy-benzoyle en $C_8$-$C_{14}$, phénylalkylcarbonyle en $C_8$-$C_{14}$ halogénphénylalkylcarbonyle en $C_8$-$C_{14}$, alkylphénylalkylcarbonyle en $C_9$-$C_{16}$, alcoxyphényl-alkylcarbonyle en $C_9$-$C_{16}$, halogénalkylphénylalkylcarbonyle en $C_9$-$C_{16}$, halogénalcoxyphénylalkylcarbonyle en $C_9$-$C_{16}$, phénylalcénylcarbonyle en $C_9$-$C_{16}$, halogènophénylalcénylcarbonyle en $C_9$-$C_{16}$, alkylphénylalcénylcarbonyle en $C_{10}$-$C_{16}$, alcoxyphénylalcénylcarbonyle en $C_{10}$-$C_{16}$, halogènealkylphénylalcénylecarbonyle en

$C_{10}$-$C_{16}$, halogènalcoxyphénylalcényl-carbonyle en $C_{10}$-$C_{16}$, phényl-alcynylcarbonyle en $C_9$-$C_{16}$, halogénophénylalcynyl-carbonyle en $C_9$-$C_{16}$, alkylphénylalcynylcarbonyle en $C_{10}$-$C_{16}$, alcoxyphényl-alcynylcarbonyle en $C_{10}$-$C_{16}$, halogénalkylphénylcarbonyle en $C_{10}$-$C_{16}$, halogénalkoxyphénylalcynylcarbonyle en $C_{10}$-$C_{16}$, alkylaminocarbonyle en $C_2$-$C_8$, halogénalkylaminocarbonyle en $C_2$-$C_8$, alkylalkylaminocarbonyle en $C_3$-$C_{10}$, alcoxyalkylaminocarbonyle en $C_3$-$C_{10}$, halogénalkylalkylaminocarbonyle en $C_3$-$C_{10}$, halogénalcoxyalkylaminocarbonyle en $C_3$-$C_{10}$, dialkyl-aminocarbonyle en $C_3$-$C_{10}$, halogénodialkylaminocarbonyle en $C_3$-$C_8$, alkyldialkylaminocarbonyle en $C_4$-$C_{12}$, alcoxydialkylaminocarbonyle en $C_4$-$C_{12}$, alkyl et alcoxy-aminocarbonyle en $C_5$-$C_{20}$, halogénalkyldialkyl-aminocarbonyle en $C_4$-$C_{12}$, halogénalcoxydialkylaminocarbonyle en $C_4$-$C_{12}$, phényl-aminocarbonyle, halogénophénylaminocarbonyle, dihalogénophénylaminocarbonyle, trihalogénophénylaminocarbonyle, alkylphélnylaminocarbonyle en $C_8$-$C_{14}$, alcoxyphénylaminocarbonyle en $C_8$-$C_{14}$, halogénalcoxyphénylaminocarbonyle en $C_8$-$C_{14}$ ou isoxazole éventuellement substitué par alkyle en $C_1$-$C_4$,

$R^8$, hydrogène ou alkyle en $C_1$-$C_8$ et

X, halogène

sous réserve que, dans la formule $I_a$, $R^1$ ne représente pas phényle quand $R^2$, $R^3$, $R^4$, $R^5$ sont mis simultanément pour hydrogène et que, dans la formule $I_b$, $R^2$, $R^3$, $R^4$, $R^5$ ne représentent pas simultanément hydrogène quand $R^1$ est mis pour alkyle en $C_4$-$C_8$, phényle, tolyle ou méthoxyphényle, ainsi que $R^1$ ne représente pas octyle quand $R^2$ et $R^3$ sont mis pour hydrogène et $R_4$ ou $R_5$ pour méthyle, méthoxy ou chlore, et sous réserve encore que, dans la formule $I_b$, $R^1$ ne représente pas n-butyle quand $R^2$ est mis pour méthyle et $R^3$, $R^4$ et $R^5$ pour hydrogène.

3. Procédé de préparation de dérivés de pyrane des formules générales $I_a$, $I_b$ ou $I_c$ selon la revendication 1, caractérisé par le fait qu'on fait réagir de manière connue en soi un éther vynylique de la formule générale II

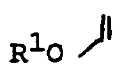

II,

en présence d'un inhibiteur radicalaire et un composé basique, à des températures de 130 à 280 degrés C et des pressions de 1 à 300 bar

a) avec un aldéhyde ou cétone III insaturé en α, β, à l'état monomère

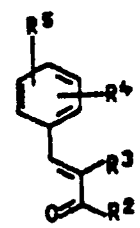

(III)

ou

b) avec un aldéhyde ou cétone (IIIa) insaturé en α, β, à l'état dimère

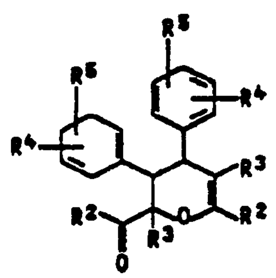

(IIIa)

et le composé $I_b$, éventuellement

c) est hydraté en le composé $I_a$, ou

d) lorsque $R^2$ = H est hydroformylé, ou

e) lorsque $R^2$ et $R^3$ = H, est transformé, avec un composé trihalogénométhane $CHX_3$ en le compose

$I_c$,

4. Pesticide, contenant un dérivé de pyranne $I_a$, $I_b$ ou $I_c$ selon la revendication 1.

5. Pesticide selon la revendication 4 contenant un dérivé de pyranne $I_a$, $I_b$ ou $I_c$ selon la revendication 2, ainsi que les supports usuels pour cette action.

6. Pesticide selon l'une des revendications 4 ou 5, caractérisé par le fait qu'il contient 0,1 à 95% en poids d'un dérivé de pyranne $I_a$, $I_b$ ou $I_c$, selon la revendication 1 ou 2.

7. Procédé de lutte contre les parasites, caractérisé par le fait que l'on traite les parasites et/ou les surfaces et/ou espaces à maintenir exempts de parasites, avec une quantité efficace contre les parasites d'un dérivé de pyranne des formules $I_a$, $I_b$, $C_c$ selon la revendication 1, ou un pesticide selon la revendication 3.

**Revendications pour l'Etat Contractant : ES**

1. Procédé de préparation de dérivés de pyranne des formules générales $I_a$, $I_b$ ou $I_c$

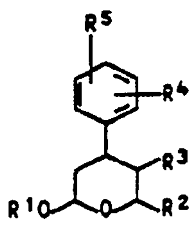

(Ia)

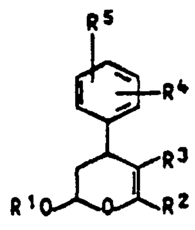

(Ib)

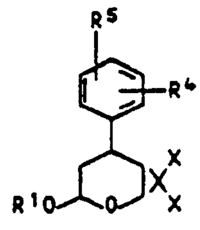

(Ic)

dans lesquelles les substituants ont les significations suivantes :

$R^1$ alkyle en $C_1$-$C_{20}$, alcynyle en $C_2$-$C_{20}$, alcinyle en $C_2$-$C_{20}$, cycloalkyle en $C_3$-$C_{20}$, aryle, biphénylyle, arylalkyle en $C_7$-$C_{30}$, arylalcényle en $C_8$-$C_{30}$, aryl-alcynyle en $C_8$-$C_{30}$, alcoxy-alkyle en $C_2$-$C_{20}$, alcoxy-alcoxy-alkyle en $C_3$-$C_{20}$, alcoxy-alcoxyalcoxy-alkyle en $C_4$-$C_{30}$, alkyle en $C_1$-$C_{20}$ substitué par alkyle-aryle en $C_7$-$C_{20}$, dialkyle-aryle en $C_8$-$C_{20}$, trialkyl-aryle en $C_9$-$C_{20}$, aryloxy, aryloxy-aryle, cycloalkyle en $C_3$-$C_{20}$, alkyl-cycloalkyle en $C_4$-$C_{20}$, cycloalcényle en $C_4$-$C_{20}$ ou alkylcycloalcényle en $C_5$-$C_{20}$, alkyl-cycloalkyle en $C_4$-$C_{20}$, aryl-cycloalkyle en $C_9$-$C_{20}$, aryloxy-cycloalkyle en $C_9$-$C_{30}$, alkyl-aryle en $C_7$-$C_{20}$, alcoxy-aryle en $C_7$-$C_{20}$, dialkyl-aryle en $C_8$-$C_{20}$, halogénalkyl-aryle en $C_7$-$C_{20}$, halogénaryle, dihalogénaryle, alkyl-halogénaryle en $C_7$-$C_{20}$, dialkyl-halogénaryle en $C_8$-$C_{20}$, aryloxy-aryle, aryloxy-arylalkyle en $C_{13}$-$C_{30}$, ou alkyle en $C_1$-$C_{20}$ substitué par un hétérocycle saturé ou insaturé, de 5-, 6-, 7- ou 8 chaînons, à un, deux ou trois hétéroatomes identiques ou différents du groupe constitué par oxygène, soufre et azote, qui est éventuellement substitué par un, deux ou trois substituants identiques ou différents du groupe constitué par alkyle en $C_1$-$C_{20}$, aryle, arylalkyle en $C_7$-$C_{20}$, halogénaryle, alkylaryle en $C_7$-$C_{20}$, dialkyl-aryle en $C_8$-$C_{20}$, trialkyl-aryle en $C_9$-$C_{20}$, ou par un spiro-cycloalkyle en $C_3$-$C_{20}$ éventuellement substitué par alkyle en $C_1$-$C_8$

$R^2$, hydrogène, alkyle en $C_1$-$C_{20}$, halogénoalkyle en $C_1$-$C_{20}$, éventuellement substitués par phényle ou halogénophényle ; halogèn-phényl-halogénalkyle en $C_7$-$C_{20}$, alcoxy en $C_1$-$C_{20}$, halogénalcoxy en $C_1$-$C_{20}$, alcényloxy en $C_2$-$C_{20}$, halogénalcényloxy en $C_2$-$C_{20}$, alkoxy-alcényloxy en $C_3$-$C_{20}$, alcynyloxy en $C_2$-$C_{20}$, halogénalcynyloxy en $C_2$-$C_{20}$, alcoxy-alcynyloxy en $C_3$-$C_{20}$, phényl-alcynyloxy en $C_8$-$C_{20}$, alkylthio en $C_1$-$C_{20}$, halogénalkylthio en $C_1$-$C_{20}$, alcoxy-alkylthio en $C_2$-$C_{20}$, formyle, cyano, phénoxy-alcoxy en $C_7$-$C_{20}$, halogénphenoxy-alcoxy en $C_7$-$C_{20}$, phénoxy-halogénalcoxy en $C_7$-$C_{20}$, halogénphénoxy-halogénalcoxy en $C_7$-$C_{20}$, alcoxy-phénoxyalcoxy en $C_8$-$C_{20}$, phénoxy-alcoxyalcoxy en $C_8$-$C_{20}$, phénoxyalcényloxy en $C_7$-$C_{20}$, halogénphénoxy-alcényle en $C_8$-$C_{20}$, phénoxy-halogénalcényloxy en $C_8$-$C_{20}$, halogénphénoxy-halogénalcényloxy en $C_8$-$C_{20}$, alcoxyphénoxy-alcényloxy en $C_9$-$C_{20}$, phénoxy-alcoxyalcényloxy en $C_9$-$C_{20}$, phénoxy-alcynyloxy en $C_8$-$C_{20}$, halogénphénoxy-alcynyle en $C_8$-$C_{20}$, phénoxy-halogénalcynyloxy en $C_8$-$C_{20}$, halogénphénoxy-halogénalcynyloxy en $C_8$-$C_{20}$, alcoxyphénoxy-alcynyloxy en $C_9$-$C_{20}$, phénylalcynyloxy en $C_8$-$C_{20}$, phénoxyphényl-alcynyloxy en $C_{14}$-$C_{30}$, carboxy, alcoxycarbonyle en $C_2$-$C_{20}$, alkylcarbonyloxy en $C_2$-$C_{20}$, alcényl-oxycarbonyle en $C_3$-$C_{20}$, alcénylcarbonyloxy en $C_3$-$C_{20}$, alcynyloxy-carbonyle en $C_3$-$C_{20}$, alcynylcarbonyloxy en $C_3$-$C_{20}$, phénoxycarbonyle, phényl-carbonyloxy, alcoxycarbonyl-alcoxy en $C_3$-$C_{20}$, phénoxy, alkylphénoxy en $C_7$-$C_{20}$, halogénophénoxy, dihalogénophénoxy, trihalogénophénoxy, halogénalkyl-phénoxy en $C_7$-$C_{20}$, alcoxyphénoxy en $C_7$-$C_{20}$, phénoxy-phénoxy, alkylendioxy-phénoxy en $C_8$-$C_{12}$, phénylthio, alkylphénylthio en $C_7$-$C_{20}$, halogèn-phénlylthio, dihalogèn-phénylthio, trihalogén-phénylthio, halogénalkyl-phénylthio en $C_7$-$C_{20}$, alcoxyphénylthio en $C_7$-$C_{20}$, phénoxy-phénylthio, alkylendioxyphénylthio en $C_8$-$C_{12}$, phényle, phénoxy-phényle, phénylalkyle en $C_7$-$C_{20}$, phénoxy-phénylalkyle en $C_{13}$-$C_{30}$, alkylendioxy-phénylalcoxy en $C_8$-$C_{12}$ ou un reste choisi parmi

$-CH=N-OH$ ; 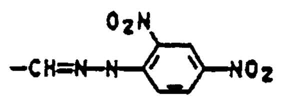 ; 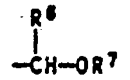 ; 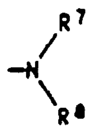 ;

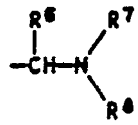 ; 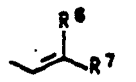 ; 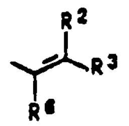 ; 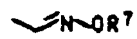 ou 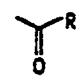 ,

$R^3$, hydrogène, alkyle en $C_1$-$C_{20}$ ou halogénoalkyle en $C_1$-$C_3$
$R^4$, $R^5$, hydrogène, fluor, chlore, alkyle en $C_1$-$C_3$, alcoxy en $C_1$-$C_3$ ou halogénalcoxy en $C_1$-$C_3$,
$R^6$, hydrogene, alkyle en $C_1$-$C_{20}$, alcényle en $C_2$-$C_{20}$, alcynyle en $C_2$-$C_{20}$, cyano, phényle, halogénophényle, alkylphényle en $C_7$-$C_{20}$ ou alcoxyphényle en $C_7$-$C_{20}$,
$R^7$, hydrogène, alkyle en $C_1$-$C_{20}$, halogénalkyle en $C_1$-$C_{20}$, alcoxyalkyle en $C_2$-$C_{20}$, alcényle en $C_2$-$C_{20}$, alcynyle en $C_2$-$C_{20}$, aryle, halogénophényle, dihalogénophényle, trihalogénophényle, cyanophényle, alkyl-phényle en $C_7$-$C_{20}$, alcoxyphényle en $C_7$-$C_{20}$, halogénalkyl-phényle en $C_7$-$C_{20}$, alcénylphényle en $C_8$-$C_{20}$, alcynylphényle en $C_8$-$C_{20}$, phénoxyphényle, méthylendioxy-phényle en $C_8$-$C_{12}$, alkylcarbonyle en $C_2$-$C_{20}$, halogénalkyl-carbonyle en $C_2$-$C_{20}$, alcoxycarbonyle en $C_2$-$C_{20}$, alkylcyclopropyl-carbonyle en $C_5$-$C_{20}$, alcényle et alkylcyclopropyl-carbonyle en $C_7$-$C_{20}$, halogénoalkyle et alkylcyclopropyl-carbonyle en $C_6$-$C_{20}$, halogénalcényle et alkylcyclopropyl-carbonyle en $C_7$-$C_{20}$, halogèno et phényl-alkylcarbonyle en $C_8$-$C_{20}$ et halogèno et halogénophénylalkylcarbonyle en $C_8$-$C_{20}$, benzoyle, halogénobenzoyle, alkylbenzoyle en $C_8$-$C_{20}$, alcoxybenzoyle en $C_8$-$C_{20}$, halogénalkylebenzoyle en $C_8$-$C_{20}$, halogénalcoxy-benzoyle en $C_8$-$C_{20}$, phénylalkylcarbonyle en $C_8$-$C_{20}$, halogénophénylyalkylcarbonyle en $C_8$-$C_{20}$, alkylphénylalkyl-carbonyle en $C_9$-$C_{20}$, alcoxyphénylalkylcarbonyle en $C_9$-$C_{20}$, halogénalkylphénylalkylcarbonyle en $C_9$-$C_{20}$, halogénalcoxyphénylalkylcarbonyle en $C_9$-$C_{20}$, phénylalcénylcarbonyle en $C_9$-$C_{20}$, halogénophénylalcényle-carbonyle en $C_9$-$C_{20}$, alkylphénylalcénylcarbonyle en $C_{10}$-$C_{20}$, alcoxyphénylalcénylcarbonyle en $C_{10}$-$C_{20}$, halogénalkylphénylalcényl-carbonyle en $C_{10}$-$C_{20}$, halogénalcoxyphénylalcénylcarbonyle en $C_{10}$-$C_{20}$, phénylalcynylcarbonyle en $C_9$-$C_{20}$, halogénophénylalcynylcarbonyle en $C_9$-$C_{20}$, alkylphénylalcynylcarbonyle en $C_{10}$-$C_{20}$, alcoxyphénylalcynyl-carbonyle en $C_{10}$-$C_{20}$, halogénalkylphénylalcynylcarbonyle en $C_{10}$-$C_{20}$, halogénalcoxyphénylalcynylcarbonyle en $C_{10}$-$C_{20}$, alkylaminocarbonyle en $C_2$-$C_{20}$, halogénalkylaminocarbonyle en $C_2$-$C_{20}$, alcoxyalkylamino-carbonyle en $C_3$-$C_{20}$, halogénalkylalkylaminocarbonyle en $C_3$-$C_{20}$, halogénalcoxyalkylaminocarbonyle en $C_3$-$C_{20}$, dialkylaminocarbonyle en $C_3$-$C_{20}$, halogéndialkylaminocarbonyle en $C_3$-$C_{20}$, alcoxydialkylaminocarbonyle en $C_4$-$C_{20}$, alkyle et -alcoxy-aminocarbonyle en $C_5$-$C_{20}$, halogénalkyldialkylaminocarbonyle en $C_4$-$C_{20}$ halogénalcoxydialkylaminocarbonyle en $C_4$-$C_{20}$, phénylaminocarbonyle, halogénophénylaminocarbonyle, dihalogénophénylaminocarbonyle, trihalogénophénylaminocarbonyle, alkylphénylaminocarbonyle en $C_8$-$C_{20}$, alcoxyphénylaminocarbonyle en $C_8$-$C_{20}$, halogénalcoxyphénylaminocarbonyle en $C_8$-$C_{20}$ ou isoxazole éventuellement substitué par alkyle en $C_1$-$C_8$,
$R^8$, hydrogène ou alkyle en $C_1$-$C_{20}$ et
X, halogène
sous réserve que, dans la formule $I_a$, $R^1$ ne représente pas phényle quand $R^2$, $R^3$, $R^4$, $R^5$ sont mis simultanément pour hydrogène et que dans la formule $I_b$, $R^2$, $R^3$, $R^4$, $R^5$ ne représentent pas simultanément hydrogène quand $R^1$ est mis pour alkyle en $C_4$-$C_8$, phényle, tolyle ou méthoxyphényle, ainsi que $R^1$ ne représente pas octyle quand $R^2$ et $R^3$ sont mis pour hydrogène et $R^4$ ou $R^5$ pour méthyle, méthoxy ou chlore, et sous réserve encore que, dans la formule $I_b$, $R^1$ ne représente pas n-butyle quand $R^2$ est mis pour méthyle et $R^3$, $R^4$ et $R^5$ pour hydrogène caractérisé par le fait qu'on fait réagir de manière connue en soi un éther vynylique de la formule générale II

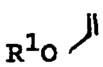 II,

en présence d'un inhibiteur radicalaire et un composé basique, à des températures de 130 à 280 degrés C et des pressions de 1 à 300 bar

a) avec un aldéhyde ou cétone III insaturé en α, β, à l'état monomère

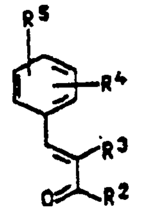

(III)

ou

b) avec un aldéhyde ou cétone (IIIa) insaturé en α, β, à l'état dimère

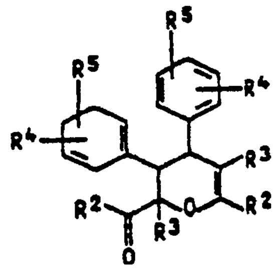

(IIIa)

et le composé $I_b$, éventuellement

c) est hydraté en le composé $I_a$, ou

d) lorsque $R^2$ = H est hydroformylé, ou

e) lorsque $R^2$ et $R^3$ = H, est transformé, avec un composé trihalogénométhane $CHX_3$ en le compose $I_c$,

2. Procédé selon la revendication 1 de préparation de dérivés de pyranne des formules générales $I_a$, Ib et $I_c$ selon la revendication 1, dans lesquelles les substituants ont les significations suivantes,

$R^1$, alkyle en $C_1$-$C_{12}$, alcényle en $C_2$-$C_{14}$, alcynyle en $C_2$-$C_8$, cycloalkyle en $C_3$-$C_8$, phényle, naphtyle, biphénylyle, phénylalkyle en $C_7$-$C_{14}$, phénylalcényle en $C_8$-$C_{14}$, phénylalcynyle en $C_8$-$C_{14}$, alcoxy-alkyle en $C_2$-$C_8$, alcoxy-alcoxy-alkyle en $C_3$-$C_{12}$, alcoxy-alcoxy-alcoxy-alkyle en $C_4$-$C_{16}$, alkyle en $C_1$-$C_8$ substitué par alkylaryle en $C_7$-$C_{10}$, dialkyle-phényle en $C_8$-$C_{14}$, trialkyl-aryle en $C_9$-$C_{14}$, phénoxy, phénoxy-phényle, cycloalkyle en $C_3$-$C_8$, alkyl-cycloalkyle en $C_4$-$C_{12}$, cycloalcényle en $C_4$-$C_8$ ou alkyle-cycloalcényle en $C_5$-$C_{12}$, alkylecycloalkyle en $C_4$-$C_{12}$, phényl-cycloalkyle en $C_9$-$C_{14}$, phénoxy-cycloalkyle en $C_9$-$C_{14}$, alkyle-phényle en $C_7$-$C_{14}$, alcoxy-phényle en $C_7$-$C_{14}$, dialkyl-phényle en $C_8$-$C_{16}$, halogénalkyle-phényle en $C_7$-$C_{14}$, halogénophényle, dihalogénophényle, alkyl-halogénophényle en $C_7$-$C_{14}$, dialkyl-halogénophényle en $C_8$-$C_{14}$, phénoxy-phényle, phénoxy-phénylalkyle en $C_{13}$-$C_{20}$.ou alkyle en $C_1$-$C_8$ substitué par un hétérocycle saturé ou insaturé, de 5 à 6 chaînons à un ou deux hétéroatomes identiques ou différents du groupe constitué par oxygène et azote, qui est éventuellement substitué par un deux ou trois substituants identiques ou différents du groupe constitué par alkyle en $C_1$-$C_8$, phényle, phénylalkyle en $C_7$-$C_{14}$, halogénphényle, alkylphényle en $C_7$-$C_{14}$, dialkyle-phényle en $C_8$-$C_{16}$ trialkyl-aryle en $C_9$-$C_{16}$ ou par un spirocycloalkyle en $C_3$-$C_8$ éventuellement subssitué par alkyle en $C_1$-$C_4$,

$R^2$, hydrogène, alkyle en $C_1$-$C_8$, halogénoalkyle en $C_1$-$C_8$, éventuellement substitués par phényle ou halogénophényle, alcoxy en $C_1$-$C_8$, halogènalcoxy en $C_1$-$C_8$, alcényloxy en $C_2$-$C_8$, halogénalcényloxy en $C_2$-$C_8$, alcoxy-alcényloxy en $C_3$-$C_{12}$, alcynyloxy en $C_2$-$C_8$, halogénalcynyloxy en $C_2$-$C_8$ alcoxyalcynyloxy en $C_3$-$C_{12}$, phényl-alcynyloxy en $C_8$-$C_{16}$, alkylthio en $C_1$-$C_8$, halogénalkylthio en $C_1$-$C_8$, alcoxy-alkylthio en $C_2$-$C_{12}$, formyle, cyano, phénoxy-alcoxy en $C_7$-$C_{14}$, halogénphénoxy-alcoxy en $C_7$-$C_{14}$, phénoxy-halogénalcoxy en $C_7$-$C_{14}$, halogénphénoxy-halogénalcoxy en $C_7$-$C_{14}$, alcoxyphénoxyalcoxy en $C_8$-$C_{16}$, phénoxy-alcoxyalcoxy en $C_8$-$C_{16}$, phénoxy-alcényloxy en $C_7$-$C_{16}$, halogénphénoxy-alcényle en $C_8$-$C_{16}$, phénoxy-halogénalcényloxy en $C_8$-$C_{16}$, halogénphénoxy-halogénalcényloxy en $C_8$-$C_{16}$, alcoxyphénoxy-alcényloxy en $C_9$-$C_{16}$, phénoxy-alcoxyalcényloxy en $C_9$-$C_{16}$, phénoxy-alcynyloxy en $C_8$-$C_{14}$, halogénphénoxy-alcynyloxy en $C_8$-$C_{14}$, phénoxy-halogénalcynyloxy en $C_8$-$C_{14}$, halogénphénoxy-halogénalcynyloxy en $C_8$-$C_{14}$, alcoxyphénoxy-alcynyloxy en $C_9$-$C_{16}$, phénoxy-alcoxyalcynyloxy en $C_9$-$C_{16}$, phénylalcynyloxy en $C_8$-$C_{14}$, phénoxyphényl-alcynyloxy en $C_{14}$-$C_{20}$, carboxy, alcoxycarbonyle en $C_2$-$C_8$, alkyl-carbonyloxy en $C_2$-$C_8$, alcénylocxycarbonyle en $C_3$-$C_{12}$, alcénylcarbonyloxy en $C_3$-$C_{12}$, alcinyloxycarbonyle en $C_3$-$C_{12}$, alcynylcarbonyloxy en $C_3$-$C_{12}$, phénoxycarbonyle, phénylcarbonyloxy, alcoxycarbonyl-alcoxy en $C_3$-$C_{12}$, phénoxy, alkylphénoxy en $C_7$-$C_{14}$, halogénophénoxy, dihalogénophénoxy, trihalogénophénoxy, halogénalkyl-phénoxy en $C_7$-$C_{14}$, alcoxyphénoxy en $C_7$-$C_{14}$, phénoxy-phénoxy, alkylendioxy-phénoxy en $C_8$-$C_{14}$, phénylthio,

alkylphénylthio en $C_7$-$C_{14}$, halogén-phénylthio, dihalogén-phénylthio, trihalogén-phénylthio, halogénalkylphénylthio en $C_7$-$C_{14}$, alcoxyphénylthio en $C_7$-$C_{14}$, phénoxy-phénylthio, alkylendioxyphénylthio en $C_8$-$C_{14}$, phényle, phénoxy-phényle, phénylalkyle en $C_7$-$C_{14}$, phénoxy-phénylalkyle en $C_{13}$-$C_{20}$, alkylendioxy-phénylalkyloxy en $C_8$-$C_{14}$ ou un reste choisi parmi :

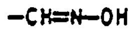 ; 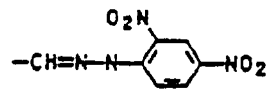 ; 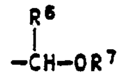 ; 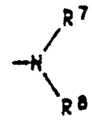 ;

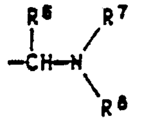 ; 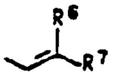 ; 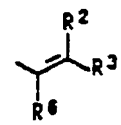 ; 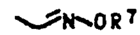 ou 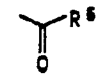

$R^3$, hydrogène, alkyle en $C_1$-$C_8$ ou halogénalkyle en $C_1$-$C_2$
$R^4$, $R^5$, hydrogène, fluor, chlore, alkyle en $C_1$-$C_3$, alcoxy en $C_1$-$C_3$ ou halogénalcoxy en $C_1$-$C_3$,
$R^6$, hydrogène, alkyle en $C_1$-$C_8$, alcényle en $C_2$-$C_8$, alcynyle en $C_2$-$C_8$, cyano, phényle, halogénophényle, alkylphényle en $C_7$-$C_{14}$ ou alcoxyphényle en $C_7$-$C_{14}$
$R^7$, hydrogène, alkyle en $C_1$-$C_8$, halogénalkyle en $C_1$-$C_8$, alcoxy-alkyle en $C_2$-$C_8$, alcényle en $C_2$-$C_8$, alcynyle en $C_2$-$C_8$, aryle, halogénophényle, dihalogénophényle, trihalogénophényle, cyanophényle, alkylphényle en $C_7$-$C_{14}$, alcoxy-phényle en $C_7$-$C_{14}$, halogénalkyl-phényle en $C_7$-$C_{14}$, alcénylphényle en $C_8$-$C_{16}$, alcynylphényle en $C_8$-$C_{16}$, phénoxyphényle, méthylendioxy-phényle en $C_8$-$C_{16}$, alkylcarbonyle en $C_2$-$C_8$, halogénalkylcarbonyle en $C_2$-$C_8$, alcoxycarbonyle en $C_2$-$C_8$, alkylcyclopropyle-carbonyle en $C_5$-$C_{12}$, alcényle en $C_7$-$C_{14}$ et alkylcyclopropyle-carbonyle, halogénalkyle et alcylcyclopropyl-carbonyle en $C_6$-$C_{14}$, halogénalcényl- et alcylcyclopropyl-carbonyle en $C_7$-$C_{14}$, halogèno et phényl-alkylcarbonyle en $C_8$-$C_{16}$, halogèno et halogènphénylalcylcarbonyle en $C_8$-$C_{16}$, benzoyle, halogénbenzoyle, alkylbenzoyle en $C_8$-$C_{14}$, alcoxybenzoyle en $C_8$-$C_{14}$, halogénalkylbenzoyle en $C_8$-$C_{14}$, halogén-alcoxy-benzoyle en $C_8$-$C_{14}$, Phenylalkylcarbonyle en $C_8$-$C_{14}$, halogénphénylalkylcarbonyle en $C_8$-$C_{14}$, alkylphénylalkylcarbonyle en $C_9$-$C_{16}$, alcoxyphényl-alkylcarbonyle en $C_9$-$C_{16}$, halogénalkylphénylalkylcarbonyle en $C_9$ $C_{16}$, halogénalcoxyphénylalkylcarbonyle en $C_9$-$C_{16}$, phénylalcénylcarbonyle en $C_9$-$C_{16}$, halogènophénylalcénylcarbonyle en $C_9$-$C_{16}$, alkylphénylalcénylcarbonyle en $C_{10}$-$C_{16}$, alcoxyphénylalcénylcarbonyle en $C_{10}$-$C_{16}$, halogène-alkylphénylalcénylecarbonyle en $C_{10}$-$C_{16}$, halogènalcoxyphénylalcényl-carbonyle en $C_{10}$-$C_{16}$, phényl-alcynylcarbonyle en $C_9$-$C_{16}$, halogénophénylalcynyl-carbonyle en $C_9$-$C_{16}$, alkylphénylalcynylcarbonyle en $C_{10}$-$C_{16}$, alcoxyphényl-alcynylcarbonyle en $C_{10}$-$C_{16}$, halogénalkylcynylcarbonyle en $C_{10}$-$C_{16}$, halogénalkoxyphénylalphénylcarbonyle en $C_{10}$-$C_{16}$, alkylaminocarbonyle en $C_2$-$C_8$, halogénalkylaminocarbonyle en $C_2$-$C_8$, alkylalkylaminocarbonyle en $C_3$-$C_{10}$, alcoxyalkylaminocarbonyle en $C_3$-$C_{10}$, halogénalkylalkylaminocarbonyle en $C_3$-$C_{10}$, halogénalcoxyalkylaminocarbonyle en $C_3$-$C_{10}$, dialkyl-aminocarbonyle en $C_3$-$C_{10}$, halogénodialkylaminocarbonyle en $C_3$-$C_8$, alkyl-dialkylaminocarbonyle en $C_4$-$C_{12}$, alcoxydialkylaminocarbonyle en $C_4$-$C_{12}$, alkyl et alcoxy-aminocarbonyle en $C_5$-$C_{20}$, halogénalkyldialkyl-aminocarbonyle en $C_4$-$C_{12}$, halogénalcoxydialkylaminocarbonyle en $C_4$-$C_{12}$, phényl-aminocarbonyle, halogénophénylaminocarbonyle, dihalogénophénylaminocarbonyle, trihalogénophénylaminocarbonyle, alkylphélnylaminocarbonyle en $C_8$-$C_{14}$, alcoxyphénylaminocarbonyle en $C_8$-$C_{14}$, halogénalcoxyphénylaminocarbonyle en $C_8$-$C_{14}$ ou isoxazole éventuellement substitué par alkyle en $C_1$-$C_4$,
$R^8$, hydrogène ou alkyle en $C_1$-$C_8$ et
X, halogène
sous réserve que, dans la formule $I_a$, $R^1$ ne représente pas phényle quand $R^2$, $R^3$, $R^4$, $R^5$ sont mis simultanément pour hydrogène et que, dans la formule $I_b$, $R^2$, $R^3$, $R^4$, $R^5$ ne représentent pas simultanément hydrogène quand $R^1$ est mis pour alkyle en $C_4$-$C_8$, phényle, tolyle ou méthoxyphényle, ainsi que $R^1$ ne représente pas octyle quand $R^2$ et $R^3$ sont mis pour hydrogène et $R^4$ ou $R^5$ pour méthyle, méthoxy ou chlore, et sous réserve encore que, dans la formule $I_b$, $R^1$ ne représente pas n-butyle quand $R^2$ est mis pour méthyle et $R^3$, $R^4$ et $R^5$ pour hydrogène

3. Pesticide contenant un dérivé de dérivés de pyranne des formules générales $I_a$, $I_b$ ou $I_c$

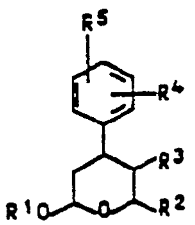

(Ia)

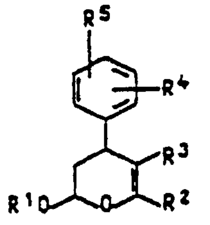

(Ib)

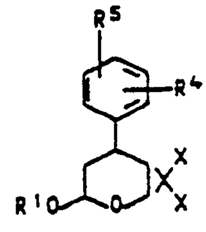

(Ic)

dans lesquelles les substituants ont les significations suivantes :

$R^1$ alkyle en $C_1$-$C_{20}$, alcényle en $C_2$-$C_{20}$, alcynyle en $C_2$-$C_{20}$, cycloalkyle en $C_3$-$C_{20}$, aryle, biphénylyle, arylalkyle en $C_7$-$C_{30}$, arylalcényle en $C_8$-$C_{30}$, aryl-alcynyle en $C_8$-$C_{30}$, alcoxy-alkyle en $C_2$-$C_{20}$, alcoxy-alcoxy-alkyle en $C_3$-$C_{20}$, alcoxy-alcoxy-alcoxy-alkyle en $C_4$-$C_{30}$, alkyle en $C_1$-$C_{20}$ substitué par alkyle-aryle en $C_7$-$C_{20}$, dialkyle-aryle en $C_8$-$C_{20}$, trialkyl-aryle en $C_9$-$C_{20}$, aryloxy, aryloxy-aryle, cycloalkyle en $C_3$-$C_{20}$, alkyl-cycloalkyle en $C_4$-$C_{20}$, cycloalcényle en $C_4$-$C_{20}$ ou alkylcycloalcényle en $C_5$-$C_{20}$, alkyl-cycloalkyle en $C_4$-$C_{20}$, aryl-cycloalkyle en $C_9$-$C_{20}$, aryloxycycloalkyle en $C_9$-$C_{30}$, alkyl-aryle en $C_7$-$C_{20}$, alcoxy-aryle en $C_7$-$C_{20}$, dialkyl-aryle en $C_8$-$C_{20}$, halogénalkyl-aryle en $C_7$-$C_{20}$, halogénaryle, dihalogénaryle, alkyl-halogénaryle en $C_7$-$C_{20}$, dialkyl-halogénaryle en $C_8$-$C_{20}$, aryloxy-aryle, aryloxy-arylalkyle en $C_{13}$-$C_{30}$, ou alkyle en $C_1$-$C_{20}$ substitué par un hétérocycle saturé ou insaturé, de 5-, 6-, 7- ou 8 chaînons, à un, deux ou trois hétéroatomes identiques ou différents du groupe constitué par oxygène, soufre et azote, qui est éventuellement substitué par un, deux ou trois substituants identiques ou différents du groupe constitué par alkyle en $C_1$-$C_{20}$, aryle, arylalkyle en $C_7$-$C_{20}$, halogénaryle, alkylaryle en $C_7$-$C_{20}$, dialkyl-aryle en $C_8$-$C_{20}$, trialkyl-aryle en $C_9$-$C_{20}$, ou par un spiro-cycloalkyle en $C_3$-$C_{20}$ éventuellement substitué par alkyle en $C_1$-$C_8$

$R^2$, hydrogène, alkyle en $C_1$-$C_{20}$, halogénoalkyle en $C_1$-$C_{20}$, éventuellement substitués par phényle ou halogénophényle ; halogèn-phényl-halogénalkyle en $C_7$-$C_{20}$, alcoxy en $C_1$-$C_{20}$, halogénalcoxy en $C_1$-$C_{20}$, alcényloxy en $C_2$-$C_{20}$, halogénalcényloxy en $C_2$-$C_{20}$, alkoxy-alcényloxy en $C_3$-$C_{20}$, alcynyloxy en $C_2$-$C_{20}$, halogénalcynyloxy en $C_2$-$C_{20}$, alcoxy -alcynyloxy en $C_3$-$C_{20}$, phényl-alcynyloxy en $C_8$-$C_{20}$, alkylthio en $C_1$-$C_{20}$, halogénalkylthio en $C_1$-$C_{20}$, alcoxy-alkylthio en $C_2$-$C_{20}$, formyle, cyano, phénoxy-alcoxy en $C_7$-$C_{20}$, halogénphenoxy-alcoxy en $C_7$-$C_{20}$, phénoxy-halogénalcoxy en $C_7$-$C_{20}$, halogénphénoxyhalogénalcoxy en $C_7$-$C_{20}$, alcoxy-phénoxyalcoxy en $C_8$-$C_{20}$, phénoxy-alcoxyalcoxy en $C_8$-$C_{20}$, phénoxy-alcényloxy en $C_7$-$C_{20}$, halogénphénoxy-alcynyle en $C_8$-$C_{20}$, phénoxy-halogénalcényloxy en $C_8$-$C_{20}$, halogénphénoxy-halogénalcényloxy en $C_8$-$C_{20}$, alcoxyphénoxy-alcényloxy en $C_9$-$C_{20}$, phénoxy-alcoxyalcényloxy en $C_9$-$C_{20}$, phénoxy-alcynyloxy en $C_8$-$C_{20}$, halogénphénoxy-alcynyle en $C_8$-$C_{20}$, phénoxy-halogénalcynyloxy en $C_8$-$C_{20}$, halogénphénoxyhalogénalcynyloxy en $C_8$-$C_{20}$, alcoxyphénoxyalcynyloxy en $C_9$-$C_{20}$, phénoxy-alcoxyalcynyloxy en $C_9$-$C_{20}$, phénylalcynyloxy en $C_{8-20}$, phénoxyphénylalcynyloxy en $C_{14}$-$C_{30}$, carboxy, alcoxycarbonyle en $C_2$-$C_{20}$, alkylcarbonyloxy en $C_2$-$C_{20}$, alcényloxycarbonyle en $C_3$-$C_{20}$, alcénylcarbonyloxy en $C_3$-$C_{20}$, alcynyloxy-carbonyle en $C_3$-$C_{20}$, alcynylcarbonyloxy en $C_3$-$C_{20}$, phénoxycarbonyle, phényl-carbonyloxy, alcoxycarbonyl-alcoxy en $C_3$-$C_{20}$, phénoxy, alkylphénoxy en $C_7$-$C_{20}$, halogénophénoxy, dihalogénophénoxy, trihalogénophénoxy, halogénalkyl-phénoxy en $C_7$-$C_{20}$, alcoxyphénoxy en $C_7$-$C_{20}$, phénoxy-phénoxy, alkylendioxy-phénoxy en $C_8$-$C_{12}$, phénylthio, alkylphénylthio en $C_7$-$C_{20}$, halogèn-phénlylthio, dihalogèn-phénylthio, trihalogén-phénylthio, halogénalkyl-phénylthio en $C_7$-$C_{20}$, alcoxyphénylthio en $C_7$-$C_{20}$, phénoxy-phénylthio, alkylendioxyphénylthio en $C_8$-$C_{12}$, phényle, phénoxy-phényle, phénylalkyle en $C_7$-$C_{20}$, phénoxy-phénylalkyle en $C_{13}$-$C_{30}$, alkylendioxy-phénylalcoxy en $C_8$-$C_{12}$ ou un reste choisi parmi

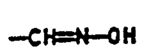 ; 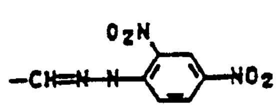 ; 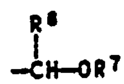 ; 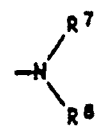 ;

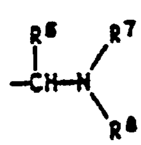 ; 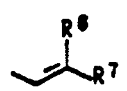 ; 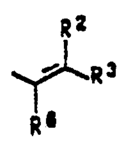 ; 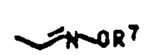 ou 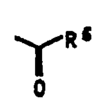

$R^3$, hydrogène, alkyle en $C_1$-$C_{20}$ ou halogénoalkyle en $C_1$-$C_3$

$R^4$, $R^5$, hydrogène, fluor, chlore, alkyle en $C_1$-$C_3$, alcoxy en $C_1$-$C_3$ ou halogénalcoxy en $C_1$-$C_3$,

$R^6$, hydrogène, alkyle en $C_1$-$C_{20}$, alcényle en $C_2$-$C_{20}$, alcynyle en $C_2$-$C_{20}$, cyano, phényle, halogénophényle,

alkylphényle en $C_7$-$C_{20}$ ou alcoxyphényle en $C_7$-$C_{20}$,

$R^7$, hydrogène, alkyle en $C_1$-$C_{20}$, halogénalkyle en $C_1$-$C_{20}$, alcoxyalkyle en $C_2$-$C_{20}$, alcényle en $C_2$-$C_{20}$, alcynyle en $C_2$-$C_{20}$, aryle, halogénophényle, dihalogénophényle, trihalogénophényle, cyanophényle, alkyl-phényle en $C_7$-$C_{20}$, alcoxyphényle en $C_7$-$C_{20}$, halogénalkyl-phényle en $C_7$-$C_{20}$, alcénylphényle en $C_8$-$C_{20}$, alcynylphényle en $C_8$-$C_{20}$, phénoxyphényle, méthylendioxy-phényle en $C_8$-$C_{12}$, alkylcarbonyle en $C_2$-$C_{20}$, halogénalkyl-carbonyle en $C_2$-$C_{20}$, alcoxycarbonyle en $C_2$-$C_{20}$, alkylcyclopropyl-carbonyle en $C_5$-$C_{20}$, alcényle et alkylcyclopropyl-carbonyle en $C_7$-$C_{20}$, halogénoalkyle et alkylcyclopropyl-carbonyle en $C_6$-$C_{20}$, halogénalcényle et alkylcyclopropyl-carbonyle en $C_7$-$C_{20}$, halogèno et phényl-alkylcarbonyle en $C_8$-$C_{20}$ et halogèno et halogénophénylalkylcarbonyle en $C_8$-$C_{20}$, benzoyle, halogénobenzoyle, alkylbenzoyle en $C_8$-$C_{20}$, alcoxybenzoyle en $C_8$-$C_{20}$, halogénalkyle-benzoyle en $C_8$-$C_{20}$, halogénalcoxy-benzoyle en $C_8$-$C_{20}$, phénylalkylcarbonyle en $C_8$-$C_{20}$, halogénophénylalkylcarbonyle en $C_8$-$C_{20}$, alkylphénylalkyl-carbonyle en $C_9$-$C_{20}$, alcoxyphénylalkylcarbonyle en $C_9$-$C_{20}$, halogénalkylphénylalkylcarbonyle en $C_9$-$C_{20}$, halogénalcoxyphénylalkylcarbonyle en $C_9$-$C_{20}$, phénylalcénylcarbonyle en $C_9$-$C_{20}$, halogénophénylalcényle-carbonyle en $C_9$-$C_{20}$, alkylphénylalcénylcarbonyle en $C_{10}$-$C_{20}$, alcoxyphénylalcénylcarbonyle en $C_{10}$-$C_{20}$, halogénalkylphénylalcényl-carbonyle en $C_{10}$-$C_{20}$, halogénalcoxyphénylalcénylcarbonyle en $C_{10}$-$C_{20}$, phénylalcynylcarbonyle en $C_9$-$C_{20}$, halogénophénylalcynylcarbonyle en $C_9$-$C_{20}$, alkylphénylalcynylcarbonyle en $C_{10}$-$C_{20}$, alcoxyphénylalcynyl-carbonyle en $C_{10}$-$C_{20}$, halogénalkylphénylalcynylcarbonyle en $C_{10}$-$C_{20}$, halogénalcoxyphénylalcynylcarbonyle en $C_{10}$-$C_{20}$, alkylaminocarbonyle en $C_2$-$C_{20}$, halogénalkylaminocarbonyle en $C_2$-$C_{20}$, alcoxyalkylamino-carbonyle en $C_3$-$C_{20}$, halogénalkylalkylaminocarbonyle en $C_3$-$C_{20}$, halogénalcoxyalkylaminocarbonyle en $C_3$-$C_{20}$, dialkylaminocarbonyle en $C_3$-$C_{20}$, halogéndialkylaminocarbonyle en $C_3$-$C_{20}$, alcoxydialkylaminocarbonyle en $C_4$-$C_{20}$, alkyle etalcoxy-aminocarbonyle en $C_5$-$C_{20}$, halogénalkyl-dialkylaminocarbonyle en $C_4$-$C_{20}$, halogénalcoxydialkylaminocarbonyle en $C_4$-$C_{20}$, phénylaminocarbonyle, halogénophénylaminocarbonyle, dihalogénophénylaminocarbonyle, trihalogénophénylaminocarbonyle, alkylphénylaminocarbonyle en $C_8$-$C_{20}$, alcoxyphénylaminocarbonyle en $C_8$-$C_{20}$, halogénalcoxyphénylaminocarbonyle en $C_8$-$C_{20}$ ou isoxazole éventuellement substitué par alkyle en $C_1$-$C_8$,

$R^8$, hydrogène ou alkyle en $C_1$-$C_{20}$ et

X, halogène

sous réserve que, dans la formule $I_a$, $R^1$ ne représente pas phényle quand $R^2$, $R^3$, $R^4$, $R^5$ sont mis simultanément pour hydrogène et que dans la formule $I_b$, $R^2$, $R^3$, $R^4$, $R^5$ ne représentent pas simultanément hydrogène quand $R^1$ est mis pour alkyle en $C_4$-$C_8$, phényle, tolyle ou méthoxyphényle, ainsi que $R^1$ ne représente pas octyle quand $R^2$ et $R^3$ sont mis pour hydrogène et $R^4$ ou $R^5$ pour méthyle, méthoxy ou chlore, et sous réserve encore que, dans la formule $I_b$, $R^1$ ne représente pas n-butyle quand $R^2$ est mis pour méthyle et $R^3$, $R^4$ et $R^5$ pour hydrogène

4. Pesticide selon la revendication 3 contenant un dérivé de pyranne $I_a$, $I_b$ ou $I_c$, selon la revendication 3, dans lesquelles les substituants ont les significations suivantes,

$R^1$, alkyle en $C_1$-$C_{12}$, alcényle en $C_2$-$C_{14}$, alcynyle en $C_2$-$C_8$, cycloalkyle en $C_3$-$C_8$, phényle, naphtyle, biphénylyle, phénylalkyle en $C_7$-$C_{14}$, phénylalcényle en $C_8$-$C_{14}$, phénylalcynyle en $C_8$-$C_{14}$, alcoxy-alkyle en $C_2$-$C_8$, alcoxy-alcoxy-alkyle en $C_3$-$C_{12}$, alcoxy-alcoxy-alcoxy-alkyle en $C_4$-$C_{16}$, alkyle en $C_1$-$C_8$ substitué par alkyl-aryle en $C_7$-$C_{10}$, dialkylephényle en $C_8$-$C_{14}$, trialkyl-aryle en $C_9$-$C_{14}$, phénoxy, phénoxy-phényle, cycloalkyle en $C_3$-$C_8$, alkyl-cycloalkyle en $C_4$-$C_{12}$, cycloalcényle en $C_4$-$C_8$ ou alkyle-cycloalcényle en $C_5$-$C_{12}$, alkylecycloalkyle en $C_4$-$C_{12}$, phényl-cycloalkyle en $C_9$-$C_{14}$, phénoxy-cycloalkyle en $C_9$-$C_{14}$, alkyle-phényle en $C_7$-$C_{14}$, alcoxy-phényle en $C_7$-$C_{14}$, dialkylphényle en $C_8$-$C_{16}$, halogénalkyle-phényle en $C_7$-$C_{14}$, halogénophényle, dihalogénophényle, alkyl-halogénophényle en $C_7$-$C_{14}$, dialkyl-halogénophényle en $C_8$-$C_{14}$, phénoxy-phényle, phénoxy-phénylalkyle en $C_{13}$-$C_{20}$.ou alkyle en $C_1$-$C_8$ substitué par un hétérocycle saturé ou insaturé, de 5 à 6 chaînons à un ou deux hétéroatomes identiques ou différents du groupe constitué par oxygène et azote, qui est éventuellement substitué par un deux ou trois substituants identiques ou différents du groupe constitué par alkyle en $C_1$-$C_8$, phényle, phénylalkyle en $C_7$-$C_{14}$, halogénphényle, alkylphényle en $C_7$-$C_{14}$, dialkyle-phényle en $C_8$-$C_{16}$ trialkyl-aryle en $C_9$-$C_{16}$ ou par un spirocycloalkyle en $C_3$-$C_8$ éventuellement substitué par alkyle en $C_1$-$C_4$,

$R^2$, hydrogène, alkyle en $C_1$-$C_8$, halogénoalkyle en $C_1$-$C_8$, éventuellement substitués par phényle ou halogénophényle, alcoxy en $C_1$-$C_8$, halogènalcoxy en $C_1$-$C_8$, alcényloxy en $C_2$-$C_8$, halogénalcényloxy en $C_2$-$C_8$, alcoxy-alcényloxy en $C_3$-$C_{12}$, alcynyloxy en $C_2$-$C_8$, halogénalcynyloxy en $C_2$-$C_8$ alcoxyalcynyloxy en $C_3$-$C_{12}$, phényl-alcynyloxy en $C_8$-$C_{16}$, alkylthio en $C_1$-$C_8$, halogénalkylthio en $C_1$-$C_8$, alcoxy-alkylthio en $C_2$-$C_{12}$, formyle, cyano, phénoxy-alcoxy en $C_7$-$C_{14}$, halogénphénoxy-alcoxy en $C_7$-$C_{14}$, phénoxy-halogénalcoxy en $C_7$-$C_{14}$, halogénphénoxyhalogénalcoxy en $C_7$-$C_{14}$, alcoxyphénoxyalcoxy en $C_8$-$C_{16}$, phénoxy-alcoxyalcoxy en $C_8$-$C_{16}$, phénoxy-alcényloxy en $C_7$-$C_{16}$, halogénphénoxy-alcényle en $C_8$-$C_{16}$, phénoxy-halogénalcényloxy en $C_8$-$C_{16}$, halogénphénoxy-halogénalcényloxy en $C_8$-$C_{16}$, alcoxyphénoxy-alcényloxy en $C_9$-$C_{16}$, phénoxy-alcoxyalcényloxy en $C_9$-$C_{16}$, phénoxy-alcynyloxy en $C_8$-$C_{14}$, halogénphénoxy-alcynyloxy en $C_8$-$C_{14}$, phé-

noxy-halogénalcynyloxy en $C_8$-$C_{14}$, halogénphénoxy-halogénalcynyloxy en $C_8$-$C_{14}$, alcoxyphénoxy-alcynyloxy en $C_9$-$C_{16}$, phénoxy-alcoxyalcynyloxy en $C_9$-$C_{16}$, phénylalcynyloxy en $C_8$-$C_{14}$, phénoxyphényl-alcynyloxy en $C_{14}$-$C_{20}$, carboxy, alcoxycarbonyle en $C_2$-$C_8$, alkyl-carbonyloxy en $C_2$-$C_8$, alcénylocxycarbonyle en $C_3$-$C_{12}$, alcénylcarbonyloxy en $C_3$-$C_{12}$, alcinyloxycarbonyle en $C_3$-$C_{12}$, alcynylcarbonyloxy en $C_3$-$C_{12}$, phénoxycarbonyle, phénylcarbonyloxy, alcoxycarbonyl-alcoxy en $C_3$-$C_{12}$, phénoxy, alkylphénoxy en $C_7$-$C_{14}$, halogénophénoxy, dihalogénophénoxy, trihalogénophénoxy, halogénalkyl-phénoxy en $C_7$-$C_{14}$, alcoxyphénoxy en $C_7$-$C_{14}$, phénoxy-phénoxy, alkylendioxy-phénoxy en $C_8$-$C_{14}$, phénylthio, alkylphénylthio en $C_7$-$C_{14}$, halogén-phénylthio, dihalogén-phénylthio, trihalogén-phénylthio, halogénalkyl-phénylthio en $C_7$-$C_{14}$, alcoxyphénylthio en $C_7$-$C_{14}$, phénoxy-phénylthio, alkylendioxyphénylthio en $C_8$-$C_{14}$, phényle, phénoxy-phényle, phénylalkyle en $C_7$-$C_{14}$, phénoxy-phénylalkyle en $C_{13}$-$C_{20}$, alkylendioxy-phénylalkyloxy en $C_8$-$C_{14}$ ou un reste choisi parmi :

$-CH=N-OH$ ; 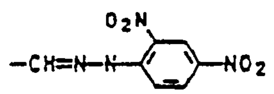 ; 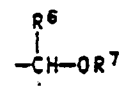 ; 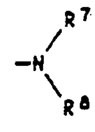 ;

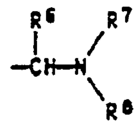 ; 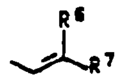 ; 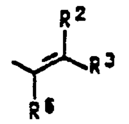 ; 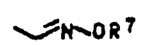 ou 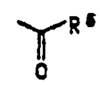

$R^3$, hydrogène, alkyle en $C_1$-$C_8$ ou halogénalkyle en $C_1$-$C_2$

$R^4$, $R^5$, hydrogène, fluor, chlore, alkyle en $C_1$-$C_3$, alcoxy en $C_1$-$C_3$ ou halogénalcoxy en $C_1$-$C_3$,

$R^6$, hydrogène, alkyle en $C_1$-$C_8$, alcényle en $C_2$-$C_8$, alcynyle en $C_2$-$C_8$, cyano, phényle, halogénophényle, alkylphényle en $C_7$-$C_{14}$ ou alcoxyphényle en $C_7$-$C_{14}$

$R^7$, hydrogène, alkyle en $C_1$-$C_8$, halogénalkyle en $C_1$-$C_8$, alcoxy-alkyle en $C_2$-$C_8$, alcényle en $C_2$-$C_8$, alcynyle en $C_2$-$C_8$, aryle, halogénophényle, dihalogénophényle, trihalogénophényle, cyanophényle, alkylphényle en $C_7$-$C_{14}$, alcoxy-phényle en $C_7$-$C_{14}$, halogénalkyl-phényle en $C_7$-$C_{14}$, alcénylphényle en $C_8$-$C_{16}$, alcynylphényle en $C_8$-$C_{16}$, phénoxyphényle, méthylendioxy-phényle en $C_8$-$C_{16}$, alkylcarbonyle en $C_2$-$C_8$, halogénalkylcarbonyle en $C_2$-$C_8$, alcoxycarbonyle en $C_2$-$C_8$, alcylcyclopropyle-carbonyle en $C_5$-$C_{12}$, alcényle en $C_7$-$C_{14}$ et alkylcyclopropyle-carbonyle, halogénalkyle et alkylcyclopropyl-carbonyle en $C_6$-$C_{14}$, halogénalcényl- et alcylcyclopropyl-carbonyle en $C_7$-$C_{14}$, halogèno et phényl-alkylcarbonyle en $C_8$-$C_{16}$, halogèno et halogènphénylalcylcarbonyle en $C_8$-$C_{16}$, benzoyle, halogénbenzoyle, alkylbenzoyle en $C_8$-$C_{14}$, alcoxybenzoyle en $C_8$-$C_{14}$, halogénalkylbenzoyle en $C_8$-$C_{14}$, halogén-alcoxy-benzoyle en $C_8$-$C_{14}$, Phenylalkylcarbonyle en $C_8$-$C_{14}$ halogénphénylalkylcarbonyle en $C_8$-$C_{14}$, alkylPhénylalkylcarbonyle en $C_9$-$C_{16}$, alcoxyphényl-alkylcarbonyle en $C_9$-$C_{16}$, halogénalkylphénylalkylcarbonyle en $C_9$-$C_{16}$ halogénalcoxyphénylalkylcarbonyle en $C_9$-$C_{16}$, phénylalcénylcarbonyle en $C_9$-$C_{16}$, halogènophénylalcénylcarbonyle en $C_9$-$C_{16}$, alkylphénylalcényl-carbonyle en $C_{10}$-$C_{16}$, alcoxyphénylalcénylcarbonyle en $C_{10}$-$C_{16}$, halogène-alkylphénylalcénylecarbonyle en $C_{10}$-$C_{16}$, halogènalcoxyphénylalcényl-carbonyle en $C_{10}$-$C_{16}$, phényl-alcynylcarbonyle en $C_9$-$C_{16}$, halogénophénylalcynyl-carbonyle en $C_9$-$C_{16}$, alkylphénylalcynylcarbonyle en $C_{10}$-$C_{16}$, alcoxyphényl-alcynylcarbonyle en $C_{10}$-$C_{16}$, halogénalkylphénylcarbonyle en $C_{10}$-$C_{16}$, halogénalkoxyphénylalcynylcarbonyle en $C_{10}$-$C_{16}$, alkylaminocarbonyle en $C_2$-$C_8$, halogénalkylaminocarbonyle en $C_2$-$C_8$, alkylalkylaminocarbonyle en $C_3$-$C_{10}$, alcoxyalkylaminocarbonyle en $C_3$-$C_{10}$, halogénalkylalkylaminocarbonyle en $C_3$-$C_{10}$, halogénalcoxyalkylaminocarbonyle en $C_3$-$C_{10}$, dialkyl-aminocarbonyle en $C_3$-$C_{10}$, halogénodialkylaminocarbonyle en $C_3$-$C_8$, alkyldialkylaminocarbonyle en $C_4$-$C_{12}$, alcoxydialkylaminocarbonyle en $C_4$-$C_{12}$, alkyl et alcoxyaminocarbonyle en $C_5$-$C_{20}$, halogénalkyldialkyl-aminocarbonyle en $C_4$-$C_{12}$, halogénalcoxydialkylaminocarbonyle en $C_4$-$C_{12}$, phényl-aminocarbonyle, halogénophénylaminocarbonyle, dihalogénophénylaminocarbonyle, trihalogénophénylaminocarbonyle, alkylphélnylaminocarbonyle en $C_8$-$C_{14}$, alcoxyphénylaminocarbonyle en $C_8$-$C_{14}$, halogénalcoxyphénylaminocarbonyle en $C_8$-$C_{14}$ ou isoxazole éventuellement substitué par alkyle en $C_1$-$C_4$,

$R^8$, hydrogène ou alkyle en $C_1$-$C_8$ et

X, halogène

sous réserve que, dans la formule $I_a$, $R^1$ ne représente pas phényle quand $R^2$, $R^3$, $R^4$, $R^5$ sont mis simultanément pour hydrogène et que, dans la formule $I_b$, $R^2$, $R^3$, $R^4$, $R^5$ ne représentent pas simultanément hydrogène quand $R^1$ est mis pour alkyle en $C_4$-$C_8$, phényle, tolyle ou méthoxyphényle, ainsi que $R^1$ ne représente pas octyle quand $R^2$ et $R^3$ sont mis pour hydrogène et $R^4$ ou $R^5$ pour méthyle, méthoxy ou chlore, et sous réserve encore que, dans la formule $I_b$, $R^1$ ne représente pas n-butyle quand $R^2$ est mis pour méthyle

et $R^3$, $R^4$ et $R^5$ pour hydrogène
à côté de supports usuels pour cela.

5. Pesticide selon la revendication 3, caractérisé par le fait qu'il contient 0,1 à 95% en poids d'un dérivé de pyranne $I_a$, $I_b$ ou $I_c$.

6. Procédé de lutte contre les parasites, caractérisé par le fait que l'on traite les parasites et/ou les surfaces et/ou espaces à maintenir exempts de parasites, avec une quantité efficace contre les parasites d'un agent selon l'une des revendications 3 ou 4.